# EUROPEAN PATENT SPECIFICATION

(11) **EP 3 484 449 B1**
(45) Date of publication and mention of the grant of the patent: **04.11.2020**
(21) Application number: 17720020.1
(22) Date of filing: 12.04.2017
(51) Int. Cl.: A61K 9/14, A61K 31/455, A61K 31/19, A61K 9/50, A61K 9/48, A61K 9/28, A61K 9/16, A61P 3/02

(54) **SHELLAC MICROCAPSULE FORMULATIONS AND COMPOSITIONS FOR TOPICAL INTESTINAL DELIVERY OF VITAMIN B3**
SCHELLACK-MIKROKAPSEL-FORMULIERUNGEN UND ZUSAMMENSETZUNGEN FÜR DIE TOPISCH-INTESTINALE VERABREICHUNG VON VITAMIN B3
FORMULATIONS DE MICROCAPSULES DE GOMME-LAQUE ET COMPOSITIONS DESTINÉES À L'ADMINISTRATION INTESTINALE TOPIQUE DE LA VITAMINE B3

(30) Priority: 19.04.2016 EP 16165989
(43) Date of publication of application: 22.05.2019
(73) Proprietor: CONARIS Research Institute AG, 24118 Kiel (DE); Christian-Albrechts-Universität zu Kiel, 24118 Kiel (DE)
(72) Inventor: SCHWARZ, Karin, 24118 Kiel (DE); KEPPLER, Julia, 24118 Kiel (DE); THEISMANN, Eva-Maria, 24118 Kiel (DE); KNIPP, Jörg, 24118 Kiel (DE); FANGMANN, Daniela, 24105 Kiel (DE); LAUDES, Matthias, 24105Kiel (DE); SCHREIBER, Stefan, 24105 Kiel (DE); WÄTZIG, Georg, 24118 Kiel (DE)
(74) Representative: Eisenführ Speiser
(86) International application number: PCT/EP2017/058733
(87) International publication number: WO 2017/182347

(56) References cited:
- WO-A1-2010/014011
- WO-A1-2013/087391
- WO-A2-2008/091870
- US-B1- 6 620 431
- FARAG Y ET AL: "Physiochemical Properties of Various Shellac Types", INTERNET CITATION, 1 May 2005 (2005-05-01), pages 33-39, XP002689795, ISSN: 1521-298X Retrieved from the Internet: URL:http://www.dissolutiontech.com/DTresou r/200905Articles/DT200905_A04.pdf [retrieved on 2013-01-08]

## Description

### FIELD OF THE INVENTION

The present invention relates to a microcapsule, comprising a core containing vitamin B3, the use of such a microcapsule as a medicament, nutraceutical, dietary supplement, food ingredient or food, and the use of such a microcapsule in the therapy and/or prophylaxis of a multitude of diseases. The present invention further relates to formulations and compositions comprising such a microcapsule and a method for producing for such a microcapsule.

### BACKGROUND

Administration of vitamin B3 [comprising nicotinic acid (NA) and nicotinamide (NAM)] has recently and surprisingly been demonstrated to have beneficial effects beyond nutritional vitamin supplementation. When delivered in controlled release formulations targeting the lower small intestine and/or colon, vitamin B3 has been shown to have beneficial effects on the intestinal microbiota, resulting in a significant amelioration of intestinal inflammation and substantial changes in the intestinal microbiota in mouse models (PCT/EP2013/062363; PCT/EP2014/077637) as well as improvement of lipid profiles in both animal models and human volunteers (PCT/EP2014/077646). The mechanism of these effects has been shown to involve signalling pathways in intestinal epithelial cells (Hashimoto et al. 2012, Nature 487:477), resulting in beneficially altered or normalised production patterns of antimicrobial peptides. Further mechanisms, *e.g.*, a surprising synergism of NAM with 5-aminosalicylic acid (PCT/EP2014/077637), are currently under investigation. The effects of NA and/or NAM on the intestinal microbiota can be harnessed not only to ameliorate pathological changes (dysbiosis), but also to prevent such dysbiosis.

Many inflammatory diseases of the intestinal wall are caused or influenced by changes in the intestinal microbiota and/or an impaired interaction between the intestinal microbiota and the intestines. Such intestinal inflammations occur in humans, *e.g.*, inflammatory bowel diseases (IBD), such as Crohn's disease or ulcerative colitis, but also in other mammals (*e.g.*, chronic idiopathic colitis in dogs). These diseases are based on complex immunological processes which are not fully understood. However, changes in, and impaired interactions of, the intestinal microbiota can also be causative factors in a number of other diseases. Examples include atopic diseases, such as atopic eczema, allergic conditions or asthma (see, *e.g.*, Bisgaard et al. 2011, J. Allergy Clin. Immunol. 128:646; lebba et al. 2011, Dig. Dis. 29:531; Abrahamsson et al. 2012, J. Allergy Clin. Immunol. 129:434; Candela et al. 2012, BMC Microbiol. 12:95; Olszak et al. 2012, Science 336:489), as well as metabolic diseases with an inflammatory component, such as atherosclerosis and/or arteriosclerosis with resulting coronary heart diseases, adiposity or diabetes (Ott et al. 2006, Circulation 113:929; Koren et al. 2011, PNAS 108 Suppl 1:4592; for reviews see Caesar et al. 2010, J. Intern. Med. 268:320, and Vrise et al. 2010, Diabetologia 53:606).

Therapeutic intervention by establishment or re-establishment of a normal intestinal microbiota or by supplementation of beneficial bacteria has been shown to be efficacious in diverse disease models and in the respective human diseases. For example, Olszak et al. (Science 2012, 336:489) recently demonstrated that the pathological accumulation of invariant natural killer T cells in diseased organs in germ-free murine models of IBD or asthma can be prevented by colonising neonate mice with normal microbiota. In different diseases, studies have demonstrated beneficial effects of certain pre-, pro- or synbiotics. In ulcerative colitis, but not in Crohn's disease, some probiotics like *E. coli* Nissle 1913 or VSL#3 (a mixture of *Bifidobacterium breve, Bifidobacterium longum, Bifidobacterium infantis, Lactobacillus acidophilus, Lactobacillus plantarum, Lactobacillus paracasei, Lactobacillus delbrueckii ssp. bulgaricus* and *Streptococcus thermophilus)* have been successfully used in a limited number of clinical studies. However, the drug development of probiotics in IBD and many other indications has largely failed proof of clinical efficacy. There are some promising data - albeit with rather small effect sizes - on lactobacillus probiotics which can reduce dyslipidemia (*e.g.*, Jones et al. 2012, Br. J. Nutr. 107:1505; Jones et al. 2012, Eur. J. Clin. Nutr. 66:1234), but the mechanism is still not completely clear (reviewed by Caesar et al. 2010, J. Intern. Med. 268:320). It appears that the supplementation of at least several strains of bacteria is usually more likely to provide significant therapeutic benefit. A recent example of spectacular efficacy of a complex bacterial intervention is the successful use of faecal microbial transplants (FMT) against Clostridium difficile (see, *e.g.*, van Nood et al. 2013, New Engl. J. Med. 368:407 and Lee et al. 2016, JAMA 315:142). In contrast, the present invention uses a more subtle approach to maintain or regenerate the body's own microbiota rather than the introduction of an alien microbial ecosystem.

NA and NAM are classified as nutrition supplements (European Commission EC regulation no. 1170/2009). The European Scientific Committee on Food has defined the tolerable upper intake level for NA in adults at 10 mg/d without anticipated side effects (SCF/CS/NUT/UPPLEV/39). However, in the pharmacological use for the treatment of dyslipidemia, clinically relevant lipid regulation was only seen in systemic doses of 2000 mg/d and above. At doses higher than 100-300 mg/d, side effects including flush, tachycardia, blood pressure dysregulation and diarrhoea have to be anticipated (Carlson 2005, J. Intern. Med. 258:94; statement no. 018/2012 of the German Federal Institute for Risk Assessment). In order to smoothen systemic uptake and to avoid high peak concentrations resulting in side effects, NA has been preferably administered in delayed or extended release formulations (*e.g.*, Niaspan®) or in combination with acetylsalicylic acid or laropiprant (*e.g.*, Tredaptive®). Such NA formulations were also the major intellectual property resulting in proprietary drugs, as unformulated NA cannot be administered in sufficient quantities. However, these drugs were recently withdrawn from the European market due to their unfavourable risk-benefit ratio resulting from significant side effects, which were largely due to systemic availability and, in the case of Tredaptive®, also to the additive laropriprant. In contrast to NA, NAM with its much better side effect profile - which is reflected by the recommended maximum nutritional doses of up to 900 mg/d for adults (SCF/CS/NUT/UPPLEV/39) - can be used without delayed or controlled release formulations (Takahashi et al. 2004, Kidney Int. 65:1099; Cheng et al. 2008, Clin. J. Am. Soc. Nephrol. 3:1131; Shahbazian et al. 2011, Nefrologia 31:58). Nevertheless, in order to optimise systemic absorption, most vitamin supplements are delayed or extended release formulations, which at least protect their contents from gastric acid.

Accordingly, the pharmacokinetics for diverse NA formulations (*e.g.*, the extended release formulation Niaspan®) or NAM formulations (*e.g.*, Nicobion® or Endur-Amide®) show quick and almost quantitative resorption and metabolisation of the drug substance in different cohorts of patients and healthy volunteers (Petley et al. 1995, Diabetes 44:152; Dragovic et al. 1995, Radiother. Oncol. 36:225; Stratford et al. 1996, Br. J. Cancer 74:16; Bernier et al. 1998, Radiother. Oncol. 48:123; Menon et al. 2007, Int. J. Clin. Pharmacol. Ther. 45:448; Reiche et al. 2011, Nephrol. Dial. Transplant. 26:276). Baseline plasma levels for NA are difficult to determine, because NA undergoes extensive, rapid and saturable first-pass metabolism with at least two separate pathways (Reiche et al. 2011, Nephrol. Dial. Transplant. 26:276; Villines et al. 2012, Curr. Atheroscler. Rep. 14:49). After administration of extended release NA, plasma Cₘₐₓ was 9.3 µg/mL in healthy volunteers after a dose of 2 g (Menon et al. 2007, Int. J. Clin. Pharmacol. Ther. 45:448) or 4.22 µg/mL in patients with chronic kidney disease after a dose of 1.5 g (Reiche et al. 2011, Nephrol. Dial. Transplant. 26:276). Baseline NAM levels in healthy volunteers without NAM supplementation have been measured in the range of 0.008-0.052 µg/mL in a cohort of 30 healthy volunteers (calibration study of a German reference laboratory: Medizinisches Labor Bremen, www.mlhb.de, 2002). A systematic pharmacokinetic comparison study in healthy human volunteers reported a plasma Cₘₐₓ of 2.1 µg/mL (after a 500-mg dose) and 16.2 µg/mL (after a 2-g dose) for the sustained release NAM formulation Endur-Amide® (Petley et al. 1995, Diabetes 44:152). In a high-dose trial with Nicobion® in radiotherapy, a 6-g dose of NAM even led to peak plasma concentrations of approximately 142 µg/mL (Bernier et al. 1998, Radiother. Oncol. 48:123). Due to hepatotoxicity concerns, such high doses are far beyond the scope of the present invention.

In contrast to the prior art in the field of delayed, extended and/or controlled release vitamin B3 formulations, which all aimed at maximal systemic exposure with minimal side effects, the beneficial modification of the interaction between the intestinal microbiota and the intestines requires completely different formulations aiming at topical exposure of the intestinal epithelium and microbiota and not at systemic availability (PCT/EP2013/062363; PCT/EP2014/077637; PCT/EP2014/077646).

However, such topical controlled release formulations of vitamin B3 are difficult to formulate due to the rapid absorption of NA and NAM, the different water solubility - which is much higher for NAM (PCT/NL2014/050388) than for NA - as well as certain disadvantageous properties of these chemicals, such as low flowability in the case of NA (data not shown) and static electricity in the case of NAM (PCT/NL2014/050388). Moreover, most pharmaceutical formulations, as described, *e.g.*, in PCT/EP2013/062363 and PCT/EP2014/077637, cannot be used as nutraceuticals, dietary supplements or functional food, which could be a mainstay of future personalised medicine and prevention. In such formulations, all additives must be approved as a food additive or have GRAS (Generally Recognised As Safe) status.

In contrast to synthetic polymers used in many pharmaceuticals, enteric coatings derived from natural components are biodegradable, relatively abundant and have no daily intake limits (Czarnocka & Alhnan 2015, Int. J. Pharm. 486:167). However, a recent comparative study concluded that "none of the GRAS-grade coatings fully complied with the different biological demands of delayed release coating systems" (Czarnocka & Alhnan 2015, Int. J. Pharm. 486:167).

In contrast to the prior art in this field, where shellac coating has been described as one option in formulations aiming at systemic delivery of NA or NAM for nutritional or medical purposes (see, *e.g.*, PCT/US1998/015990; PCT/EP2001/003192; PCT/US2008/051662; Limmatvapirat et al. 2007, Eur. J. Pharm. Biopharm. 67:690; Farag & Leopold 2011, Eur. J. Pharm. Sci. 42:400; Czarnocka & Alhnan 2015, Int. J. Pharm. 486:167), Example 6 of PCT/EP2014/077646 provides a first demonstration of a nutritional topical formulation for NA using a shellac coating developed from the work of Berg et al. (2012, J. Food Eng. 108:158). However, this example also demonstrates certain limitations, particularly a rather continuous (albeit significantly reduced compared to prior art) release of vitamin B3 under pH 1.2 and pH 6.8, underscoring that further improvement of such formulations was necessary.

Shellac is the purified form of the natural resin lac, the resinous secretion of the scale insect *Kerria lacca* (Buch et al. 2009, Drug Dev. Ind. Pharm. 35:694; Chen et al. 2011, J. Insect Sci. 11:106). Shellac has good coating properties and GRAS status (Czarnocka & Alhnan 2015, Int. J. Pharm. 486:167). Moreover shellac's dissociation is pH-dependent and possesses good resistance to gastric fluid due to its acidic character (Limmatvapirat et al. 2007, Eur. J. Pharm. Biopharm. 67:690; Czarnocka & Alhnan 2015, Int. J. Pharm. 486:167). In contrast to other food-grade enteric coatings, shellac inherently has a prolonged drug release after the pH change from the acidic gastric environment to the near-neutral pH of the small intestine, which is advantageous for the purposes of the present invention (Limmatvapirat et al. 2007, Eur. J. Pharm. Biopharm. 67:690; Czarnocka & Alhnan 2015, Int. J. Pharm. 486:167). Without further excipients or modifications, however, shellac coating with its endogenous dissolution pH of approximately 7.3 is only suitable for colonic targeting of protected substances (Farag & Leopold 2011, Eur. J. Pharm. Sci. 42:400).

A closer look at the literature on shellac-based controlled release formulations reveals different coating and subcoating strategies. Czarnocka & Alhnan (2015, Int. J. Pharm. 486:167) performed a subcoating with 10% (w/v) of the enterically inactive cellulose ether Methocel E5 (2.0% weight gain) in order to prevent interactions of the protected drug core with the enteric shellac coating. Farag & Leopold (2011, Eur. J. Pharm. Sci. 42:400) investigated different subcoatings for shellac and found, *e.g.*, that a subcoating of citric acid (with polyvinylpyrrolidone as a carrier) delayed release at pH 6.8, because the beginning swelling and dissolution of the shellac coating allowed a partial dissociation of the underlying citric acid, leading to a pH reduction at the subcoat-coat interface and, in turn, to a reduced dissociation of the acid-stabilised shellac coating. In contrast to this study, Pearnchob et al. (2004, J. Control Release 94:313) found that the addition of organic acids (sorbic, benzoic, fumaric, adipic or citric acid) as pore formers and plasticizers in ethanolic and aqueous shellac systems rather accelerated release at pH 6.8. An important aspect are coating defects, which can be limiting for the release profiles of shellac granulates. Förmer et al. (2006, Pharmazie 61:1005) prepared pellets by powder layering of ascorbic acid on nonpareil pellets with an ethanolic shellac solution as a binder and a final coating using an ethanolic shellac solution containing 10% of tartaric acid as a plasticizer, resulting in film thickness of 2-3 mm. Purified talc powder was used as an antiadherent. Interestingly, accumulated talc particles not incorporated completely into the shellac film caused surface defects and ultimately defined the release profile of the pellets (Förmer et al. 2006, Pharmazie 61:1005). Ichikawa et al. (1991, J. Pharm. Sci. 80:1062) used sodium hydrogen carbonate (sodium bicarbonate) as a carbon dioxide source in an effervescent layer below a swellable layer which contained, among several other components, also shellac. However, in contrast to the use of organic acids as described above, the sodium bicarbonate was not used for pH modification in this approach.

In summary, there are several significant disadvantages even in a professional shellac-based coating like the PROTECT™ system of Sensient Pharmaceutical (St. Louis, MO, USA), leading to failures in comparative drug release and acid disintegration resistance testing (Czarnocka & Alhnan 2015, Int. J. Pharm. 486:167). Moreover, even advantageous subcoating strategies of the prior art like the citric acid subcoating described by Farag & Leopold (2011, Eur. J. Pharm. Sci. 42:400) only led to prolonged sustained release profiles for systemic exposure.

Therefore, there is a large unmet need for - preferably nutritional - formulations, that (1) prevent or improve dysbiosis of the human intestinal microbiota and (patho)physiological states associated therewith by providing topical instead of systemic vitamin B3 and/or (2) have low production costs and/or (3) have a favourable side effect profile, also for long-term administration, as a medicament, nutraceutical, dietary supplement, food ingredient or food. In order to optimally use the topical mechanism of action, such formulations should preferably deliver copious amounts of vitamin B3 to the lower small intestine, more preferably the terminal ileum, followed by a sustained release covering a large part of the colon.

When systematically investigating shellac formulations for vitamin B3, the inventors of the present application surprisingly discovered and developed counter-intuitive coating and subcoating modifications, which specifically enabled the development of such formulations.

### SUMMARY OF THE INVENTION

The object of the present invention was to provide improved formulations and compositions for topical delivery of vitamin B3 (NA and/or NAM) to the lower small intestine, preferably the terminal ileum, and/or the colon.

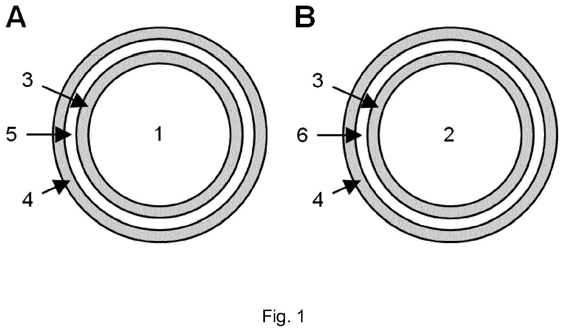

The microcapsules of the present invention (Figure 1) are characterised by a core containing vitamin B3 (1, 2) in the form of NA (A) (1) and/or NAM (B) (2) and a coating layer system comprising an inner layer of shellac (3), an outer layer of shellac (4) and a pH-modulating substance (5, 6) [A, overall basic for NA (5); B, overall acidic for NAM (6)] provided between the two layers of shellac (3, 4).

The pH-modulating substance comprises any chemical, compound, combination, composition, mixture or buffer system that may modulate the pH. It may be provided as an intermediate layer between the two shellac coating layers and has the function to fine-tune and control the disintegration of the shellac coatings. In the case of NA, the overall basic substance partially counteracts and controls the stabilising acidic effect of NA on the shellac coatings in order to enable NA release already at the pH prevalent in the lower small intestine, whereas in the case of NAM, the overall acidic substance subtly reduces the disintegration of the shellac coatings in order to achieve the desired release profile in the lower small intestine and/or colon. In all microcapsules of the present invention, the novel addition of a second (inner) layer of shellac led to a surprising and counter-intuitive improvement of the performance of the formulations.

These formulations and compositions can be used for the prophylaxis and/or therapy of human or animal diseases which are associated with unfavourable or abnormal changes or imbalances in the intestinal microbiota and/or an impaired interaction between the intestinal microbiota and the intestine.

According to the invention, the problem defined in the Background section is solved by a formulation or composition or treatment or prevention regimen as defined in the claims and described in more detail herein, which comprises vitamin B3 (NA and/or NAM) as an active substance, which beneficially influences the intestinal microbiota and their interaction with the intestines, which, in turn, prevents or ameliorates diseases in humans and/or animals. In preferred embodiments, vitamin B3 is administered to locally influence the intestinal mucosa and the intestinal microbiota. For example, the active substance is formulated to be administered selectively, *e.g.*, for at least partial topical efficacy, in the lower small intestine and/or colon, preferably in the terminal ileum and/or colon, where the intestinal microbiota to be modified are located.

In addition, compositions are provided which contain NA and/or NAM. These two active substances act individually or in combination in a beneficial manner on the microbiota in the small intestine and/or colon and/or their interaction with the intestine. A combination of NA and/or NAM and/or other active substances may be present in the same or separate dosage forms, which may be administered simultaneously, sequentially or on separate occasions. The compositions are suitable for oral administration with controlled and/or delayed release of the active ingredient(s) for specific local or topical efficacy in the lower small intestine and/or colon, preferably in the terminal ileum and/or colon. Exemplary conditions treated include therapy (using a medicament) and/or prophylaxis (using a medicament, nutraceutical, dietary supplement, food ingredient or food) of a disease and/or syndrome associated with and/or accompanied by unfavourable or abnormal or imbalanced intestinal microbiota and/or a disease and/or syndrome associated with and/or accompanied by unfavourable or abnormal or imbalanced blood and/or plasma and/or serum lipid levels, preferably in non-alcoholic fatty liver disease (NAFLD) and/or non-alcoholic steatohepatitis (NASH) for decreasing the liver fat content and/or beneficially influencing blood and/or plasma and/or serum lipid levels, and/or a disease and/or syndrome associated with and/or accompanied by intestinal inflammation, preferably in inflammatory bowel diseases, and/or other unfavourable or abnormal changes in the intestine, and/or in one or more selected from the group consisting of lipid metabolism disorders, dyslipidemia, NAFLD, NASH, cardiovascular diseases, arteriosclerosis, atherosclerosis, metabolic syndrome, obesity, diabetes, inflammatory diseases of the small intestine and/or colon, inflammatory bowel diseases, Crohn's disease, ulcerative colitis, indeterminate colitis, irritable bowel syndrome, colon carcinoma, psoriasis, allergy, atopic eczema, asthma, chronic obstructive pulmonary disease, cystic fibrosis, and other diseases and/or syndromes associated with and/or accompanied by unfavourable or abnormal or imbalanced blood and/or plasma and/or serum lipid levels and/or intestinal inflammation and/or other unfavourable or abnormal changes in the intestine and/or unfavourable or abnormal changes in the intestinal microbiota and/or an impaired interaction between the intestinal microbiota and the intestine.

The invention also includes methods of treating or preventing one or more of the diseases and conditions described herein with a microcapsule and/or composition described herein. In addition, the invention provides the use of a microcapsule and/or composition described herein in the manufacture of a medicament for treating or preventing one or more of the diseases and conditions described herein and of a dietary supplement, nutraceutical, food ingredient or food for helping to prevent one or more of the diseases and/or conditions described herein.

### BRIEF DESCRIPTION OF THE DRAWINGS

Figure 1 shows exemplary schematic representations of the microcapsules of the present invention, which are characterised by a core containing vitamin B3 (1, 2) in the form of nicotinic acid (A) (1) and/or nicotinamide (B) (2) and a coating layer system comprising an inner layer of shellac (3), an outer layer of shellac (4) and a pH-modulating substance (5, 6) [A, overall basic for nicotinic acid (5); B, overall acidic for nicotinamide (6)] provided between the two layers of shellac (3, 4).

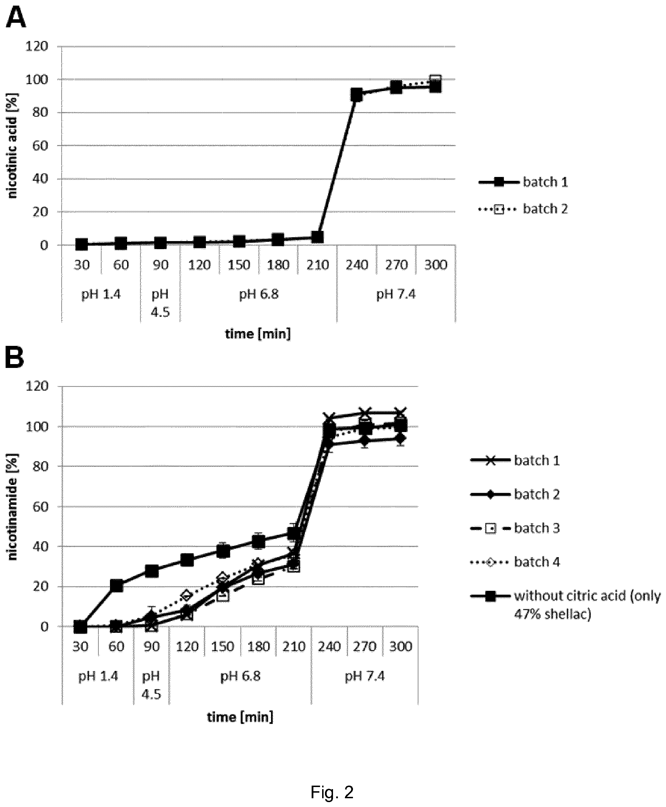

Figure 2 shows the pH-adapted release profile and batch-to-batch consistency of exemplary microcapsules of the present invention. The graphs represent means ± SD (n = 2). A, Nicotinic acid microcapsules coated with an inner coating of shellac, a pH-modulating intermediate coating of sodium bicarbonate and an outer coating of shellac. B, The graph compares one batch of nicotinamide (NAM) microcapsules with a conventional one-layered shellac coating without citric acid and four batches of NAM microcapsules with an inner coating of shellac, a pH-modulating intermediate coating of citric acid and an outer coating of shellac.

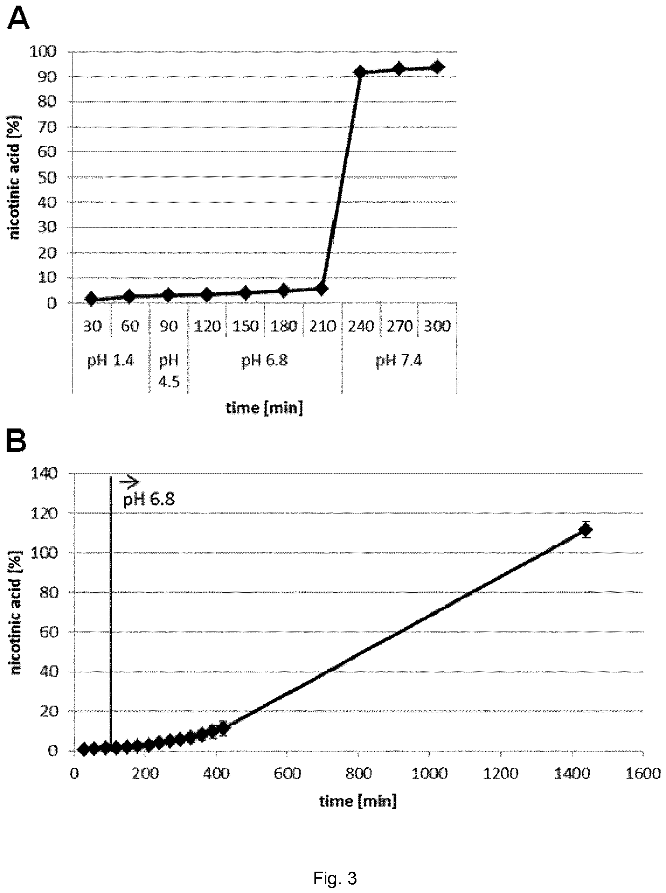

Figure 3 shows the *in vitro* release of nicotinic acid (NA) from the pooled NA microcapsule batch used for the first-in-man study. The graphs represent means ± SD (n = 3). A, Standard release profile; B, extended release profile in pH 6.8. Percentage release refers to the total mass of NA in the microcapsules (119.05 mg/g).

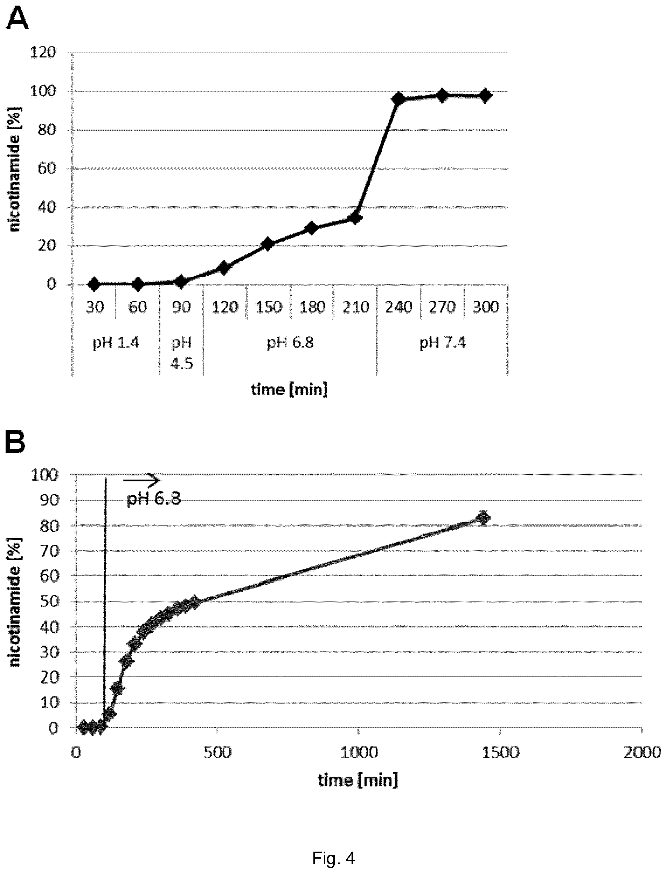

Figure 4 shows the *in vitro* release of nicotinamide (NAM) from the pooled NAM microcapsule batch used for the first-in-man study. The graphs represent means ± SD (n = 3). A, Standard release profile; B, extended release profile in pH 6.8. Percentage release refers to the total mass of NAM in the microcapsules (552.79 mg/g).

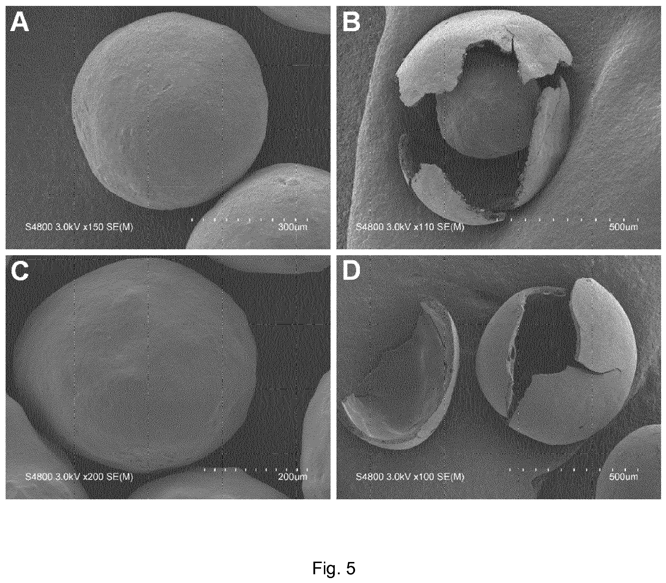

Figure 5 shows scanning electron micrographs of microcapsules before (A, C) and after (B, D) gastrointestinal transit in human volunteers of the first-in-man study. A, Nicotinic acid (NA) microcapsules before ingestion. B, NA microcapsule retrieved from stool, showing the cellet core in the opened microcapsule. C, Nicotinamide (NAM) microcapsules before ingestion. D, NAM microcapsules retrieved from stool, showing the structure of the opened coating.

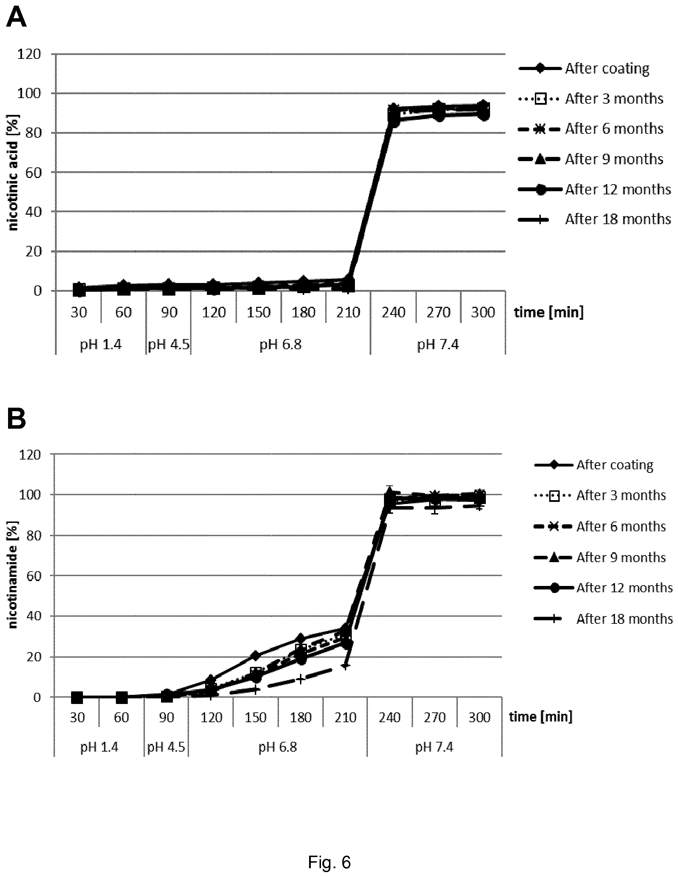

Figure 6 shows the *in vitro* release of nicotinic acid (NA; A) or nicotinamide (NAM; B) from the pooled microcapsule batches used for the first-in-man study immediately after coating and after up to 18 months of storage at room temperature and protected from light. The graphs represent means ± SD (n = 3). Percentage release refers to the total mass of NA or NAM in the microcapsules.

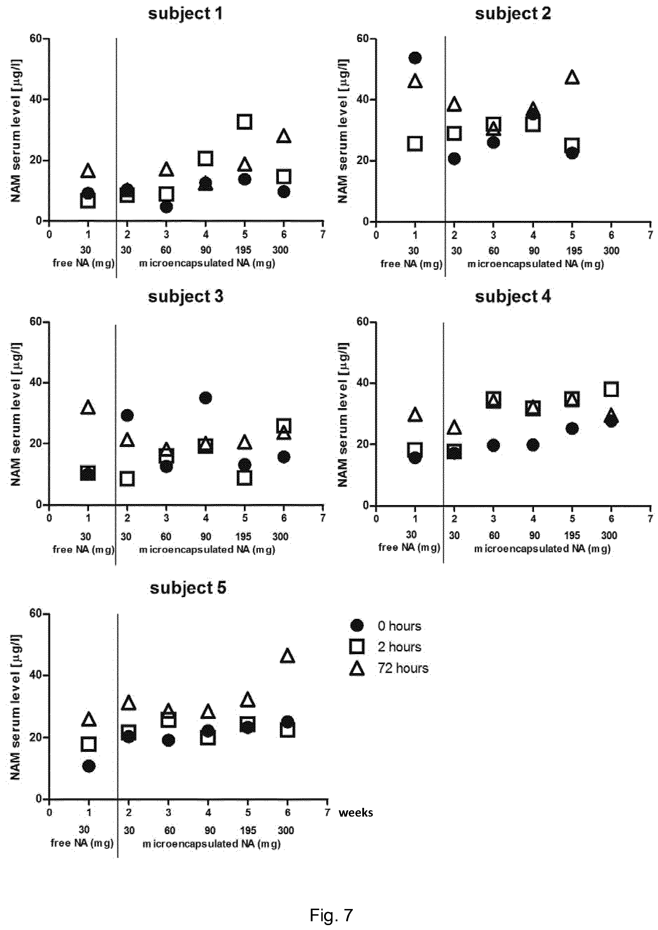

Figure 7 shows nicotinamide (NAM) serum levels of the subjects in the nicotinic acid (NA) group of the first-in-man study at 0, 2 and 72 hours. Even a ten-fold increase in the dose of microencapsulated NA did not lead to a consistent or dose-dependent increase of NAM serum levels compared to the levels observed with 30 mg of free, unformulated NA in week 1 of the study.

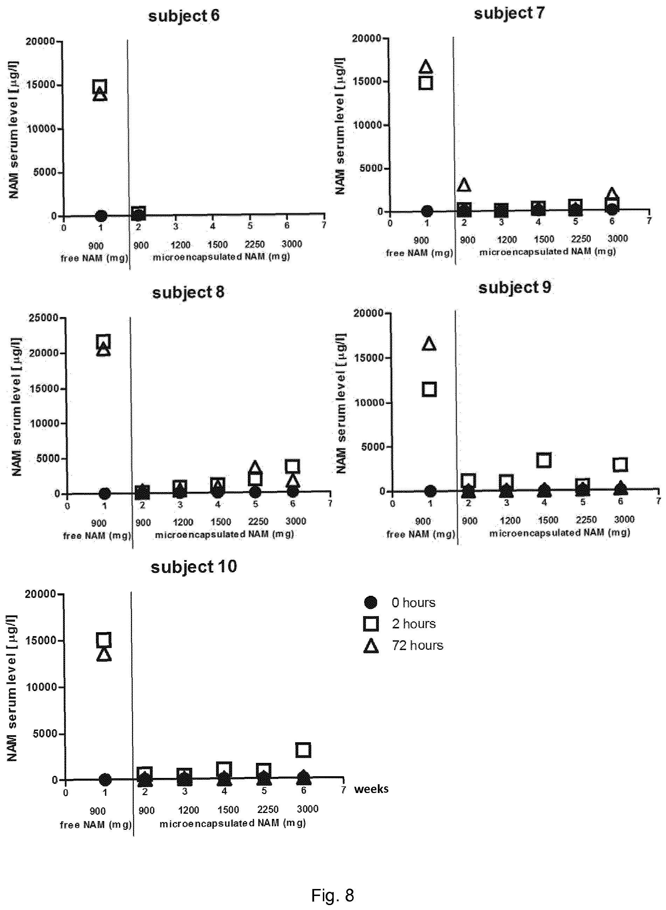

Figure 8 shows nicotinamide (NAM) serum levels of the subjects in the NAM group of the first-in-man study at 0, 2 and 72 hours. Even a 3.3-fold increase in the dose of microencapsulated NAM did not lead to a consistent or dose-dependent increase of NAM serum levels compared to the levels observed with 900 mg of free, unformulated NAM in week 1 of the study.

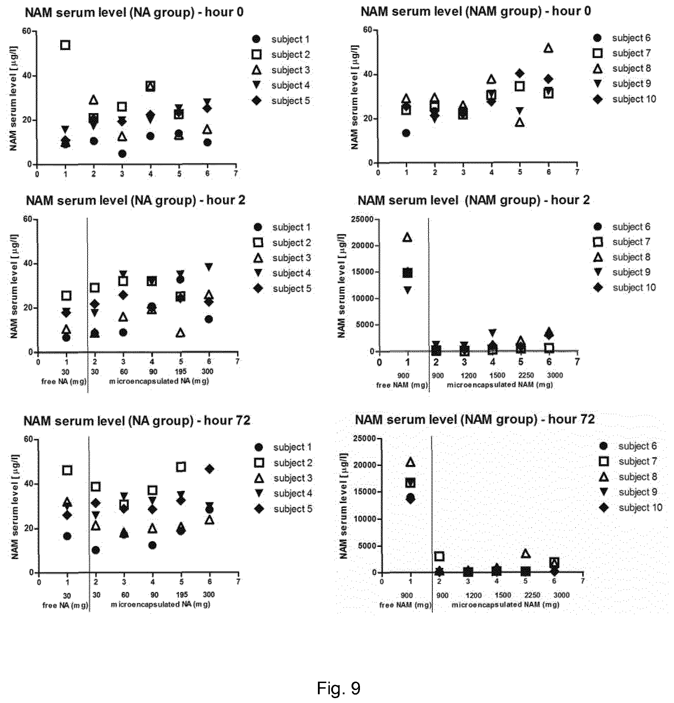

Figure 9 shows the nicotinamide (NAM) serum levels of all subjects from the nicotinic acid (NA) and NAM groups of the first-in-man study grouped by time point of acquisition.

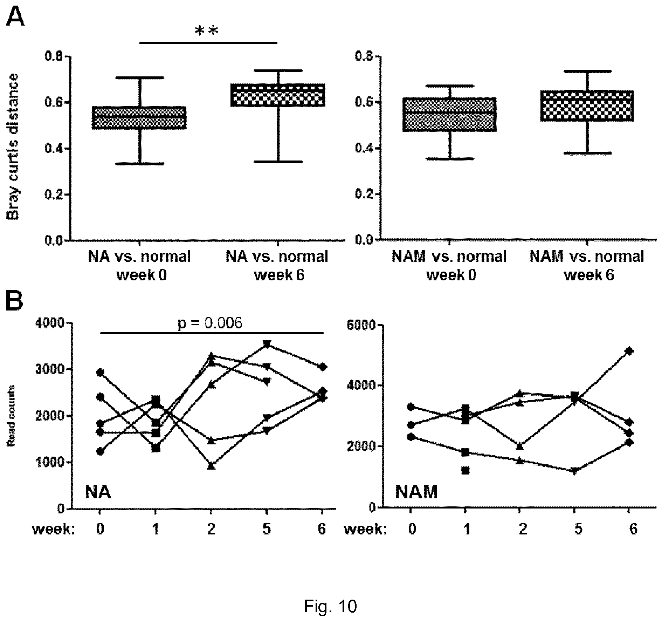

Figure 10 shows changes in microbiome parameters. A, Abundance-based bacterial communities distance (Bray-Curtis dissimilarity) between nicotinic acid (NA)- and nicotinamide (NAM)-treated subjects and a healthy normal reference group; **, p < 0.01. B, Read counts (frequency) of Bacteroidetes in the NA and NAM groups before, during and at the end of the study.

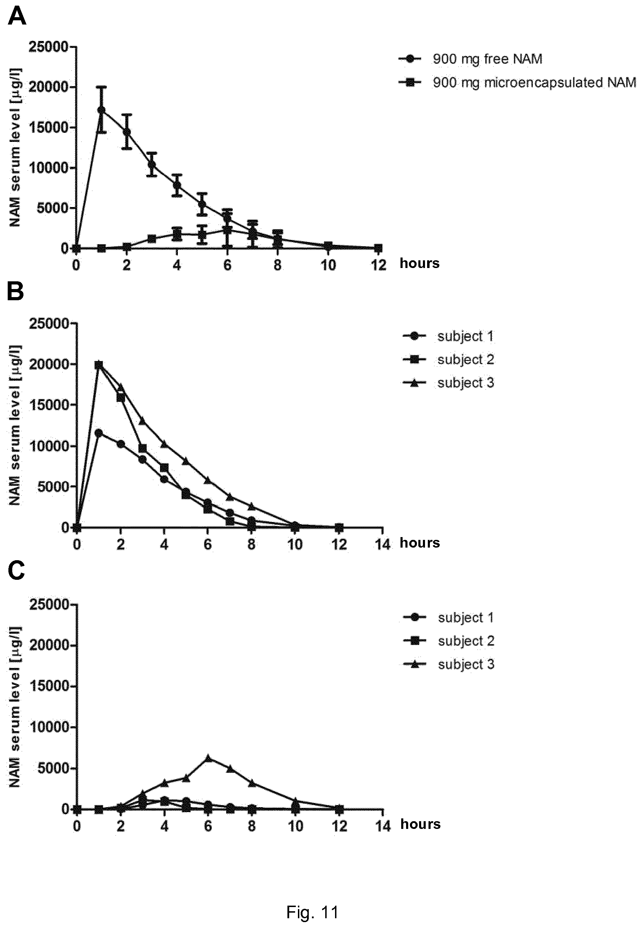

Figure 11 shows the nicotinamide (NAM) serum levels of the three subjects of the pilot NAM pharmacokinetic (PK) study. A, Comparison of NAM PK after ingestion of 900 mg of free or microencapsulated NAM in the same subjects. The graphs represent means ± SD (n = 3). B, Individual NAM PK after ingestion of free NAM. C, Individual NAM PK after ingestion of microencapsulated NAM.

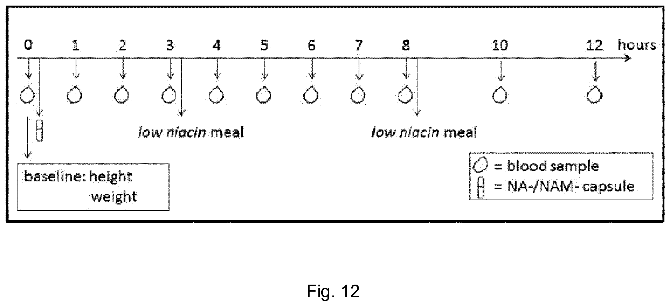

Figure 12 shows the schematic overview of a study day of the pharmacokinetic study with nicotinic acid (NA) or nicotinamide (NAM) microcapsules in healthy volunteers.

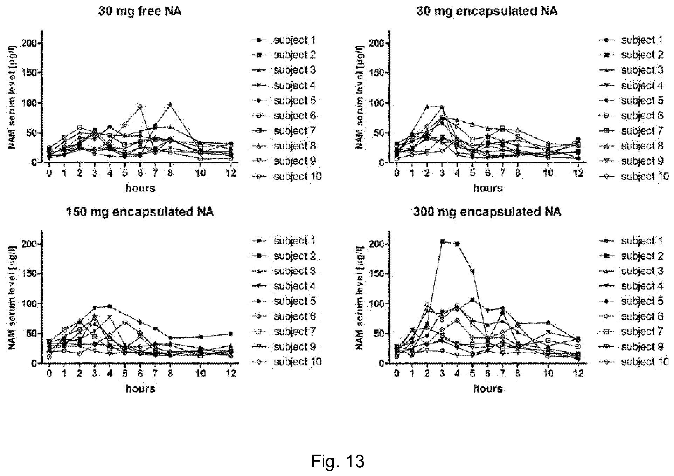

Figure 13 shows the nicotinamide (NAM) serum levels of all 10 subjects in the nicotinic acid (NA) group of the pharmacokinetic study after ingestion of 30 mg of free NA and different doses of microencapsulated NA.

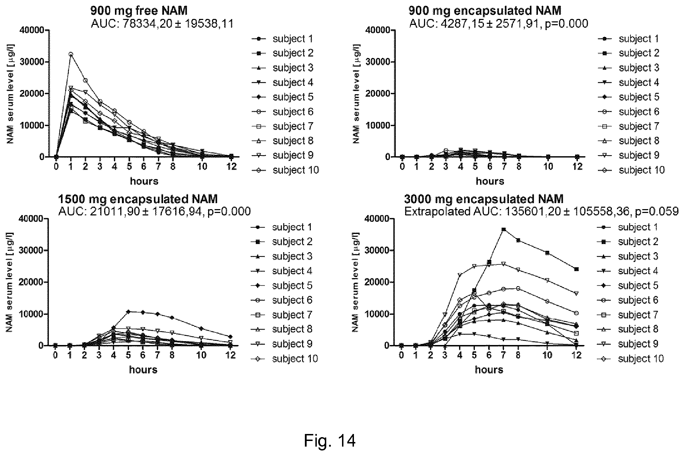

Figure 14 shows the nicotinamide (NAM) serum levels of all 10 subjects in the NAM group of the pharmacokinetic study after ingestion of 900 mg of free NAM and different doses of microencapsulated NAM. Means ± standard deviations of the area under the curve (AUC) demonstrate significantly reduced systemic exposure with microencapsulated NAM compared to free NAM. P values refer to the comparison with the AUC after ingestion of 900 mg of free NAM. For 3000 mg of microencapsulated NAM, p = 0.059 refers to the extrapolated AUCs (the p value for the non-extrapolated AUCs was p = 0.201).

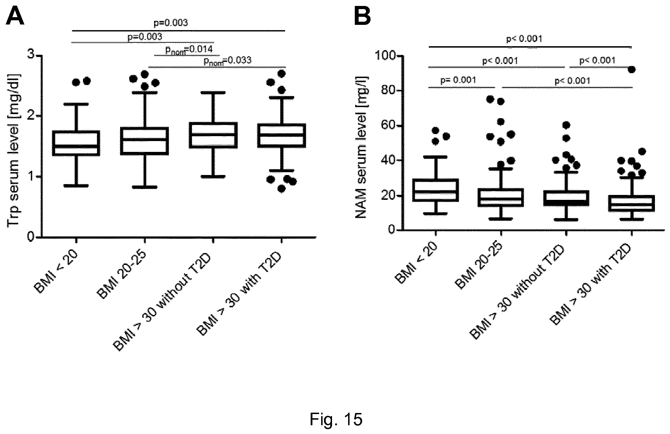

Figure 15 shows tryptophan (Trp; A) and nicotinamide (NAM; B) serum levels in a total of 511 study subjects subdivided into four groups: (1) underweight [body mass index (BMI) <20 kg/m², n = 66], (2) lean (BMI 20-25 kg/m², n = 149), (3) obese without T2D (BMI>30 kg/m², n = 148) and (4) obese with T2D (BMI>30 kg/m², n = 148). Obese and lean groups were matched by age and sex. pₙₒₘ, nomimal p values not robust to Bonferroni correction for multiple testing; the other p values presented are Bonferroni-corrected.

### DETAILED DESCRIPTION

The core of the present invention is a microcapsule comprising a core containing vitamin B3 (1, 2) [comprising its two chemical forms NA (1) and/or NAM (2)], characterised by a coating layer system comprising an inner layer of shellac (3), an outer layer of shellac (4) and a pH-modulating substance (5, 6) provided between the two layers of shellac (3, 4).

Herein, the term "vitamin B3" refers to NA and/or NAM, both alone or in combination.

Herein, the term "pH-modulating substance" comprises any chemical, compound, combination, composition, mixture or buffer system that may modulate the pH. In the present invention, the pH-modulating substance has the function to fine-tune and control the disintegration of the shellac coatings. The pH-modulating substance may be provided with or without excipients, e.g., as an intermediate layer between the two shellac coating layers. Combinations of two or more pH-modulating chemicals, also divergent in nature (*e.g.*, acidic and/or basic substances with different pKa and/or pKb, combinations of weak and strong acids and/or bases) are also within the scope of the present invention. Therefore, the terms "basic substance" or "acidic substance" as used herein are not limiting in that such a substance should contain only basic or acidic components, respectively, but refer to the overall effect and properties of the pH-modulating substance. For example, such a substance may also comprise components which may be pH-neutral, basic (in the case of an overall acidic substance) or acidic (in the case of an overall basic substance).

Herein, the words "preferred" or "preferably" refer to embodiments that may have certain benefits under certain circumstances, but other embodiments may also be preferred under the same or other circumstances. The recitation of one or more preferred embodiments does not imply exclusion of other useful embodiments from the scope of the invention. Terms like "comprises" and variations thereof do not have a limiting meaning in the description and claims. Citation of certain sections of documents from the literature does not imply that the rest of such documents is not relevant or not incorporated by reference. The recitations of numerical ranges by one or two endpoints includes all numbers subsumed within that range (*e.g.*, "1 to 10" includes 1, 2.4, 4.576, etc., and "lower than 1" includes all numbers smaller than 1). For any method disclosed or cited herein that includes discrete steps, the steps may be conducted in any feasible order, and any combination of two or more steps may be conducted simultaneously. Any example or list of examples should not be interpreted as a restriction of any kind or as an exclusive list.

In the case that the vitamin B3-containing core of the microcapsule comprises or consists of more NA than NAM, the pH-modulating substance is overall basic to partially counteract and control the stabilising acidic effect of NA on the shellac coatings in order to enable vitamin B3 release already at the pH prevalent in the lower small intestine (preferably in the terminal ileum), with or without a prolonged release in the colon. In addition to the dual shellac layer, the use of a basic pH-modulating substance is also novel over the state of the art. Components of such basic pH-modulating substances are preferably selected from hydrogen carbonate, carbonate and/or acetate salts, *e.g.*, sodium hydrogen carbonate (sodium bicarbonate), sodium carbonate, potassium hydrogen carbonate (potassium bicarbonate), potassium carbonate, ammonium hydrogen carbonate (ammonium bicarbonate), ammonium carbonate, calcium hydrogen carbonate (calcium bicarbonate), calcium carbonate, sodium acetate and/or calcium acetate.

Thus, one embodiment of the microcapsule according to the present invention is characterised in that vitamin B3 comprises or consists of NA, and the pH-modulating substance comprises or consists of a basic substance, preferably selected from the group consisting of hydrogen carbonate, carbonate salts, acetate salts, and their mixtures. In a preferred embodiment, the basic substance comprises or consists of sodium hydrogen carbonate (sodium bicarbonate).

In the case that the vitamin B3-containing core of the microcapsule comprises or consists of more NAM than NA, the pH-modulating substance is overall acidic to subtly reduce the disintegration of the shellac coatings, resulting in prolonged shellac stability until reaching the lower small intestine, a burst release of vitamin B3 in the lower small intestine (preferably in the terminal ileum) and optionally a continued release in the colon. Components of such acidic pH-modulating substances are preferably selected from organic and/or inorganic acids, *e.g.*, citric acid, malic acid, tartaric acid, ascorbic acid, sorbic acid, lactic acid, acetic acid and/or phosphoric acid.

Thus, one embodiment of the microcapsule according to the present invention is characterised in that vitamin B3 comprises or consists of NAM, and the pH-modulating substance comprises or consists of an acidic substance, preferably selected from the group consisting of organic acids, inorganic acids, and their mixtures. In a preferred embodiment, the acidic substance comprises or consists of citric acid.

In the case that the vitamin B3-containing core of the microcapsule comprises or consists of both NA and NAM, the properties of the pH-modulating substance preferably depend on the composition and overall pH properties of the core.

The present invention also refers to a method for producing a microcapsule and/or a composition as described herein, comprising the steps of
a) providing a core material comprising or consisting of vitamin B3,
b) coating the core material with a first shellac layer,
c) providing a pH-modulating substance,
d) applying the pH-modulating substance onto the first shellac layer, *e.g.* in the form of an intermediate layer, and
e) applying a second shellac layer.

The core comprising or consisting of vitamin B3 may be produced, *e.g.*, by granulation of NA and/or NAM with or without excipients and/or other substances, or it may contain an excipient structure (*e.g.*, a Cellet core) onto or into which NA and/or NAM with or without excipients and/or other substances are applied, *e.g.*, by spray coating. Furthermore, a core comprising or consisting of NA and/or NAM may also be coated with NA and/or NAM. NA and/or NAM may be used in any form available on the market, *e.g.*, produced by Merck KgaA.

For producing the core, the shellac layers and/or the pH-modulating substance, numerous excipients can be used, *e.g.*, but not limited to maltodextrin, glycerol, cellulose ethers [*e.g.*, hydroxypropylmethylcellulose (HPMC), hydroxypropylcellulose (HPC), methylcellulose, ethylcellulose, and/or carboxymethylcellulose], polyethylene oxide, carbopol polymers, polyacrylic acid, polysaccharides (*e.g.*, xanthan gum, guar gum, chitosan, alginate, pectin, carrageenan, tragacanth, and/or different types of starch, *e.g.* pea starch and/or rice starch), polyvinyl alcohol, polyvinylpyrrolidone and/or silicon dioxide. Two non-limiting examples for such procedures and the resulting microcapsules are provided in Example 1.

Depending on the size, structure and weight of the core, the amount of shellac and/or pH-modulating substance(s) applied in each step influences the percentage weight gain and the resulting size and properties of the microcapsule. The smaller the core and the more uneven its surface structure, regardless of its inherent composition and structure, the more surface it has in relationship to its volume, and the more shellac may have to be applied in relationship to the weight of the core in order to form an inner shellac layer with the desired properties. In addition, the shellac layers and the pH-modulating substances may be applied in different amounts, thicknesses and percentage weight gains, depending, *e.g.*, on the intended release profile, the species of the subject which shall ingest the microcapsule (human or animal, see below), and/or the structure and composition of the core and the coating materials used (*e.g.*, the particular properties and specifications of the shellac batch, pH-modulating substance and/or excipients). Herein, the percentage weight gain of each process step relates to the weight of the starting material used in this process step. For example, a weight gain of 10% by applying the outer shellac layer means that the microcapsules produced by this process step have 10% more weight than the starting material of this step, which already comprises the core, the inner shellac layer and the pH-modulating substance.

In the case that the vitamin B3-containing core structure of the microcapsule comprises or consists of NA, the percentage weight gain resulting from applying the inner shellac layer is 0.1-100%, preferably 0.25-90%, more preferably 0.5-80% and most preferably 2-60%. In the case that NA is sprayed onto a core structure, e.g., a Cellet core, the percentage weight gain resulting from applying the inner shellac layer is 0.1-100%, preferably 0.2-50%, more preferably 0.3-40% and most preferably 0.5-30%. The percentage weight gain of applying the pH-modulating substance is 0.1-30%, preferably 0.1-25% and most preferably 0.2-20%. Finally, the percentage weight gain resulting from applying the outer shellac layer is 0.1-100%, preferably 0.5-80%, more preferably 1-60% and most preferably 2-40%.

In the case that the vitamin B3-containing core structure of the microcapsule comprises or consists of NAM, the percentage weight gain resulting from applying the inner shellac layer is 0.1-100%, preferably 0.25-90%, more preferably 0.5-80% and most preferably 2-60%. In the case that NAM is sprayed onto a core structure, *e.g.*, a Cellet core, the percentage weight gain resulting from applying the inner shellac layer is 0.1-100%, preferably 0.2-50%, more preferably 0.3-40% and most preferably 0.5-30%. The percentage weight gain of applying the pH-modulating substance is 0.1-30%, preferably 0.1-25% and most preferably 0.2-20%. Finally, the percentage weight gain resulting from applying the outer shellac layer is 0.1-100%, preferably 0.5-80%, more preferably 1-60% and most preferably 2-40%.

In the case that the vitamin B3-containing core structure of the microcapsule comprises or consists of both NA and NAM, the percentage weight gain resulting from applying the inner shellac layer is 0.1-100%, preferably 0.25-90%, more preferably 0.5-80% and most preferably 2-60%. In the case that NA and/or NAM are sprayed onto a core structure, e.g., a Cellet core, the percentage weight gain resulting from applying the inner shellac layer is 0.1-100%, preferably 0.2-50%, more preferably 0.3-40% and most preferably 0.5-30%. The percentage weight gain of applying the pH-modulating substance is 0.1-30%, preferably 0.1-25% and most preferably 0.2-20%. Finally, the percentage weight gain resulting from applying the outer shellac layer is 0.1-100%, preferably 0.5-80%, more preferably 1-60% and most preferably 2-40%.

The present invention also comprises use of the microcapsule as a medicament, nutraceutical, dietary supplement, food ingredient or food.

In particular, the present invention relates to use of the microcapsule for beneficially influencing the intestinal microbiota and/or for beneficially influencing or preventing unfavourable and/or abnormal and/or imbalanced blood and/or plasma and/or serum lipid levels and/or for beneficially influencing or preventing intestinal inflammation and/or other unfavourable and/or abnormal changes in the intestine, wherein the microcapsule releases vitamin B3 for at least partial topical efficacy in the lower small intestine, preferably in the terminal ileum, and/or in the colon.

It has been previously demonstrated that NAM has a surprising anti-inflammatory effect by influencing the intestinal microbiota (the entirety of all microorganisms in the intestines, in particular the bacteria) (PCT/EP2013/062363; PCT/EP2014/077637). The mechanism behind this surprising effect has subsequently been shown to involve NAM-induced changes in the secretion patterns of antimicrobial peptides in the intestines, which support the maintenance and/or regeneration of the normal and/or healthy intestinal microbiota (Hashimoto et al. 2012, Nature 487:477).

Thus, as used herein, "beneficially influencing the intestinal microbiota" refers to causing a change in the intestinal microbiota that has a beneficial impact on health, especially on one or more of the diseases and conditions described herein, and/or to maintaining the healthy intestinal microbiota in preventive settings. For example, beneficial impacts may be associated with reducing the number of pathogenic bacteria, reducing the ratio of pathogenic bacteria to beneficial bacteria, increasing the diversity of the microbiota, increasing the amount of beneficial bacteria, partly or completely reverting pathological changes in the enterotype of the microbiota (*e.g.*, enterotypes associated with *Bacteroides, Prevotella* and *Ruminococcus*) and/or maintaining the healthy endogenous microbiota. In the context of IBD, bacteria generally regarded as pathogenic include *Enterobacteriaceae* (*e.g., Escherichia coli*) with invasive properties or virulence factors, sulphide-producing *Desulfovibrio* spp. and *Fusobacterium* spp with invasive properties, whereas bacteria generally regarded as beneficial include species from the genera *Lactobacillus, Bifidobacterium* and *Faecalibacterium,* such as *L. casei, L. plantarum* and *F. prausnitzii* (Manichanh et al. 2012, Nat. Rev. Gastroenterol. Hepatol. 9:599). Marchesi et al. (Gut 65:330, 2016) stated that "a definitive change of the normal gut microbiota with a breakdown of host-microbial mutualism is probably the defining event in IBD development", with the most consistent changes being a reduction in *Firmicutes* and an increase in certain *Proteobacteria.* The same authors have reviewed emerging therapies targeting the intestinal microbiota in various diseases also within the scope of the present invention, such as metabolic syndrome and obesity-related diseases, liver diseases, IBD and colorectal cancer. For example, intestinal dysbiosis can cause NAFLD, and probiotics can reduce liver aminotransferases and total cholesterol, while improving insulin resistance in patients with NAFLD (Lata et al. 2011, World J. Gastroenterol. 17:2890; Ma et al. 2013, World J. Gastroenterol. 19:6911; Marchesi et al. 2016, Gut 65:330). A particularly interesting study demonstrated a causal influence of intestinal microbiota on human metabolism by showing that FMT from lean donors to recipients with metabolic syndrome significantly increased their insulin sensitivity, faecal butyrate concentrations and microbial diversity (Vrieze et al. 2012, Gastroenterology 143:913).

As used herein, the term "topical efficacy" refers to a topical effect in the pharmacological sense, and thus refers to a local, rather than systemic, target for a medication. As mentioned supra, e.g., in view of hepatotoxicity concerns and other side effects, high doses for high systemic availability as used in the prior art formulations are beyond the scope of the present invention. In contrast, the mode of administration and the dosage according to the invention minimise the probability for the occurrence of the well-known side effects of NA and/or NAM. Accordingly, topical or local efficacy means a local therapy and/or prophylaxis of an active substance specifically or selectively to a location where, for example, the medication or nutraceutical or dietary supplement or food ingredient shall deliver its direct therapeutic and/or prophylactic effect, while entering the circulatory system to a significantly lower extent than the unformulated active substance, *e.g.*, thereby not causing any or only a comparably low systemic exposure and/or action. In this regard, the topical efficacy of the present invention is also contrasted with enteral (in the digestive tract) and intravascular/intravenous (injected into the circulatory system) administrations aiming at systemic exposure. In comparison to compositions aiming at high systemic availability and/or exposure, the topical efficacy of compositions may also be characterised by longer latency times until systemic levels of the active substance(s) increase. Such latency times for topical release can be correlated with intestinal transit times known in the art (see, *e.g.*, Davis et al. 1986, Gut 27:886; Evans et al. 1988, Gut 29:1035; Kararli 1995, Biopharm. Drug Dispos. 16:351; Sutton 2004, Adv. Drug Deliv. Rev. 56:1383). For example, after a variable time for gastric emptying (depending on the dosage form and feeding status and ranging from less than 1 hour to more than 10 hours), small intestinal transit times are rather constant with usually 3-4 hours across formulations and studies (Davis et al. 1986, Gut 27:886). Thus, an exemplary latency time in a fasted patient would be, *e.g.,* 2 hours, at which time a formulation may reach the small intestine and systemic levels may start to rise. In the context of the present invention, topical efficacy preferably means that blood and/or plasma and/or serum levels of the active substance and/or metabolites thereof do not exceed levels which are preferably three orders (more preferably two orders, most preferably one order) of magnitude higher than the levels measured in the same person before dosing. Alternatively or additionally, topical efficacy can be expressed in terms of a reduction of the blood and/or plasma and/or serum levels of at least 50%, 60%, 70%, 80%, 90% or even 95% or more relative to the same amount of active substance administered purely (without a formulation) in the same way and under the same conditions. In a preferred embodiment, average blood and/or plasma and/or serum levels of a suitable cohort of persons are used for these definitions of topical efficacy rather than the respective levels of single persons, which can yield highly divergent results (see Example 4).

As used herein, the terms "systemic exposure" is defined such that blood and/or plasma and/or serum levels of the active substance and/or metabolites exceed levels which are three orders of magnitude higher than the levels measured in the same person before dosing. Accordingly, a low systemic exposure is defined such that blood and/or plasma and/or serum levels of the active substance and/or metabolites do not exceed levels which are three orders of magnitude higher than the levels measured in the same person before dosing. Thus, in contrast to the prior art, the present invention is designed for a topical efficacy by means of beneficially modifying the intestinal microbiota and their interaction with the intestines, *e.g.*, locally in the lower small intestine, preferably in the terminal ileum, and/or in the colon, while particularly showing no or low systemic exposure.

As used herein, the "lower small intestine" is the second half (length) of the small intestine, and the "terminal ileum" is the second half (length) of the ileum.

The inventive, specific, topical use of vitamin B3 for locally influencing the intestinal mucosa and the intestinal microbiota, and the direct therapy of their interactions or, *e.g.*, the prophylaxis of disturbances in their interactions, result from the insights into the formerly unknown and unexpected role of vitamin B3, as described in PCT/EP2013/062363, PCT/EP2014/077637 and PCT/EP2014/077646. This use significantly differs from conventional uses of vitamin B3, where vitamin B3 is supposed to be absorbed and to act systemically. The main advantages of the present invention therefore are to avoid unnecessary systemic exposure, to reduce doses by delivering lower amounts of vitamin B3 to the actual sites of efficacy and, consequently, to reduce or avoid systemic side effects by reducing or avoiding systemic uptake of vitamin B3.

As used herein, "beneficially influencing or preventing unfavourable and/or abnormal and/or imbalanced blood and/or plasma and/or serum lipid levels" refers to at least one lipid parameter as defined in the current guidelines of the European Society of Cardiology and/or the European Atherosclerosis Society (2011, Eur. Heart J. 32:1769) and/or in the current guidelines of the American Association of Clinical Endocrinologists (Jellinger et al. 2012, Endocr. Pract. 18 Suppl. 1:1; Jellinger et al. 2012, Endocr. Pract. 18:269) and/or in the current guidelines of the American College of Cardiology and the American Heart Association (Stone et al. 2014, Circulation 129 suppl. 2:S1). Of note, the topical efficacy for beneficially influencing blood and/or plasma and/or serum lipid levels indirectly by beneficially modifying the intestinal microbiota and/or their interaction with the intestines as described in PCT/EP2014/077646 is independent from the previously found anti-inflammatory topical effect. Hence, PCT/EP2014/077646 particularly shows the said topical efficacy also in treatments for the therapy and/or prophylaxis of, *e.g.*, unfavourable or abnormal or imbalanced blood and/or plasma and/or serum lipid levels and/or metabolic diseases in humans and animals, in which a large-scale tissue inflammation (*e.g.*, like in IBD) is not necessarily the dominant or only disease-driving mechanism. Accordingly, the microcapsules of the present invention are also suitable in the treatment and/or prophylaxis in patients or subjects that do not have major and/or relevant signs and/or symptoms of an intestinal inflammation. Thus, as used herein, a "beneficial effect" on blood and/or plasma and/or serum lipid levels refers to changing the blood and/or plasma and/or serum levels of one or more blood lipids from a state of dyslipidemia partially or completely towards the reference levels observed in healthy individuals, which are matched to the diseased individuals in terms of, *e.g.,* age, sex, body weight, medication, etc. In another embodiment, said "beneficial effect" may also be the partial or complete prevention of the development of unfavourable and/or abnormal and/or imbalanced blood and/or plasma and/or serum lipid levels. Such beneficial effects are preferably an increase in high density lipoprotein (HDL) if HDL levels are below the reference levels, a decrease in low density lipoprotein (LDL) if LDL levels are above the reference levels, a decrease in triglycerides if triglyceride levels are above the reference levels, and/or a decrease in total cholesterol if total cholesterol levels are above the reference levels. In another preferred embodiment, the beneficial effect is to keep said or other lipid parameters within the reference level range of healthy individuals.

On account of its anti-inflammatory effect and/or its beneficial effects on the intestinal microbiota (PCT/EP2013/062363; PCT/EP2014/077637), vitamin B3 is also suitable as active substance for treating inflammatory diseases of the small intestine and/or colon. Particular conditions include the treatment of intestinal inflammations such as IBD, Crohn's disease, ulcerative colitis or indeterminate colitis, and the therapy or prophylaxis of other diseases that result from changes in the intestinal microbiota and/or an impaired interaction between the intestinal microbiota and the intestines. For example, as chronic intestinal inflammation and/or dysbiosis strongly increases the risk of developing colon carcinoma (for review see *e.g.*, Ullman & Itzkowitz 2011, Gastroenterology 140:1807; Marchesi et al. 2016, Gut 65:330), the microcapsules according to the invention may also be used in the prophylaxis of colon cancer.

As dysbiosis and/or pathological changes in the intestinal microbiota can also play a role in numerous other diseases ranging from atopic disorders to metabolic diseases, the therapy and/or prophylaxis of such diseases is also within the scope of the invention. Therefore, the present invention also comprises use of the microcapsules in the therapy (using a medicament) and/or prophylaxis (using a medicament, nutraceutical, dietary supplement, food ingredient or food) of
a disease and/or syndrome associated with and/or accompanied by unfavourable or abnormal or imbalanced intestinal microbiota and/or
a disease and/or syndrome associated with and/or accompanied by unfavourable or abnormal or imbalanced blood and/or plasma and/or serum lipid levels, preferably in non-alcoholic fatty liver disease (NAFLD) and/or non-alcoholic steatohepatitis (NASH), for decreasing the liver fat content and/or beneficially influencing blood and/or plasma and/or serum lipid levels, and/or
a disease and/or syndrome associated with and/or accompanied by intestinal inflammation, preferably in inflammatory bowel diseases (IBD), and/or
other unfavourable or abnormal changes in the intestine, and/or
one or more selected from the group consisting of lipid metabolism disorders, dyslipidemia, NAFLD, NASH, cardiovascular diseases, arteriosclerosis, atherosclerosis, metabolic syndrome, obesity, diabetes, inflammatory diseases of the small intestine and/or colon, IBD, Crohn's disease, ulcerative colitis, indeterminate colitis, irritable bowel syndrome, colon carcinoma, psoriasis, allergy, atopic eczema, asthma, chronic obstructive pulmonary disease, cystic fibrosis, and other diseases and/or syndromes associated with and/or accompanied by unfavourable or abnormal or imbalanced blood and/or plasma and/or serum lipid levels and/or intestinal inflammation and/or other unfavourable or abnormal changes in the intestine and/or unfavourable or abnormal changes in the intestinal microbiota and/or an impaired interaction between the intestinal microbiota and the intestine.

As used herein, the terms "therapy", "treatment" and "treat" refer to reversing, alleviating, delaying the onset of, or inhibiting the progress of a disease or disorder, or one or more symptoms thereof, as described herein. In some embodiments, such treatment may be administered after one or more symptoms have developed. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence, *e.g.* in remission maintenance of a chronic relapsing disorder.

As used herein, the terms "prophylaxis", "prevention" and "prevent" refer to delaying the onset of or reducing the likelihood of developing a disease or disorder or one or more symptoms thereof, as compared to an untreated control population.

For the sake of clarity, dyslipidemias can be hypolipidemias (*e.g.*, if high density HDL levels are too low) or hyperlipidemias (*e.g.*, if LDL levels are too high) or a combination of hypo- and hyperlipidemias of two or more lipids or lipoproteins in the blood and/or plasma and/or serum. The lipid metabolism disorders and dyslipidemias described herein include genetic and non-genetic forms of such conditions or diseases or disorders. A genetical predisposition includes, but is not limited to, risk genotypes in the SLCO1B1, ABCG2 or ABCB1 genes. Dyslipidemia can be accompanied by high lipoprotein (a) [Lp (a)] levels and/or alterations in homocysteine levels. The patients referred to in this context include, but are not limited to, patients with statin intolerance. In this context, the microcapsules of the present invention are particularly preferred for use in the following three indications: (1) general dyslipidemia, especially as an additive to statins (*e.g.*, to increase HDL, which is not sufficiently provided by statins, such as atorvastatin, cerivastatin, fluvastatin, lovastatin, mevastatin, pitavastatin, pravastatin, rosuvastatin or simvastatin), (2) dyslipidemia in patients with high Lp (a) and statin intolerance (including, but not limited to, patients with SLCO1B1, ABCG2 and/or ABCB1 risk genotypes) and (3) NAFLD (non-alcoholic fatty liver disease) and/or NASH (non-alcoholic steatohepatitis), preferably in NAFLD and/or NASH for decreasing the liver fat content and/or beneficially influencing blood and/or plasma and/or serum lipid levels. In addition, in one embodiment, the microcapsules of the present invention are particularly preferred for use as nutraceuticals, dietary supplements or food ingredients for subjects with a dyslipidemia that does not yet require medical treatment, but is a risk factor for developing one or more of the above diseases.

The claimed microcapsules are equally usable for the therapy and/or prophylaxis of diseases and/or syndromes with similar genesis in both human and other mammals, in particular in domestic and useful animals. Examples of such animals are dogs, cats, horses, camels, cattle or pigs without objective restriction.

In addition, the present invention also refers to a composition comprising a microcapsule of the invention. In a preferred embodiment, such a composition is formulated for oral administration with controlled and/or delayed release of vitamin B3 so that it releases (*e.g.*, partially releases, selectively releases) for at least partial topical efficacy in the lower small intestine, preferably in the terminal ileum, and/or in the colon.

In the present invention, the terms "formulation" or "composition" or "treatment" or "prevention", and in particular the term "composition", have a broad meaning of a pharmaceutically and/or nutritionally and/or physiologically acceptable formulation, composition and/or mode of administration of the said active substance(s), which includes, but is not limited to, pharmaceutical formulations in the sense of medicaments (drugs), nutraceuticals, dietary supplements, food ingredients and/or foods, also depending on the dose of the active substance(s) and the nature of the formulation. Preferred are medicaments, nutraceuticals, and dietary supplements.

The term "controlled release" refers preferably to a pharmaceutical formulation or component thereof that releases, or delivers, one or more active ingredients over a prolonged period of time (time-dependent release) and/or under certain physiological conditions (*e.g.*, pH-dependent release). In certain embodiments, the period of time or the release according to physiological conditions (e*.g.*, pH) is sufficient for at least a portion of the active substances in a formulation to release in the lower small intestine (*e.g.*, in the terminal ileum) and/or in the colon.

The term "delayed release" relates preferably to a pharmaceutical formulation that releases the active ingredients after a period of delay. In certain embodiments, the delay is sufficient for at least a portion of the active substances in a formulation to release in the lower small intestine (*e.g.*, in the terminal ileum) and/or in the colon.

Depending on the nature and severity of the disease or condition as well as individual patient or subject characteristics, the dosage forms are administered once or several times daily, or in another dosage regimen to be chosen by a physician in the case of medicaments or, in the case of nutraceuticals and/or dietary supplements, as defined by the instructions to the consumer. The total dosage of NA and/or NAM used according to the invention can be in the range of from 1 to 5000 mg. Suitable total dosage ranges of NA and/or NAM comprise of from 10 to 5000 mg, preferably of from 30 to 3000 mg.

As a non-limiting example, a high dose composition can comprise up to 5000 mg of NA, NAM or a combination thereof. For example, a fixed-dose high dose composition can comprise a total of NA, NAM or a combination thereof in the range of 3000-5000 mg, *e.g.,* 4000 mg.

As a non-limiting example, a low dose composition can comprise up to 1000 mg, *e.g.*, 30-900 mg, of NA, NAM or a combination thereof.

As a non-limiting example, a standard dose composition can comprise up to 3000 mg of NA, NAM or a combination thereof, and preferably is in a range of from 1000-3000 mg, more preferably in the range of from 1500-2500 mg.

If a combination of NA and NAM is used, the ratio between NA and NAM can vary, depending on the composition and/or the application. Non-limiting particular examples of fixed-dose combination compositions with a 1:10 ratio of NA/NAM are 300 mg NA and 3000 mg NAM for a high dose composition, 150 NA and 2000 mg NAM for a standard dose composition and 30 mg NA and 900 mg NAM for a low dose composition. However, as stated above, other high, standard or low dose compositions with other ratios of NA and NAM are also within the scope of the invention.

Accordingly, the present invention also relates to a composition, characterised in that the oral application is performed with an active substance content for NA and/or NAM of 1-5000 mg each per finished dosage form, preferably with an active substance content of 30-3000 mg each per finished dosage form.

For oral administration, the microcapsules of the invention may, for example, be administered in free form (e.g., mixed with food, beverages, probiotics, prebiotics or synbiotics), formulated in simple or coated tablets or dragees together with further excipients, or filled into capsules or sachets. The tablets are usually round or biconvex. Oblong tablet forms, which allow the tablet to be separated, are also possible.

The composition can also contain further excipient substances, such as binders, fillers, glidants, lubricants and/or flow regulating agents. The compositions according to the invention can be formulated, where appropriate, together with further active substances and with excipients conventional in pharmaceutical and/or nutritional compositions, *e.g.*, talcum, gum arabic, lactose, starch, magnesium stearate, cocoa butter, aqueous and non-aqueous carriers, lipid components of animal or vegetable origin, paraffin derivatives, glycols (in particular polyethylene glycol), various plasticizers, dispersants, emulsifiers, preservatives and/or other excipients as, *e.g.*, specified in the Examples below.

Accordingly, the present invention also relates to a composition for use for beneficially influencing the intestinal microbiota and/or for beneficially influencing or preventing unfavourable and/or abnormal and/or imbalanced blood and/or plasma and/or serum lipid levels and/or for beneficially influencing or preventing intestinal inflammation and/or other unfavourable and/or abnormal changes in the intestine, and/or for use in the therapy (using a medicament) and/or prophylaxis (using a medicament, nutraceutical, dietary supplement, food ingredient or food) of
a disease and/or syndrome associated with and/or accompanied by unfavourable or abnormal or imbalanced intestinal microbiota and/or
a disease and/or syndrome associated with and/or accompanied by unfavourable or abnormal or imbalanced blood and/or plasma and/or serum lipid levels, preferably in non-alcoholic fatty liver disease (NAFLD) and/or non-alcoholic steatohepatitis (NASH), for decreasing the liver fat content and/or beneficially influencing blood and/or plasma and/or serum lipid levels, and/or
a disease and/or syndrome associated with and/or accompanied by intestinal inflammation, preferably in inflammatory bowel diseases (IBD), and/or
other unfavourable or abnormal changes in the intestine, and/or
one or more selected from the group consisting of lipid metabolism disorders, dyslipidemia, NAFLD, NASH, cardiovascular diseases, arteriosclerosis, atherosclerosis, metabolic syndrome, obesity, diabetes, inflammatory diseases of the small intestine and/or colon, IBD, Crohn's disease, ulcerative colitis, indeterminate colitis, irritable bowel syndrome, colon carcinoma, psoriasis, allergy, atopic eczema, asthma, chronic obstructive pulmonary disease, cystic fibrosis, and other diseases and/or syndromes associated with and/or accompanied by unfavourable or abnormal or imbalanced blood and/or plasma and/or serum lipid levels and/or intestinal inflammation and/or other unfavourable or abnormal changes in the intestine and/or unfavourable or abnormal changes in the intestinal microbiota and/or an impaired interaction between the intestinal microbiota and the intestine.

In another preferred embodiment, the present invention also comprises combination preparations of (a) the active substances of the present invention, such as variable dose combinations or fixed dose combinations of NA and NAM (see above), and (b) either NA or NAM or a combination thereof together with one or more other active substance(s). The combinations described herein may be present in the same or separate dosage forms, which may be administered simultaneously, sequentially or on separate occasions.

While it is preferred according to the invention that the composition and/or the microcapsule according to the invention comprise both the vitamin B3 component (NA and/or NAM) and one or more additional components, it may be beneficial to have separate compositions each comprising one or more of the active ingredients (vitamin B3 and/or one or more additional components) to be administered simultaneously or sequentially under a treatment or prevention regimen. Of note, such a set of compositions is per definition for this application a composition according to the invention, too.

For clarification, the inventors use the following definitions:
Probiotic: ingestible live microbial cultures, which survive transit through the gastrointestinal tract and beneficially affect the host by improving its intestinal microbial balance. An example for a probiotic is VSL#3 (see above).

Prebiotic: non-digestible and selectively fermented food ingredient or supplement that allows specific changes in the composition and/or activity of the gastrointestinal microbiota which are beneficial for host well-being and health. Examples for prebiotics are resistant starch, fructo-oligosaccharides, galacto-oligosaccharides, xylooligosaccharides, polydextrose, lactulose, inulin or soluble fibre (*e.g.*, psyllium husk or acacia fibres).

Synbiotics: a combination of pro- and prebiotics.

As used herein, the term "variable dose combination" refers to a combination of two or more active substances in medicaments, nutraceuticals or dietary supplements, whereby each of these substances is applied in the form of a separate composition, *e.g.*, two single dosage forms. The separate compositions may be administered simultaneously, sequentially or on separate occasions by an administration regimen. In a preferred embodiment, microcapsules of the invention containing either NA or NAM may be combined in variable dose combinations. In another preferred embodiment, a vitamin B3 microcapsule in any suitable dosage thereof may be administered simultaneously, sequentially or on separate occasions with a separate composition of another active substance in any suitable dosage thereof. Thus, variable dosages of vitamin B3 may be combined with variable dosages of any other active substance. These variable dose combinations may use conventionally available compositions or may be also achieved, *e.g.*, by customised polypharmacy or compounding.

In contrast to a variable dose combination, a "fixed-dose combination" is defined as a combination medicament, nutraceutical or dietary supplement which is a formulation including two or more active ingredients, *e.g.*, active substances, combined in a single dosage form, which is manufactured and distributed in certain respective fixed doses. A fixed-dose combination mostly refers to a mass-produced product having a predetermined combination of active substances and respective dosages (as opposed to, *e.g.*, customised polypharmacy or compounding). For example, microcapsules of the invention containing either NA or NAM may be combined in fixed dose combinations by including a specific ratio of these microcapsules in a larger dosage form, *e.g.*, a capsule, tablet or sachet.

Thus, the microcapsules of the present invention can be formulated to comprise combinations of NA and/or NAM and/or other substances, but the invention also encompasses combinations of vitamin B3 microcapsules with other active substances and/or compositions. For example, the components may also be physically segregated even within the same dosage form, *e.g.* by adding vitamin B3 microcapsules to a probiotic, a prebiotic, a synbiotic or any other substance or composition, or by filling vitamin B3 microcapsules according to the invention and one or more controlled or delayed release formulations, *e.g.* microcapsules or granules, of other active substances into one capsule for easier administration. Thus, the invention also pertains to a composition, which is a controlled and/or delayed release formulation of vitamin B3 (NA and/or NAM) alone, or a variable dose combination or, preferably, a fixed dose combination of a controlled and/or delayed release formulation of vitamin B3 with a probiotic, a prebiotic, a synbiotic or any other substance, such substance being preferably formulated for controlled and/or delayed release.

Therefore, the present invention also relates to microcapsules and compositions comprising variable and or fixed dose combinations of NA and/or NAM and/or one or more other active substances and/or compositions, for microcapsules preferably within the core of the capsule, and preferably being selected from the group of probiotics; prebiotics; synbiotics; polyphenols; substances, proteins and/or enzymes supporting probiotics; antibiotic, antimycotic, antiprotozoal, antihelminthic, antiviral or anti-inflammatory agents; aminosalicylates; acetylsalicylic acid; prostaglandin D2 antagonists, preferably laropiprant; short-chain fatty acids; medium-chain fatty acids; systemic or topical corticosteroids; β2-adrenergic receptor agonists; theophylline and other substances of the xanthine family; statins, preferably selected from the group consisting of atorvastatin, cerivastatin, fluvastatin, lovastatin, mevastatin, pitavastatin, pravastatin, rosuvastatin and simvastatin; small molecules; peptides, biologicals, fusion proteins, monoclonal antibodies or derivatives thereof.

A further aspect of the invention described herein is the efficient use of the claimed medicaments, nutraceuticals, dietary supplements, food ingredients or foods on the basis of genetic and/or microbiological and/or blood parameter and/or other biomarker data and specific needs of the individuals to be treated. For example, new insights into the genetic predisposition of individuals for all types of diseases (in particular also diseases where the interaction between the intestinal microbiota and the intestine is impaired) and into pharmacogenetics indicate that an evidence-based personalised medicine including genetic analyses of relevant risk genes and also of genes which code e.g., for cell surface receptors, transporter proteins, metabolism enzymes or signal transduction proteins, which interact with an active substance and/or its metabolites and/or its downstream effectors, can contribute information and improvements with respect to the type of use, the mode of application, the time(s) of use, the dose and/or the dosage regimen of the medicaments, nutraceuticals, dietary supplements, food ingredients or foods described herein. Individuals who may benefit from this personalised treatment include those with disease-specific or non-specific changes in blood and/or plasma and/or serum lipids and/or other biomarkers. This applies analogously to analyses of the intestinal microbiota, particularly when a stool sample indicates a change in the microbiota. The present invention thus also comprises the use of suitable genetic and/or microbiological and/or blood parameter and/or other biomarker test methods to identify individuals particularly susceptible to or benefiting from the medicaments, nutraceuticals, dietary supplements, food ingredients or foods according to the invention and/or to adapt their use according to the invention to the individual circumstances. This also comprises expressly the use of the chemical forms of vitamin B3 (NA and/or NAM) or their combinations with other active substances in different modes of administration depending on the genetic and microbiological properties of the individual. For these purposes, it is possible to use laboratory tests and/or suitable test kits and also measuring methods, devices and/or kits to be employed by a physician, user and/or patient, e.g., to take stool samples or to analyse suitable parameters in the blood, urine or other body fluids.

In particular, the present invention also relates to using the intestinal microbiota in part and/or in their entirety (the microbiome) as biomarkers to identify beneficial microbiota and/or detrimental microbiota, to support patient or subject selection for the treatments or preventions described herein, to personalise and adapt the compositions and/or treatments and/or preventions described herein, and/or to determine end points and efficacy benchmarks for the compositions and/or treatments and/or preventions described herein.

### EXEMPLIFICATION

There are variable possibilities to advantageously develop, and develop further, the teaching of the present invention. For this purpose, reference is made to the examples below which describe the invention in a representative way.

If not indicated otherwise, the meaning of "%" is "% by weight".

### Example 1: Production and characterisation of NA and NAM microcapsules

One embodiment of the present invention is that cores comprising the active substances NA and/or NAM are coated by two layers of shellac, which are separated by an intermediate layer of a pH-modulating substance (Figure 1). The pH-modulating substance is overall basic in the case of a core containing mainly NA (Figure 1A) and overall acidic in the case of a core containing mainly NAM (Figure 1B). In the present example, the basic pH-modulating substance in NA microcapsules was sodium bicarbonate and the acidic pH-modulating substance in NAM microcapsules was citric acid.

### Equipment and materials

**Table 1: Equipment used for the preparation and characterisation of the microcapsules**

| | |
|---|---|
| Capsule filler | Aponorm® capsule filler for 60 capsules with size 0 plate set; WEPA Apothekenbedarf, Hillscheid, Germany |
| Dissolution tester | Model DT 70; Pharmatest Group, Hainburg, Germany |
| Fluidized bed coater | Mini Glatt; Glatt Ingenieurtechnik, Binzen, Germany |
| Fluidized bed granulator | ProCell Labsystem with Vario 3; Glatt Ingenieurtechnik, Binzen, Germany |
| High vacuum sputter coater | Leica EM SCD 500; Leica Microsystems, Wetzlar, Germany |
| Leit-C conductive carbon cement | Neubauer; Münster, Germany |
| Quartz cuvette | SUPRASIL®; Type-No. 100-QS; 10 mm; Heraeus, Hanau, Germany |
| Scanning electron microscopy | Hitachi S-4800 SEM; Hitachi High-Tech, Tokyo, Japan |
| Spectrophotometer | Helios Gamma, UVG145021; Thermo Fisher Scientific, Dreieich, Germany |

**Table 2 (part 1): Materials used for the preparation of the microcapsules and capsules**

| | |
|---|---|
| Cellets | Cellets® 350 (350-500 µm), microcrystalline, pelletized cellulose, batch no. 13G043; iPc Process Center, Dresden, Germany |
| Citric acid monohydrate | Citric acid monohydrate for food use, test sample; Jungbunzlauer, Basel, Switzerland |
| Gelatin capsules | Coni-Snap® capsules, yellow, size 0, batch no. 34037611; Capsugel, Morristown, NJ, USA |
| Glycerol | Glycerol 85%, batch no. 5113Q-01589; Mohren-Apotheke, Kiel, Germany |
| Hydroxypropylmethylcellulose (HPMC) | Coating for NA: AnyAddy® AN6, batch no. AFF006-320010; Harke Pharma/Food, Mülheim a. d. Ruhr, Germany; Glatt granulation: Pharmacoat 606; Shin-Etsu Chemical Co., Tokyo, Japan |
| Lycoat™ (pea starch) | Lycoat RS 780, LAB 3819, batch no. E004S; Roquette, Lestrem, France |
| Maltodextrin | C* Dry maltodextrin 01915, batch no. 02023411; Cargill, Minneapolis, MN, USA |

**Table 2 (part 2): Materials used for the preparation of the microcapsules and capsules (continued)**

| | |
|---|---|
| Nicotinic acid (NA) | Nicotinic acid, 10V2031; batch no. 165004; SternVitamin, Ahrensburg, Germany |
| Nicotinamide (NAM) | Nicotinamide, 10V2002; batch no. 169256; SternVitamin, Ahrensburg, Germany |
| Shellac | 0560 0003, SSB® Aquagold, 25% shellac ammonium salt, batch no. 168920; Stroever, Bremen, Germany |
| Sodium bicarbonate | Sodium bicarbonate (E500), batch no. L098; Dr. August Oetker Nahrungsmittel, Bielefeld, Germany |

**Table 3: Materials used for the preparation of the dissolution buffers**

| | | |
|---|---|---|
| Citric acid anhydrous | X863.2, >99.5%, batch no. 452193486 | Carl Roth, Karlsruhe, Germany |
| Di-sodium hydrogen phosphate dihydrate | 4984.1, >99.5%, batch no. 175226790 | |
| Hydrochloric acid | 4625.1, 37%, batch no. 433206404 | |
| Potassium dihydrogen phosphate | 3904.1, >99%, batch no. 234214969 | |
| Sodium chloride | 3957.2, >99.5%, batch no. 483205341 | |
| Tri-sodium citrate dihydrate | 3580.3, >99%, batch no. 123186150 | |

### Methods

### Preparation of coating solutions

The composition of each formulation part is listed in Table 4. The ingredients were dissolved at room temperature under moderate stirring in tap water (H₂O).

**Table 4: Composition of the coatings and NAM granules**

| **Coating** | **Ingredient 1** | **%** | **Ingredient 2** | **%** | **Ingredient 3** | **%** | **H₂O %** |
|---|---|---|---|---|---|---|---|
| **NA coating** | Nicotinic acid | 9.3 | Hydroxypropylmethylcellulose (HPMC) | 0.7 | - | - | 90 |
| **NAM granulation** | Nicotinamide | 26 | HPMC | 4 | - | - | 70 |
| **pH coating NA** | Sodium bicarbonate | 2.3 | Lycoat™ RS 780 (pea starch) | 4.6 | Glycerol (85%) | 0.3 | 92.8 |
| **pH coating NAM** | Citric acid | 1 | Maltodextrin | 9 | - | - | 90 |
| **Inner and outer shellac coatings** | Shellac SSB® Aquagold (25%) | 60 | - | - | - | - | 40 |

### Production of NA microcapsules

First, a layer of NA and HPMC was applied to 175 g of Cellets 350 in a Mini Glatt fluid bed coater with bottom spray using a 0.5 mm two-way nozzle and an atomizing air pressure of 0.5-0.67 bar. The inlet air pressure was adjusted to 0.4 bar, and the inlet air temperature was set to 39°C, which resulted in a product temperature of about 32.4°C. The final weight gain was about 16%. The following coatings, *i.e.*, the inner shellac coating, the sodium bicarbonate intermediate coating and the outer shellac coating, were all applied subsequently under the following conditions with 200 g of the NA/HPMC-coated Cellets: atomizing air pressure of 0.6-0.72 bar, inlet air pressure of 0.3-0.38 bar, inlet air temperature of 40-41°C, product temperature of about 33.7°C and an increasing spraying rate from 0.43 to 0.87 g/min. After the last shellac coating step, the microcapsules were dried at 50°C in a drying oven for 1 h. The calculated weight gains were 2% for the inner shellac coating, 0.98% (referred to sodium bicarbonate alone) or 3.1% (referred to the total dry mass increase including sodium bicarbonate, Lycoat™ pea starch and glycerol) for the pH-modulating sodium bicarbonate coating and 19% for the outer shellac coating. The measured total weight gain for the inner shellac layer, the pH-modulating sodium bicarbonate layer and the outer shellac layer was 20% (approximately 5% loss compared to the added calculated weight gains of 25.08%).

### Production of NAM microcapsules

For producing the NAM granule cores, a 30% NAM solution including 4% pharmacoat 606 (HPMC) in water was prepared, 300 g maltodextrin DE 15 were provided as starting material, and a continuous granulation process was performed in a ProCell fluidized bed granulator with a Vario 3 continuous fluidized bed insert (Glatt, Binzen, Germany). The average size of the resulting granules was 354.6 µm. In the next step, the inner shellac coating was applied to 150 g of the NAM granules (diameter 315-400 µm) in a Mini Glatt fluid bed coater with bottom spray (Glatt, Binzen, Germany) using a 0.5 mm two-way nozzle and an atomizing air pressure of 0.54-0.62 bar. Inlet air pressure was adjusted to 0.33-0.45 bar, and the inlet air temperature was set to 39-41°C, which resulted in a product temperature of about 33.8°C. The spraying rate was increased from 0.37 to 1 g/min. The final weight gain was about 47%. After this first shellac coating step, the microcapsules were dried at 50°C in a drying oven for 1 h and sieved (250 µm) to remove dust. The citric acid intermediate coating was applied under the following conditions to 184-200 g of the shellac-coated NAM granules: atomizing air pressure of 0.48-0.63 bar, inlet air pressure of 0.33-0.5 bar, inlet air temperature of 40-41°C, product temperature of about 35.8°C and an increasing spraying rate from 0.37 to 0.57 g/min. The calculated weight gain was 1% (referred to citric acid) and 10% (referred to total dry mass including maltodextrin). The outer shellac coating was applied under the same conditions as the inner shellac coating except for the inlet air pressure, which was higher (0.47-0.53 bar) because of the weight gain. The calculated weight gain was 10% for the outer shellac coating (referring to the weight of the microcapsules after the first shellac coating step), and the measured combined weight gain for the citric acid and outer shellac layers was a total of 18% (2% loss compared to the added calculated weight gains of 20%). After the second shellac coating step, the microcapsules were again dried at 50°C in a drying oven for 1 h.

### In vitro dissolution testing

Dissolution tests were performed at 37°C with 0.5 g of NA or NAM microcapsules in 250 ml simulated gastric fluid (pH 1.4; Table 5), citrate buffer (pH 4.5; Table 5) or phosphate buffers (pH 6.8 or 7.4; Table 5) using a standard paddle apparatus at 100 rpm. Dissolution experiments were run for 1 h at pH 1.4, 0.5 h at pH 4.5, 2 h at pH 6.8 and 1.5 h at pH 7.4. Extended dissolution tests were run for 1 h at pH 1.4, 0.5 h at pH 4.5 and 22.5 h at pH 6.8. Active substance release was recorded spectrophotometrically at 262 nm every 30 min using Quartz cuvettes. Before each measurement, samples were diluted (NA: 1:5; NAM: 1:20).

**Table 5: Composition of buffers for the in vitro dissolution tests**

| **Buffer** | **pH** | **Ingredient 1** | **Amount or %** | **Ingredient 2** | **Amount o**r **%** | **Solvent** | **Vol.** |
|---|---|---|---|---|---|---|---|
| **Simulated gastric fluid** | 1.4 | Sodium chloride | 2g | Hydrochloric acid (6 M) | 7 ml | dH₂O* | ad 1 L |
| **Citrate buffer** | 4.5 | Citric acid monohydrate | 4.8 g | Tri-sodium-citrate dihydrate | 7.35 g | dH₂O* | ad 1 L |
| **Phosphate buffer** | 6.8 | Potassium dihydrogen phosphate (0.91%)* | 50.8% | Di-sodium hydrogen phosphate dihydrate (1.19%)* | 49.2% | - | - |
| **Phosphate buffer** | 7.4 | Potassium dihydrogen phosphate (0.91%)* | 18.2% | Di-sodium hydrogen phosphate dihydrate (1.19%)* | 81.8% | - | - |

| | | | | | | | |
|---|---|---|---|---|---|---|---|
| * Solvent: demineralised water (dH₂O). | | | | | | | |

### Determination of the total content of NA or NAM in coated microcapsules

In order to calculate the percentage of active substance release, the total content of NA or NAM in the microcapsules was determined. For this purpose, 0.2 g of microcapsules from each batch were crushed, 100 ml phosphate buffer (pH 7.4) was added, and the mixture was left to incubate for 30 min without stirring to solubilise the shellac. After 30 min, the mixture was filtered using MN615 paper filters (pore size: 4-12 µm; Macherey-Nagel, Düren, Germany) to remove debris, diluted (NA: 1:5; NAM: 1:20) and measured spectrophotometrically at 262 nm. The total amount of NA or NAM in the pooled microcapsule batches used for the first-in-man (FIM) and PK studies (see Examples 2 to 4) was determined spectrophotometrically at 262 nm with 0.5 g microcapsules in 250 ml phosphate buffer (pH 7.4) in a standard paddle apparatus at 37°C and 100 rpm. After 30, 60 and 90 minutes, samples were taken, diluted (NA: 1:5; NAM: 1:20) and measured. For enhanced precision and to validate the in-house content analyses, microcapsules from these pooled batches were additionally analysed by liquid chromatography and mass spectrometry (LC-MS/MS; Agilent 1100 HPLC/ CTC-PAL Autosampler/Sciex API 4000 Triple Quadrupole) at an external reference laboratory (Medizinisches Labor Bremen, www.mlhb.de). For this purpose, 10 mg of fine blended microcapsules were added to 10 ml solvent (NA: methanol:H₂O 10:90 [v/v]; NAM: phosphate buffer pH 7.4), put in a ultrasonic bath and gently shaken for 30 min. Before the measurements, samples were diluted (NA: 1:1000; NAM: 1:2000).

### Capsule filling

The NA or NAM microcapsules were filled into standard size 0 gelatin capsules using a manual capsule filler for 60 capsules. A maximum of approximately 0.53 g of microcapsules fit into one gelatin capsule. In the FIM study, differential dosing was achieved by administration of differential amounts of full or partially filled capsules.

### Scanning electron microscopy

In order to visualise the microcapsule structures and the release of NA or NAM, microcapsules were mounted on a holder with Leit-C conductive carbon cement. Subsequently, they were sputter-coated with a layer of 8-10 nm gold-palladium using either a Leica EM SCD 500 high-vacuum sputter coater or with the internal sputter coater of the microscope and examined in a Hitachi S-4800 scanning electron microscope (SEM) at an accelerating voltage of 3 kV. Microcapsules from stool samples of human volunteers were washed in demineralised water and dried overnight before mounting and analysing them as described above.

### Results and discussion

### Characterisation of NA microcapsules in vitro

Shellac has a comparatively high dissolution pH of about 7.3, which is unsuitable for the desired substantial topical release in the terminal ileum (pH 6.8 to pH 7.4) (Limmatvapirat et al. 2007, Eur. J. Pharm. Biopharm. 67:690). Moreover, the acidic character of NA further reduces shellac dissociation (see Background section) and, thus, NA release. Even with a thin (2% weight gain) inner shellac coating together with the pH-modulating sodium bicarbonate layer according to the present invention, a satisfactory burst release profile for strong topical exposure of the terminal ileum was achieved (Figure 2A). During the application of the sodium bicarbonate layer, microcapsules were highly electrostatically charged and tended to stick to the coaters' wall, which may compromise subcoating efficacy if not corrected properly. Nevertheless, batch-to-batch consistency was very high (Figure 2A). The total NA content of the microcapsules as determined spectrophotometrically was 114.7 mg NA/g for batch 1 and 112.8 mg NA/g for batch 2.

### Characterisation of NAM microcapsules in vitro

As shown in Figure 2B, NAM microcapsules with conventional shellac coating (47% referred to the total mass of the coating) did not show gastric resistance (>10% NAM release at pH 1.4). It was hypothesised that the pH value of NAM (pH 6.61) resulted in a faster release of active substance. Therefore, a pH-modulating layer of citric acid was applied to reduce the intrinsic pH value in order to achieve both gastric resistance and a prolonged release profile (see Figure 2B). In contrast to the prior art using a citric acid layer with polyvinylpyrrolidone below a shellac coating (Farag & Leopold 2011, Eur. J. Pharm. Sci. 42:400), the inner shellac coating according to the invention led to a prolonged resistance also at pH 6.8 with a burst release under conditions found in the terminal ileum and an extended release of the residual NAM thereafter (Figure 2B). Mixing citric acid and shellac for coating is impracticable, as the shellac coagulates due to the low pH. Batch-to-batch consistency of the four batches of the first production campaign was very high (Figure 2B). The total NAM content of the microcapsules as determined spectrophotometrically was 509.0 mg NAM/g for batch 1, 554.3 mg NAM/g for batch 2, 523.3 mg NAM/g for batch 3 and 523.7 mg NAM/g for batch 4; the NAM content of the control batch without citric acid subcoat was 638.4 mg NAM/g.

A comparison between the burst release profile obtained for NA with a thin inner shellac coating (2% weight gain; Figure 2A) and the prolonged release profile obtained for NAM with a thicker inner shellac coating (47% weight gain; Figure 2B) illustrates how the release profiles of the microcapsules of the invention can be fine-tuned by adjusting the thickness of the inner shellac coating and the type and amount of pH-modulating substances, which solves an important problem of shellac coatings reported in the state of art (Czarnocka & Alhnan 2015, Int. J. Pharm. 486:167). In this context, the pH properties of the active substance in the core also play a stabilising (NA: acidic) or disintegration-promoting role (NAM: rather neutral), which can be counteracted according to the desired release profile by the pH-modulating substances of the intermediate layer.

### Characterization of NA and NAM microcapsules for the FIM study

For the FIM study in human healthy volunteers (see Example 2), the microcapsule batches described above were pooled. The total NA content of the NA microcapsules as determined spectrophotometrically was 119.05 ± 0.76 mg of NA per gram of microcapsules (mean ± SD; n = 3), and the total NAM content of the NAM microcapsules was 552.79 ± 4.73 mg NAM/g microcapsules (mean ± SD; n = 3). In order to validate these in-house measurements with a different method, an external reference laboratory measured these parameters using LC-MS/MS. The total NA content of the NA microcapsules as determined by LC-MS/MS was 88.4 ± 2.8 mg NA/g microcapsules (mean ± SD; n = 2), and the total NAM content of the NAM microcapsules was 525.33 ± 35.05 mg NAM/g microcapsules (mean ± SD; n = 6), thus yielding comparable results.

The release profiles of the NA and NAM microcapsule pools used in the FIM study are shown in Figures 3 and 4, respectively. In the B panels of Figures 3 and 4, long-term incubation experiments in buffer with pH 6.8 are shown to demonstrate the prolonged stability of the formulations under such conditions. SEM pictures of the surface of coated microcapsules showed no defects and a smooth surface structure (Figures 5A and 5C). After gastrointestinal passage in the human volunteers, the microcapsules isolated from stool were open and showed a porous and spongy surface and profile (Figures 5B and 5D).

In order to ensure comparability of exposure and results during the FIM study, the stability of the NA or NAM content (determined by spectrophotometry; mean ± SD; n = 3) and the release profiles of the microcapsules were monitored (Figure 6). The NA content of the NA microcapsules after 5 months of storage at room temperature and protected from light (119.18 mg ± 0.11 NA/g microcapsules) was practically identical to the NA content measured immediately after coating (119.05 ± 0.76 mg NA/g, see above). Likewise, the NAM content of the NAM microcapsules after 5 months (562.18 ± 3.47 mg NA/g) was practically the same as immediately after coating (552.79 ± 4.73 mg NA/g). As shown in Figure 6, also the release profiles did not change significantly over time up to 18 months of storage. After 18 months of storage, the release of NAM at pH 6.8 was slightly slower compared to the measurements at 12 months or before (Figure 6B). Taken together, the analysis of the characteristics and stability of the NA and NAM microcapsules clearly supported their suitability for topical controlled release in the FIM study.

### Example 2: First-in-man (FIM) dose escalation study with NA and NAM microcapsules

### Aims of the study

In the FIM study, size 0 gelatin capsules filled with well-characterised pooled batches of NA or NAM microcapsules (see Example 1) were administered as a dietary supplement to 5 healthy human volunteers for each active agent. The aims of the study were (1) to investigate the difference of systemic exposure to NA or NAM between unformulated NA or NAM and the respective microcapsules, (2) to determine the maximum administrable dose in terms of exposure and safety by dose escalation and (3) to analyse whether the short-term exposure of the human subjects already resulted in any changes in the intestinal microbiota, as observed previously in other contexts and with other formulations (PCT/EP2013/062363; PCT/EP2014/077637; PCT/EP2014/077646).

### Methods

### Study population and design

The study was performed at the Department of Internal Medicine 1 of the University Hospital Schleswig-Holstein, Campus Kiel (Kiel, Germany). The study was approved by the ethics committee of the University of Kiel (reference number: D439/15). Written informed consent was obtained from each volunteer.

Ten healthy volunteers were included and allocated to 2 groups of 5 subjects each. One group received NA and the other group received NAM. The baseline characteristics of the study population are summarised in Table 6. Subject 2 (NA group) had to be excluded after week 5 due to elevated aspartate transaminase (AST) levels, and subject 6 (NAM group) dropped out in week 2 due to an accident not associated with the study.

The design of the study is summarised in Tables 7 and 8. In this dose escalation study, subjects ingested the test items in the morning from Monday to Thursday of every week, followed by a three-day washout period from Friday to Sunday (Table 7). Before and until 30 min after administration (or until after the second blood sampling on Mondays), subjects fasted with water only. During week 1, the subjects took one daily dose of 30 mg unformulated NA or 900 mg unformulated NAM, respectively, to measure the maximum systemic exposure and peak serum levels according to nutritional tolerable upper intake levels or levels without anticipated side effects (Table 8). From week 2 until week 6, NA microcapsules or NAM microcapsules packaged in gelatin capsules (see Example 1) were administered with weekly dose increases (Tables 7 and 8). Due to the low systemic exposure observed with encapsulated NA or NAM in weeks 2-4 (see below), doses were increased by 105 mg NA per week (vs. 30 mg before) or 750 mg NAM per week (vs. 300 mg before) in weeks 5 and 6 up to the target doses of 300 mg NA or 3000 mg NAM, respectively (Table 8).

**Table 6: Baseline characteristics of the FIM study population**

| | **NA group (n = 5)** | **NAM group (n = 5)** | **All subjects (n = 10)** |
|---|---|---|---|
| **Age (years)** | 50.20 ± 7.50¹ | 39.40 ± 12.10 | 44.80 ± 11.06 |
| **Age span (years)** | 40-59 | 24-51 | 24-59 |
| **Gender** | 2 male, 3 female | 5 female | 2 male, 8 female |
| **Height (m)** | 1.75 ± 0.06 | 1.70 ± 0.08 | 1.72 ± 0.73 |
| **Weight (kg)** | 85.64 ± 15.50 | 73.52 ± 10.23 | 79.58 ± 13.93 |
| **BMI (kg/m²)** | 27.97 ± 4.33 | 25.55 ± 2.86 | 26.76 ± 3.68 |
| **BMI span (kg/m²)** | 23.27-33.70 | 21.02-28.26 | 21.02-33.70 |
| **BP mm Hg (systolic)** | 119.00 ± 11.40 | 121.00 ±7.42 | 120.00 ± 9.13 |
| **BP mm Hg (diastolic)** | 75.00 ± 11.18 | 72.00 ± 4.47 | 73.50 ± 8.18 |
| **NAM² (µg/L)** | 10.90 (9.6, 34.75)³ | 24.90 (18.65, 27.35) | 19.80 (10.70, 26.42) |
| **Fasting glucose (mg/dL)** | 96.80 ± 7.63 | 91.40 ± 5.50 | 94.10 ± 6.89 |
| **Fasting insulin (mIU/L)** | 8.56 ± 3.24 | 7.10 ± 5.77 | 7.83 ± 4.48 |
| **HOMA-IR index** | 2.06 ± 0.81 | 1.56 ± 1.19 | 1.81 ± 1.00 |
| **Uric acid (µmol/L)** | 304.20 ± 37.38 | 274.60 ± 45.23 | 289.40 ± 42.11 |
| **LDL (mmol/L)** | 3.50 ± 1.08 | 2.88 ± 1.02 | 3.19 ± 1.04 |
| **HDL (mmol/L)** | 2.07 ± 0.52 | 1.75 ± 0.32 | 1.91 ± 0.44 |
| **Lp(a)⁴ (nmol/L)** | 24.50 (7.35, 127.70) | 17.10 (7.60, 193.95) | 20.80 (7.53, 160.45) |
| **Triglycerides (mmol/L)** | 1.06 ± 0.17 | 1.04 ± 0.52 | 1.05 ± 0.37 |
| **CRP (mg/L)** | 1.52 (0.89, 7.71) | 2.07 (0.99, 4.13) | 1.82 (0.97, 3.35) |
| ¹ mean ± SD, applies to all values in this format; | | | |
| ² baseline NA serum levels of all subjects were below the detection limit of 12 µg/L and are therefore not included in this table; | | | |
| ³ median (25^{th} and 75^{th} percentiles), applies to all values in this format; | | | |
| ⁴ for Lp(a), values for two subjects were slightly below the detection limit of 7 nmol/L and set to 7.0 for the calculations; | | | |
| BMI, body mass index; BP mm Hg, blood pressure (mm of mercury); NAM (µg/L), nicotinamide serum levels; HOMA-IR, homeostasis model assessment insulin resistance index; LDL, low density lipoprotein; HDL, high density lipoprotein; Lp(a), lipoprotein (a); CRP, C-reactive protein. | | | |

**Table 7: Dosing regimen, sampling and analyses in the FIM study**

| | **Dosing** | **Blood sampling** | **Stool sampling** | **Further analyses** |
|---|---|---|---|---|
| **Monday** | Dose 1-6 | 0 h, 2 h | Baseline sample (day 0) | 0 h: blood pressure, body weight |
| **Tuesday** | Dose 1-6 | - | - | - |
| **Wednesday** | Dose 1-6 | - | - | - |
| **Thursday** | Dose 1-6 | 72 h | Weekly sample | Blood pressure |
| **Friday** | Washout | - | - | Laboratory analyses to determine exposure and monitor safety parameters |
| **Saturday** | Washout | - | - | |
| **Sunday** | Washout | - | - | |

**Table 8: Dosing regimen of the FIM study**

| | **NA group** | **NAM group** |
|---|---|---|
| **Week 1: dose 1** | 30 mg free NA (n = 5) | 900 mg free NAM (n = 5) |
| **Week 2: dose 2** | 30 mg enc. ¹ NA (n = 5) | 900 mg enc. NAM (n = 4) |
| **Week 3: dose 3** | 60 mg enc. NA (n = 5) | 1200 mg enc. NAM (n = 4) |
| **Week 4: dose 4** | 90 mg enc. NA (n = 5) | 1500 mg enc. NAM (n = 4) |
| **Week 5: dose 5** | 195 mg enc. NA (n = 5) | 2250 mg enc. NAM (n = 4) |
| **Week 6: dose 6** | 300 mg enc. NA (n = 4) | 3000 mg enc. NAM (n = 4) |
| ¹ enc., microencapsulated | | |

### Sample analysis

In order to determine the intake of NA and NAM via the usual diet, a nutritional protocol was completed by the subjects from Mondays to Wednesdays. Blood samples were collected on Mondays and Thursdays (Table 7). On Mondays, the first blood sample was collected before ingestion of the capsules ("0 h" samples), and a second blood sample was taken after 2 hours ("2 h"), while subjects were still fasting. On Thursdays, blood samples were taken after administration of the capsules ("72 h"). Blood samples were subjected to routine laboratory analyses at the central laboratory of the University Hospital Schleswig-Holstein in Kiel (Germany). In addition, NA and NAM levels were measured in all blood samples by LC-MS/MS in an external specialised laboratory to determine exposure (see Example 1). Blood pressure was measured on Mondays and Thursdays (Table 7). For calculating the body mass index (BMI) of the subjects, body weight was recorded every Monday using a Tanita scale (Body Composition Analyzer; Type BC-418 MA; Tanita, Tokyo, Japan) and subjects stated their height.

For microbiota analyses, stool samples were collected on the first Monday of the study (day 0, baseline sample) and subsequently once weekly on Thursdays (Table 7). Total genomic DNA was extracted from feces collected longitudinally from each subject using MoBio Powersoil DNA Isolation kit (Dianova GmbH, Hamburg, Germany) according to the manufacturer's instructions. Aliquots of extracted DNA were used to amplify the V3-V4 variable region of 16S rRNA using composite primers (319F and 806R), as described by Fadrosh et al. (Microbiome 2:6, 2014). Amplification was performed by Phusion® hot start flex 2X master mix (New England Biolabs, Frankfurt/M., Germany) in a GeneAmp PCR system 9700 (Life Technologies/Applied Biosystems, Darmstadt, Germany) using the following cycling conditions: an initial denaturation of 3 min at 98°C followed by 30 cycles of denaturation at 98°C for 10 s, annealing at 55°C for 30 s and elongation at 72°C for 30 s, and a final extension step at 72°C for 10 min. PCR performance was assessed by agarose gel electrophoresis for quality (expected amplicon size) and quantity (band intensity). Quantitative normalization was performed using the SequalPrep kit (Life/Technologies/Invitrogen, Darmstadt, Germany) to pool equal amounts of amplicons per sample. Sequencing was performed by Illumina MiSeq (2 x 250 sequencing kit; Illumina, San Diego, CA, USA), which allowed to generate paired end reads with entire overlapping in contigs generation. Sequencing reads were primarily processed for quality control using the software mothur (Schloss et al. 2009, Appl. Environ. Microbiol. 75:7537). Forward and reverse reads (fastq) were assembled to contig sequences and discarded if they had more than 475 bases, any ambiguous base or more than 8 homopolymers. Sequences were aligned against the mothur-curated silva alignment database and screened to have alignment in the amplified specified V3-V4 region only. Chimeric sequences were detected with the Uchime algorithm (Edgar et al. 2011, Bioinformatics 27:2194) and were also removed. Sequences were assigned taxonomically using mothur-formatted silva training sets (version: silva.nr_v119) and eliminated if classified as unknown, archaea, eukaryote, chloroplast or mitochondria. A phylip-formatted distance matrix was computed from remaining quality-aligned sequences. Sequences with at least 97% similarity were clustered in into species Operational Taxonomical Units (OTUs) using the neighbour-joining algorithm within mothur.

### Statistical analyses

Statistical analyses were carried out using SPSS version 22.0 (SPSS, Chicago, IL, USA), and graphic data analysis was performed with GraphPad Prism version 5.0 (GraphPad Software, San Diego, CA, USA). Data were checked for normality by using the Shapiro-Wilk test and are presented as mean ± standard deviation (SD) (in the case of normally distributed data) or as median with the interquartile range (in the case of non-normally distributed data). The independent sample t-test and Mann-Whitney-U-test were used to determine group differences at baseline for continuous variables, and the X²-test was used for categorical variables. To determine significant changes in the microbial composition over time, repeated ANOVA and paired t-test were performed. Statistical significance was set at p<0.05.

### Results and discussion

Apart from the drop-out of Subject 2 (NA group), who had to be excluded after week 5 due to elevated AST levels, no safety signals were observed. All safety-relevant parameters are summarised in Table 9.

When measuring NA and NAM levels, it was observed that NA seemed to be rapidly metabolised to NAM as described in the literature (Reiche et al. 2011, Nephrol. Dial. Transplant. 26:276; Villines et al. 2012, Curr. Atheroscler. Rep. 14:49). In most cases, NA serum levels were below the detection limit of 12 µg/L (Table 10; cf. Table 6). Therefore, only NAM levels are shown in Figures 7-9. All measurements of NA and NAM serum levels are reported in Tables 10 and 11, respectively. Despite the dose escalation with microencapsulated NA, no consistent or dose-dependent increase in NA or NAM serum levels was observed compared to the levels obtained after uptake of the maximum daily nutritional dose of free NA (Tables 10 and 11; Figure 7). In contrast, higher doses of microencapsulated NAM led to higher systemic exposure, albeit with levels far below those obtained after ingestion of 900 mg of free NAM and with high interindividual variability (Table 11; Figure 8). No NA or NAM accumulation was observed when comparing serum levels at 72 hours with baseline levels. In Figure 9, all NAM serum levels are grouped by time point of acquisition rather than by individual subjects to illustrate interindividual variation and common trends.

For the microbiome analyses, a total of 976,351 16S rRNA gene sequences from 44 faecal samples were obtained, of which 966,038 passed quality control. As known for human gut microbiota, the majority of sequences were classified to the Firmicutes, Bacteroidetes, Actinobacteria and Proteobacteria. Interestingly, microbiome comparisons between the baseline samples obtained before the first doses of NA or NAM and samples obtained at the end of the study showed a trend (NAM) or significant change (NA) towards a higher abundance-based (Bray curtis) bacterial communities distance compared with a healthy normal reference group (Figure 10A). Moreover, subjects in the NA group showed a moderate, but overall significant increase in Bacteroidetes (Figure 10B). In many studies, an decrease of Bacteroidetes has been found to be associated with obesity in humans (Ley et al. 2006, Nature 444:1022; Turnbaugh et al. 2009, Nature 457:480), which supports the use of microcapsules of the present invention for targeted gut microbiota improvement (*e.g.*, by enrichment of Bacteroidetes) by topical application of NA or NAM.

Taken together with the microcapsule characterisation performed in Example 1, these results demonstrate that the formulations of the present invention deliver NA and NAM to the intestine with a significantly reduced systemic exposure and a favourable safety profile, inducing trends towards a modification of the intestinal microbiota even in this short-term dose escalation study.

**Table 9 (part 1): Safety parameters of the FIM study population**

| | **S1** | **S2** | **S3** | **S4** | **S5** | **S6** | **S7** | **S8** | **S9** | **S10** |
|---|---|---|---|---|---|---|---|---|---|---|
| **Age (y)** | | | | | | | | | | |
| w1h0 | 48 | 56 | 40 | 48 | 59 | 29 | 45 | 51 | 48 | 24 |

| **Height (m)** | | | | | | | | | | |
|---|---|---|---|---|---|---|---|---|---|---|
| w1h0 | 1.76 | 1.80 | 1.68 | 1.70 | 1.80 | 1.78 | 1.72 | 1.58 | 1.76 | 1.64 |

| **Weight (kg)** | | | | | | | | | | |
|---|---|---|---|---|---|---|---|---|---|---|
| w1h0 | 91.2 | 109.2 | 83.0 | 69.4 | 75.4 | 83.7 | 83.6 | 61.6 | 65.1 | 73.6 |
| w6h0 | 91.3 | 109.0* | 80.2 | 70.5 | 73.3 | - | 84.7 | 61.4 | 64.5 | 73.5 |

| **Body mass index (BMI) (kg/m²)** | | | | | | | | | | |
|---|---|---|---|---|---|---|---|---|---|---|
| w1h0 | 29.40 | 33.70 | 29.41 | 24.01 | 23.27 | 26.42 | 28.26 | 24.68 | 21.02 | 27.36 |
| w6h0 | 29.47 | 33.64* | 28.42 | 24.39 | 22.62 | - | 28.63 | 24.60 | 20.82 | 27.33 |

| Blood **pressure (mm Hg; systolic /diastolic)** | | | | | | | | | | |
|---|---|---|---|---|---|---|---|---|---|---|
| w1h0 | 125/75 | 130/90 | 120/70 | 100/60 | 120/80 | 120/70 | 110/70 | 120/80 | 125/70 | 130/70 |
| w1h71 | 120/80 | 130/85 | 115/70 | 105/70 | 130/80 | 120/80 | 105/75 | 120/80 | 120/80 | 120/75 |
| w6h72 | 110/70 | 130/80 | 120/80 | 100/70 | 120/80 | - | 110/75 | - | 110/75 | 105/70 |

| **Glucose (mg/dL)** | | | | | | | | | | |
|---|---|---|---|---|---|---|---|---|---|---|
| w1h0 | 91 | 97 | 101 | 88 | 107 | 86 | 94 | 95 | 97 | 85 |
| w1h71 | 77 | 92 | 89 | 85 | 94 | 80 | 92 | 84 | 83 | 87 |
| w6h72 | 85 | 99 (101)* | 96 | 90 | 97 | - | 94 | 90 | 92 | 82 |

| **Insulin (mIU/L)** | | | | | | | | | | |
|---|---|---|---|---|---|---|---|---|---|---|
| w1h0 | 10.2 | 8.8 | 12.8 | 4.4 | 6.6 | 17.2 | 3.8 | 5.6 | 3.0 | 5.9 |
| w1h71 | 9.6 | 15.9 | 8.7 | 3.3 | 4.2 | 11.0 | 5.4 | 4.8 | 1.7 | 8.5 |
| w6h72 | 13.4 | 12.2 (20.1)* | 7.8 | 7.2 | 4.3 | - | 5.9 | 5.7 | 5.0 | 6.2 |

| **Homeostasis model assessment insulin resistance (HOMA-IR) index** | | | | | | | | | | |
|---|---|---|---|---|---|---|---|---|---|---|
| w1h0 | 2.29 | 2.11 | 3.19 | 0.96 | 1.74 | 3.65 | 0.88 | 1.31 | 0.72 | 1.24 |
| w1h71 | 1.83 | 3.61 | 1.91 | 0.69 | 0.97 | 2.17 | 1.23 | 1.00 | 0.35 | 1.83 |
| w6h72 | 2.81 | 2.98 (5.01)* | 1.85 | 1.60 | 1.03 | - | 1.37 | 1.27 | 1.14 | 1.26 |

| **Uric acid (µmol/L)** | | | | | | | | | | |
|---|---|---|---|---|---|---|---|---|---|---|
| w1h0 | 261 | 347 | 325 | 319 | 269 | 260 | 313 | 262 | 325 | 213 |
| w1h71 | 320 | 323 | 294 | 265 | 258 | 260 | 336 | 312 | 325 | 235 |
| w6h72 | 300 | 351 (376)* | 285 | 262 | 295 | - | 312 | 230 | 350 | 251 |

| **Low density lipoprotein (LDL) (mmol/L)** | | | | | | | | | | |
|---|---|---|---|---|---|---|---|---|---|---|
| w1h0 | 1.68 | 3.54 | 4.49 | 4.05 | 3.75 | 2.05 | 2.38 | 2.79 | 4.63 | 2.53 |
| w1h71 | 1.81 | 3.79 | 4.47 | 3.46 | 4.36 | 2.23 | 2.40 | 2.32 | 4.36 | 2.54 |
| w6h72 | 1.74 | 4.06 (4.30)* | 4.05 | 4.14 | 4.35 | - | 2.79 | 2.45 | 3.56 | 3.22 |

| **High density lipoprotein HDL (mmol/L)** | | | | | | | | | | |
|---|---|---|---|---|---|---|---|---|---|---|
| w1h0 | 2.21 | 1.20 | 2.31 | 2.56 | 2.08 | 2.03 | 1.94 | 1.94 | 1.52 | 1.30 |
| w1h71 | 2.32 | 1.25 | 2.48 | 2.15 | 2.23 | 2.50 | 1.42 | 1.91 | 1.32 | 1.32 |
| w6h72 | 2.08 | 1.35 (1.27)* | 2.51 | 2.09 | 2.52 | - | 2.10 | 2.34 | 1.83 | 1.54 |

| **Lipoprotein (a) [Lp(a)] (nmol/L)** | | | | | | | | | | |
|---|---|---|---|---|---|---|---|---|---|---|
| w1h0 | <7 | 96.8 | 158.6 | 24.5 | 7.7 | 8.2 | 17.1 | 221.9 | 166.0 | <7 |
| w1h71 | <7 | 98.8 | 168.9 | 21.3 | 16.9 | 7.4 | 11.0 | 189.5 | 173.9 | <7 |
| w6h72 | <7 | 103.7 (109.8)* | 171.7 | 23.3 | 12.2 | - | 15.7 | 225.8 | 200.5 | <7 |

| **Triglycerides (mmol/L)** | | | | | | | | | | |
|---|---|---|---|---|---|---|---|---|---|---|
| w1h0 | 1.2 | 1.2 | 1.1 | 0.8 | 1.0 | 1.6 | 0.7 | 0.5 | 1.6 | 0.8 |
| w1h71 | 0.9 | 0.7 | 1.4 | 1.0 | 1.0 | 1.0 | 1.2 | 0.4 | 1.3 | 0.7 |
| w6h72 | 1.3 | 0.9 (1.8)* | 0.9 | 1.0 | 0.8 | - | 0.9 | 0.6 | 0.9 | 0.8 |

| | **S1** | **S2** | **S3** | **S4** | **S5** | **S6** | **S7** | **S8** | **S9** | **S10** |
|---|---|---|---|---|---|---|---|---|---|---|
| **C-reactive protein (CRP)** | | | **(mg/L)** | | | | | | | |
| w1h0 | 1.05 | 2.41 | 13.00 | 1.52 | 0.72 | 6.17 | 2.07 | 0.41 | 1.57 | 2.08 |
| w1h71 | 0.96 | 2.48 | 11.80 | 1.05 | 0.63 | 3.55 | 2.26 | 0.35 | 0.93 | 2.87 |
| w6h72 | 1.30 | 3.34 (3.01)* | 8.93 | 0.95 | 0.72 | - | 2.76 | 0.43 | 1.78 | 3.45 |

| **Aspartate aminotransferase (AST) (U/L)** | | | | | | | | | | |
|---|---|---|---|---|---|---|---|---|---|---|
| w1h0 | 15.9 | 24.8 | 14.3 | 30.2 | 24.8 | 21.6 | 16.6 | 22.0 | 21.9 | 23.8 |
| w1h71 | 15.0 | 22.7 | 16.5 | 25.2 | 28.0 | 19.8 | 21.8 | 22.4 | 22.4 | 21.1 |
| w6h72 | 15.1 | 116.0 (32.4)* | 16.7 | 30.3 | 22.6 | - | 23.9 | 22.0 | 25.0 | 21.9 |

| **Alanine aminotransferase (ALT) (U/L)** | | | | | | | | | | |
|---|---|---|---|---|---|---|---|---|---|---|
| w1h0 | 16.4 | 14.3 | 19.2 | 30.1 | 19.1 | 27.5 | 9.7 | 10.8 | 21.5 | 20.3 |
| w1h71 | 19.6 | 17.7 | 30.6 | 23.4 | 24.3 | 25.6 | 15.7 | 0.9 | 18.5 | 21.6 |
| w6h72 | 15.9 | 48.8 (31.1)* | 18.7 | 24.8 | 17.9 | - | 16.3 | 10.1 | 20.0 | 20.4 |

| **Gamma-glutamyl transpeptidase (γ-GT) (U/L)** | | | | | | | | | | |
|---|---|---|---|---|---|---|---|---|---|---|
| w1h0 | 20 | 24 | 36 | 23 | 18 | 29 | 13 | 28 | 28 | 9 |
| w1h71 | 19 | 26 | 32 | 22 | 21 | 29 | 16 | 24 | 27 | 10 |
| w6h72 | 16 | 24 (29)* | 27 | 20 | 16 | - | 17 | 28 | 35 | 10 |

| **Bilirubin (µmol/L)** | | | | | | | | | | |
|---|---|---|---|---|---|---|---|---|---|---|
| w1h0 | 8.5 | 9.1 | <2.0 | 7.6 | 6.3 | 5.2 | 6.7 | 4.7 | 5.3 | 6.1 |
| w1h71 | 11.1 | 9.7 | 3.7 | - | 8.0 | 5.9 | 4.3 | 6.9 | 6.2 | 8.0 |
| w6h72 | 10.3 | 8.2 (8.0)* | 4.5 | 8.3 | 6.2 | - | 10.8 | 9.4 | 6.0 | 9.3 |

| **Creatinine (µmol/L)** | | | | | | | | | | |
|---|---|---|---|---|---|---|---|---|---|---|
| w1h0 | 77 | 98 | 80 | 86 | 84 | 57 | 74 | 63 | 71 | 61 |
| w1h71 | 87 | 99 | 76 | 73 | - | 61 | 91 | 81 | 62 | 61 |
| w6h72 | 81 | 98 (94)* | 77 | 80 | 91 | - | 80 | 62 | 73 | 67 |
| S1 - S10: subject 1 - 10; | | | | | | | | | | |
| w1 h0 - w6h72: week 1 hour 0 - week 6 hour 72; | | | | | | | | | | |
| * measurements for subject 2 at week 5 (drop-out due to AST elevation), control values were measured 4 days after the end of the intervention (in parentheses). | | | | | | | | | | |

**Table 10: NA serum levels of the FIM study population**

| **NA (µg/L)** | **NA group** | | | | | **NAM group** | | | | |
|---|---|---|---|---|---|---|---|---|---|---|
| | **S1** | **S2** | **S3** | **S4** | **S5** | **S6** | **S7** | **S8** | **S9** | **S10** |
| w1h0 | < 12 | < 12 | < 12 | < 12 | < 12 | < 12 | < 12 | < 12 | < 12 | < 12 |
| w1h2 | < 12 | < 12 | < 12 | < 12 | < 12 | < 12 | **12.7** | **16.1** | < 12 | < 12 |
| w1h72 | < 12 | < 12 | < 12 | < 12 | < 12 | < 12 | **12.8** | **21.8** | **20.3** | < 12 |
| w2h0 | < 12 | < 12 | < 12 | < 12 | < 12 | < 12 | < 12 | < 12 | < 12 | < 12 |
| w2h2 | < 12 | < 12 | < 12 | < 12 | < 12 | < 12 | < 12 | < 12 | < 12 | < 12 |
| w2h72 | < 12 | < 12 | < 12 | < 12 | < 12 | - | < 12 | < 12 | < 12 | < 12 |
| w3h0 | < 12 | < 12 | < 12 | < 12 | < 12 | - | < 12 | < 12 | < 12 | < 12 |
| w3h2 | < 12 | < 12 | < 12 | < 12 | < 12 | - | < 12 | < 12 | < 12 | < 12 |
| w3h72 | < 12 | < 12 | < 12 | < 12 | < 12 | - | < 12 | < 12 | < 12 | < 12 |
| w4h0 | < 12 | < 12 | < 12 | < 12 | < 12 | - | < 12 | < 12 | < 12 | < 12 |
| w4h2 | < 12 | < 12 | < 12 | < 12 | < 12 | - | < 12 | < 12 | < 12 | < 12 |
| w4h72 | < 12 | < 12 | < 12 | < 12 | < 12 | - | **21.3** | < 12 | < 12 | < 12 |
| w5h0 | < 12 | < 12 | < 12 | < 12 | < 12 | - | < 12 | < 12 | < 12 | < 12 |
| w5h2 | < 12 | < 12 | < 12 | < 12 | < 12 | - | < 12 | < 12 | < 12 | < 12 |
| w5h72 | **15.2** | < 12 | < 12 | < 12 | < 12 | - | < 12 | **13** | < 12 | < 12 |
| w6h0 | < 12 | - | < 12 | < 12 | < 12 | - | < 12 | < 12 | < 12 | < 12 |
| w6h2 | < 12 | - | < 12 | < 12 | < 12 | - | < 12 | < 12 | < 12 | < 12 |
| w6h72 | < 12 | - | < 12 | < 12 | < 12 | - | < 12 | < 12 | < 12 | < 12 |
| S1 - S10: subject 1 - 10; | | | | | | | | | | |
| w1 h0 - w6h72: week 1 hour 0 - week 6 hour 72; | | | | | | | | | | |
| **bold** figures represent measurements above the detection limit of 12 µg/L. | | | | | | | | | | |

**Table 11: NAM serum levels of the FIM study population**

| **NAM (µg/L)** | **NA grou p** | | | | | **NAM group** | | | | |
|---|---|---|---|---|---|---|---|---|---|---|
| | **S1** | **S2** | **S3** | **S4** | **S5** | **S6** | **S7** | **S8** | **S9** | **S10** |
| w1h0 | 9.1 | 53.8 | 10.1 | 15.7 | 10.9 | 13.4 | 23.9 | 29.2 | 24.9 | 25.5 |
| w1h2 | 6.6 | 25.6 | 10.5 | 18.2 | 17.9 | 14,800.0 | 14,800.0 | 21,600.0 | 11,500.0 | 15,000.0 |
| w1h72 | 16.7 | 46.2 | 32.1 | 29.9 | 26.0 | 14,000.0 | 16,700.0 | 20,600.0 | 16,600.0 | 13,600.0 |
| w2h0 | 10.4 | 20.7 | 29.3 | 17.1 | 20.4 | 23.4 | 25.3 | 29.6 | 19.6 | 21.3 |
| w2h2 | 8.5 | 29.0 | 8.6 | 17.6 | 21.7 | 277.0 | 165.0 | 120.0 | 1,178.0 | 552.0 |
| w2h72 | 10.3 | 38.7 | 21.4 | 25.7 | 31.4 | - | 3,060.0 | 354.0 | 24.4 | 43.8 |
| w3h0 | 4.7 | 26.0 | 12.6 | 19.7 | 19.2 | - | 21.8 | 26.1 | 23.7 | 22.1 |
| w3h2 | 8.8 | 32.1 | 16.0 | 34.9 | 25.8 | - | 50.7 | 815.0 | 1,000.0 | 353.0 |
| w3h72 | 17.3 | 30.6 | 18.2 | 34.2 | 28.8 | - | 62.7 | 422.0 | 63.4 | 53.3 |
| w4h0 | 12.6 | 35.4 | 35.1 | 19.9 | 22.2 | - | 30.6 | 37.9 | 31.2 | 27.7 |
| w4h2 | 20.6 | 32.0 | 19.3 | 31.8 | 20.0 | - | 269.0 | 1,131.0 | 3,355.0 | 1,001.0 |
| w4h72 | 12.5 | 37.1 | 20.1 | 32.3 | 28.5 | - | 225.0 | 849.0 | 84.0 | 48.3 |
| w5h0 | 13.8 | 22.5 | 13.2 | 25.2 | 23.4 | - | 34.5 | 18.2 | 23.3 | 40.2 |
| w5h2 | 32.7 | 25.0 | 8.9 | 34.9 | 24.4 | - | 443.0 | 1,935.0 | 501.0 | 784.0 |
| w5h72 | 18.9 | 47.5 | 20.6 | 34.7 | 32.4 | - | 106.0 | 3,545.0 | 100.0 | 61.2 |
| w6h0 | 9.7 | - | 15.7 | 27.7 | 25.1 | - | 31.3 | 51.8 | 32.3 | 37.7 |
| w6h2 | 14.7 | - | 25.9 | 38.1 | 22.5 | - | 550.0 | 3,600.0 | 2,780.0 | 2,975.0 |
| w6h72 | 28.2 | - | 23.7 | 29.6 | 46.5 | - | 1,866.0 | 1,663.0 | 205.0 | 99.8 |
| S1 - S10: subject 1 - 10; | | | | | | | | | | |
| w1 h0 - w6h72: week 1 hour 0 - week 6 hour 72. | | | | | | | | | | |

### Example 3: Pilot pharmacokinetic (PK) study with NAM microcapsules

### Aims of the study

In a small pilot PK study, gelatin capsules filled with NAM microcapsules from the batch used for the FIM dose escalation study (see Examples 1 and 2) were ingested by three other healthy volunteers in order to get a first impression of the NAM PK profile and interindividual variability. As the low NAM serum levels at 2 h after NAM microcapsule ingestion in the FIM study suggested a significantly delayed peak of exposure, the first 12 h after ingestion were monitored.

### Methods

### Study population and design

The study was a self-experiment by medical investigators performed at the Department of Internal Medicine 1 of the University Hospital Schleswig-Holstein, Campus Kiel (Kiel, Germany).The baseline characteristics of the study subjects are summarised in Table 12.

**Table 12: Baseline characteristics of the pilot NAM PK study subjects**

| | **subject 1** | **subject 2** | **subject 3** |
|---|---|---|---|
| **Gender** | male | female | female |
| **Age (years)** | 43 | 52 | 26 |
| **Height (m)** | 1.78 | 1.70 | 1.68 |
| **Weight (kg)** | 78 | 67 | 62 |
| **BMI (kg/m²)** | 24.62 | 23.18 | 21.97 |
| **NA (µg/L)** | <12 | <12 | <12 |
| **NAM (µg/L)** | 45.8 | 16.6 | 17.9 |
| BMI, body mass index; NA, nicotinic acid serum levels; NAM, nicotinamide serum levels. | | | |

In this study, subjects ingested either 900 mg of unformulated NAM (first week) or 900 mg of microencapsulated NAM packaged in gelatin capsules (second week). Before self-administration, subjects fasted with water only, and only two light meals with minimal vitamin B3 content (3.2 mg of niacin equivalents) were eaten after the blood samplings of hour 3 and hour 8. One such "low niacin meal" consisted of 150 g of apple, 150 g of red pepper and 100 g of cucumber. One niacin equivalent is defined as 1 mg of NA(M) or 60 mg of tryptophan.

### Sample analysis

Height and body weight were recorded before ingestion of the test item. Blood samples were collected before ingestion of the test item (hour 0), every hour until 8 hours after ingestion as well as after 10 and 12 hours (Tables 13 and 14). NA and NAM serum levels were measured in all blood samples by LC-MS/MS in an external specialised laboratory (see Example 2).

### Results and discussion

As shown in Figure 11 and Table 13, ingestion of 900 mg of free NAM led to a much higher fold increase of NAM serum levels than ingestion of 900 mg of microencapsulated NAM. The peak of NAM serum levels was consistently observed at 1 hour after ingestion of free NAM, whereas the peak after ingestion of microencapsulated NAM varied between 3 and 6 hours. Interindividual variability in terms of peak height and form was considerable despite an overall similarity of PK profiles. Apart from the time points corresponding to peak serum levels after ingestion of free NAM, NA serum levels were below the detection limit of 12 µg/L (Table 14; cf. Tables 6 and 10 in Example 2).

**Table 13: NAM serum levels of the pilot NAM PK study subjects**

| **NAM (µg/L)** | **subject 1** | | **subject 2** | | **subject 3** | |
|---|---|---|---|---|---|---|
| | **free NAM** | **enc. NAM** | **free NAM** | **enc. NAM** | **free NAM** | **enc. NAM** |
| **hour 0** | 45.8 | 26.8 | 16.6 | 16.3 | 17.9 | 15.7 |
| **hour 1** | **11,600** | 41.5 | **19,900** | 19.9 | **20,100** | 26.4 |
| **hour 2** | 10,285 | 63.9 | 15,900 | 203.5 | 17,200 | 386 |
| **hour 3** | 8,375 | 551 | 9,740 | **1,155** | 13,100 | 1,975 |
| **hour 4** | 5,920 | **1,145** | 7,390 | 997 | 10,300 | 3,290 |
| **hour 5** | 4,355 | 1,040 | 3,980 | 224.5 | 8,190 | 3,890 |
| **hour 6** | 3,030 | 589 | 2,270 | 41.75 | 5,840 | 6,310 |
| **hour 7** | 1,815 | 297 | 781 | 25.9 | 3,790 | 5,030 |
| **hour 8** | 892 | 160 | 135 | 19.3 | 2,610 | 3,270 |
| **hour 10** | 233 | 82.3 | 21.9 | 26.9 | 318 | 1,055 |
| **hour 12** | 45.9 | 49.7 | 15.2 | 18.5 | 61.9 | 170 |
| **max. fold increase** | x 253 | x 43 | x 1,199 | x 71 | x 1,123 | x 402 |
| **Bold** figures represent peak values; | | | | | | |
| enc., microencapsulated; NAM, nicotinamide. | | | | | | |

**Table 14: NA serum levels of the pilot NAM PK study subjects**

| **NA (µg/L)** | **subject 1** | | **subject 2** | | **subject 3** | |
|---|---|---|---|---|---|---|
| | **free NAM** | **enc. NAM** | **free NAM** | **enc. NAM** | **free NAM** | **enc. NAM** |
| **hour 0** | < 12 | < 12 | < 12 | < 12 | < 12 | < 12 |
| **hour 1** | < 12 | < 12 | **17.6** | < 12 | **15.9** | < 12 |
| **hour 2** | **13.2** | < 12 | **12.9** | < 12 | **12.9** | < 12 |
| **hour 3** | < 12 | < 12 | < 12 | < 12 | **13.0** | < 12 |
| **hour 4** | < 12 | < 12 | < 12 | < 12 | < 12 | < 12 |
| **hour 5** | < 12 | < 12 | < 12 | < 12 | < 12 | < 12 |
| **hour 6** | < 12 | < 12 | < 12 | < 12 | < 12 | < 12 |
| **hour 7** | < 12 | < 12 | < 12 | < 12 | < 12 | < 12 |
| **hour 8** | < 12 | < 12 | < 12 | < 12 | < 12 | < 12 |
| **hour 10** | < 12 | < 12 | < 12 | < 12 | < 12 | < 12 |
| **hour 12** | < 12 | < 12 | < 12 | < 12 | < 12 | < 12 |
| **Bold** figures represent measurements above the detection limit of 12 µg/L (around the peak values of free NAM in Table 13); | | | | | | |
| enc., microencapsulated; NA, nicotinic acid; NAM, nicotinamide. | | | | | | |

Taken together, the results of this small pilot PK study confirmed the results of Examples 1 and 2 regarding a controlled and delayed release of NAM from the microcapsules of the present invention in intestinal areas of significantly lower resorptive capacity, *i.e.*, the lower small intestine and colon.

### Example 4: Pharmacokinetic study with NA and NAM microcapsules

### Aims of the study

In this PK study to investigate release profiles and exposure following ingestion of NA and NAM microcapsules, gelatin capsules filled with NA or NAM microcapsules from the batches used for the FIM dose escalation study (see Examples 1 and 2) were ingested by 10 healthy volunteers each.

### Methods

### Study population and design

The study was performed at the Department of Internal Medicine 1 of the University Hospital Schleswig-Holstein, Campus Kiel (Kiel, Germany). The study was approved by the ethics committee of the University of Kiel (reference number: D439/15). Written informed consent was obtained from each volunteer.

Twenty healthy volunteers were included and allocated to 2 groups of 10 subjects each (5 male, 5 female). One group received nicotinic acid (NA group) and the other group received nicotinamide (NAM group). The baseline characteristics of the study population are summarised in Table 15. Subject 8 of the NA group dropped out after day 2 of the study due to persistent difficulties in blood sample collection. For calculating the body mass index (BMI) of the subjects, baseline body weight was measured on the first day of the study before dosing using a Tanita scale (Body Composition Analyzer; Type BC-418 MA; Tanita, Tokyo, Japan) and subjects stated their height.

The study consisted of four study days, each separated by one week of washout and regeneration. On the calibration day of the study, the subjects ingested 30 mg of unformulated NA (NA group) or 900 mg of unformulated NAM (NAM group) as in the FIM study (see Example 2). Different doses of microencapsulated NA or NAM packaged in gelatin capsules were administered on the other three study days: 30 mg NA or 900 mg NAM, 150 mg NA or 1500 mg NAM, and 300 mg NA or 3000 mg NAM.

The design of the study days is summarised in Figure 12. After 12 h of fasting overnight (with water only), a fasted blood sample was collected. After ingestion of NA, NAM or the respective capsules, blood samples were collected every hour until 8 hours after ingestion as well as after 10 and 12 hours. During the collection of blood samples, subjects remained fasted (with water ad libitum) except for two light meals with minimal vitamin B3 content after the blood samplings of hour 3 and hour 8. One such "low niacin meal" consisted of three rice wafers, one apple and one carrot with a total of 2.3 mg of niacin equivalents.

**Table 15: Baseline characteristics of the PK study population**

| | **NA group (n = 10)** | **NAM group (n = 10)** | **All subjects (n = 20)** |
|---|---|---|---|
| **Age (years)** | 26.20 ± 5.07¹ | 27.50 ± 4.81 | 26.85 ± 4.86 |
| **Gender** | 5 male, 5 female | 5 male, 5 female | 10 male, 10 female |
| **Height (m)** | 1.78 ± 0.79 | 1.75 ± 0.08 | 1.76 ± 0.08 |
| **Weight (kg)** | 75.16 ± 19.22 | 73.70 ± 13.66 | 74.43 ± 16.25 |
| **BMI (kg/m²)** | 22.91 (20.40; 24.90)² | 22.78 (21.66; 26.80) | 22.78 (21.25; 25.57) |
| **NAM³ (µg/L)** | 18.53 ± 7.52* | 12.67 ± 2.76* | 15.60 ± 6.28 |
| **Uric acid (µmol/L)** | 296.20 ± 63.60 | 284.80 ± 81.79 | 290.50 ± 71.55 |
| **Creatinine (µmol/L)** | 86.70 ± 9.56 | 80.80 ± 13.11 | 83.75 ± 11.57 |
| **eGFR (mL/min/1.73)** | 94.20 ± 13.26 | 102.80 ± 12.18 | 98.50 ± 13.16 |
| **AST (U/L)** | 20.65 (19.82; 29.88) | 20.15 (17.85; 26.70) | 20.65 (19.03; 26.35) |
| **ALT (U/L)** | 20.55 (11.80; 26.33) | 18.85 (9.15; 31.55) | 20.55 (11.43; 30.75) |
| **γ-GT (U/L)⁴** | 16.50 (12.00; 26.75) | 17.00 (9.00; 28.28) | 17.00 (12.00; 26.50) |
| ¹mean ± SD, applies to all values in this format; | | | |
| ² median (25^{th} and 75^{th} percentiles), applies to all values in this format; | | | |
| ³ baseline NA serum levels of all subjects were below the detection limit of 12 µg/L and are therefore not included in this table; | | | |
| ⁴ for γ-GT, one value was slightly below the detection limit of 7 U/L and set to 7.0; | | | |
| * significant difference between the NA and NAM groups (unpaired T-test, p < 0.05); BMI, body mass index; NAM (µg/L), nicotinamide serum levels; eGFR, estimated glomerular filtration rate; AST, aspartate aminotransferase; ALT, alanine aminotransferase; γ-GT, gamma-glutamyl transpeptidase. | | | |

### Sample analysis

Fasted blood samples were collected to measure routine safety laboratory parameters (Table 15) at the central laboratory of the University Hospital Schleswig-Holstein in Kiel (Germany). In addition, NA and NAM levels were measured in all blood samples by LC-MS/MS in an external specialised laboratory (see Example 2).

### Statistical analyses

Statistical analyses were carried out using SPSS version 22.0 (SPSS, Chicago, IL, USA), and graphic data analysis was performed with GraphPad Prism version 5.0 (GraphPad Software, San Diego, CA, USA). Data were checked for normality by using the Shapiro-Wilk test and are presented as mean ± standard deviation (SD) (in the case of normally distributed data) or as median with the interquartile range (in the case of non-normally distributed data). The independent sample t-test and the Mann-Whitney-U-test were used to determine group differences at baseline. In order to evaluate systemic exposure to NAM, area under the curve (AUC) calculations were performed with GraphPad Prism. If serum levels after 12 hours were above baseline, curves were extrapolated using trend lines in Excel 2010 (Microsoft Cooperation, Redmond, WA, USA), and both raw and extrapolated data were documented. According to normal distribution, the paired sample t-test was used to determine differences between AUCs of unformulated or microencapsulated NAM, except for the non-normally distributed extrapolated data for 3000 mg of microencapsulated NAM, for which the Wilcoxon test was used. Statistical significance was set at p<0.05.

### Results and discussion

No safety signals were observed. All safety-relevant parameters are summarised in Tables 16 and 17. Gaps in the tables denote time points when blood sampling or measurement was impossible (e.g., dropout of patient 8 in the NA group shown in Table 16).

**Table 16: Safety parameters of the subjects in the NA group of the PK study**

| | **S1** | **S2** | **S3** | **S4** | **S5** | **S6** | **S7** | **S8** | **S9** | **S10** |
|---|---|---|---|---|---|---|---|---|---|---|
| **Height (m)** | | | | | | | | | | |
| Day 1 | 1.80 | 1.87 | 1.90 | 1.79 | 1.80 | 1.62 | 1.72 | 1.76 | 1.72 | 1.77 |

| **Weight (kg)** | | | | | | | | | | |
|---|---|---|---|---|---|---|---|---|---|---|
| Day 1 | 121.5 | 85.7 | 64.1 | 83.5 | 76.2 | 51.5 | 62.8 | 64.0 | 72.4 | 69.9 |

| **Body mass index (BMI) (kg/m²)** | | | | | | | | | | |
|---|---|---|---|---|---|---|---|---|---|---|
| Day 1 | 37.50 | 24.51 | 17.76 | 26.06 | 23.52 | 19.62 | 21.23 | 20.66 | 24.47 | 22.31 |

| **Uric acid (µmol/L)** | | | | | | | | | | |
|---|---|---|---|---|---|---|---|---|---|---|
| Day 1 | 389 | 411 | 298 | 266 | 317 | 194 | 257 | 277 | 268 | 285 |
| Day 2 | 396 | 370 | 264 | 249 | 283 | 248 | 220 | 254 | 270 | 269 |
| Day 3 | 367 | 391 | 266 | 327 | 327 | 253 | 187 | - | 261 | 257 |
| Day 4 | 332 | 351 | 243 | 268 | 328 | 194 | 160 | - | 244 | 238 |

| **Creatinine (µmol/L)** | | | | | | | | | | |
|---|---|---|---|---|---|---|---|---|---|---|
| Day 1 | 90 | 101 | 85 | 82 | 100 | 87 | 70 | 92 | 82 | 78 |
| Day 2 | 98 | 99 | 82 | 87 | 103 | 92 | 76 | 88 | 81 | 83 |
| Day 3 | 97 | 107 | 88 | 84 | 98 | 93 | 73 | - | 92 | 86 |
| Day 4 | 98 | 105 | 79 | 78 | 102 | 87 | 68 | - | 84 | 68 |

| **Estimated glomerular filtration rate (eGFR) (mL/min/1.73)** | | | | | | | | | | |
|---|---|---|---|---|---|---|---|---|---|---|
| Day 1 | 95 | 89 | 116 | 113 | 93 | 79 | 103 | 76 | 85 | 93 |
| Day 2 | 86 | 92 | 121 | 105 | 90 | 74 | 94 | 80 | 86 | 87 |
| Day 3 | 87 | 83 | 111 | 109 | 70 | 73 | 98 | - | 74 | 83 |
| Day 4 | 86 | 85 | 126 | 118 | 91 | 79 | 107 | - | 82 | 110 |

| **Aspartate aminotransferase (AST)** | | | | **(U/L)** | | | | | | |
|---|---|---|---|---|---|---|---|---|---|---|
| Day 1 | 40.0 | 25.9 | 18.4 | 26.5 | 43.0 | 20.6 | 20.7 | 19.3 | 20.2 | 20.0 |
| Day 2 | 30.9 | 14.2 | 21.2 | 22.7 | 32.4 | 24.5 | 19.3 | 45.4 | 16.0 | 20.9 |
| Day 3 | 33.8 | 17.9 | 23.1 | 34.1 | 32.1 | 21.1 | 18.1 | - | 15.3 | 18.6 |
| Day 4 | 38.5 | 17.7 | 25.1 | 24.1 | 346 | 20.5 | 19.2 | - | 16.3 | 21.7 |

| **Alanine aminotransferase (ALT) (U/L)** | | | | | | | | | | |
|---|---|---|---|---|---|---|---|---|---|---|
| Day 1 | 81.4 | 33.9 | 11.4 | 16.5 | 23.8 | 11.9 | 22.4 | 11.5 | 20.3 | 20.8 |
| Day 2 | 63.9 | 19.0 | 14.3 | 14.4 | 21.7 | 15.1 | 17.8 | 22.0 | 14.1 | 25.3 |
| Day 3 | 63.0 | 21.8 | 16.8 | 17.5 | 18.8 | 14.1 | 21.2 | - | 15.7 | 21.2 |
| Day 4 | 62.7 | 18.7 | 16.0 | 14.4 | 17.7 | 14.5 | 19.6 | - | 11.3 | 18.6 |

| **Gamma-glutamyl transpeptidase (γ-GT) (U/L)** | | | | | | | | | | |
|---|---|---|---|---|---|---|---|---|---|---|
| Day 1 | 33 | 32 | 14 | 12 | 12 | 25 | 10 | 12 | 19 | 19 |
| Day 2 | 26 | 27 | 13 | 11 | 13 | 27 | 10 | 14 | 16 | 20 |
| Day 3 | 28 | 25 | 15 | 13 | 12 | 28 | 14 | - | 15 | 20 |
| Day 4 | 25 | 25 | 13 | 10 | 12 | 27 | 12 | - | 12 | 18 |
| S1 - S10: subject 1 - 10. | | | | | | | | | | |

**Table 17: Safety parameters of the subjects in the NAM group of the PK study**

| | **S1** | **S2** | **S3** | **S4** | **S5** | **S6** | **S7** | **S8** | **S9** | **S10** |
|---|---|---|---|---|---|---|---|---|---|---|
| **Height (m)** | | | | | | | | | | |
| Day 1 | 1.68 | 1.87 | 1.77 | 1.75 | 1.83 | 1.61 | 1.83 | 1.77 | 1.65 | 1.72 |

| **Weight (kg)** | | | | | | | | | | |
|---|---|---|---|---|---|---|---|---|---|---|
| Day 1 | | 81.1 | 70.1 | 79.4 | 77.7 | 55.3 | 72.9 | 99.9 | 59.9 | 57.6 |

| **Body mass index (BMI) (kg/m²)** | | | | | | | | | | |
|---|---|---|---|---|---|---|---|---|---|---|
| Day 1 | 29.44 | 23.19 | 22.38 | 25.93 | 23.20 | 21.33 | 21.77 | 31.89 | 22.00 | 19.47 |

| **Uric acid (µmol/L)** | | | | | | | | | | |
|---|---|---|---|---|---|---|---|---|---|---|
| Day 1 | 181 | 365 | 225 | 339 | 348 | 241 | 398 | 243 | 338 | 170 |
| Day 2 | 175 | 427 | 244 | 334 | 355 | 218 | 387 | 207 | 361 | 168 |
| Day 3 | 137 | 453 | 239 | 344 | 434 | 228 | 366 | 244 | 373 | 146 |
| Day 4 | 137 | 392 | 275 | 343 | 445 | 198 | 404 | 219 | 388 | 158 |

| **Creatinine (µmol/L)** | | | | | | | | | | |
|---|---|---|---|---|---|---|---|---|---|---|
| Day 1 | 73 | 82 | 83 | 91 | 98 | 73 | 101 | 80 | 61 | 66 |
| Day 2 | 67 | 77 | 83 | 87 | 99 | 71 | 97 | 79 | 61 | 67 |
| Day 3 | 67 | 77 | 83 | 88 | 104 | 75 | 102 | 83 | 66 | 65 |
| Day 4 | 66 | 82 | 89 | 91 | 116 | 68 | 100 | 85 | 66 | 64 |

| **Estimated glomerular filtration rate (eGFR) (mL/min/1.73)** | | | | | | | | | | |
|---|---|---|---|---|---|---|---|---|---|---|
| Day 1 | 97 | 119 | 112 | 101 | 90 | 95 | 92 | 90 | 123 | 109 |
| Day 2 | 107 | 125 | 112 | 100 | 89 | 98 | | | 123 | 107 |
| Day 3 | 107 | 125 | 112 | 105 | 84 | 92 | 91 | 86 | 113 | 112 |
| Day 4 | 109 | 118 | 103 | 101 | 73 | 103 | 93 | 83 | 113 | 114 |

| **Aspartate aminotransferase (AST)** | | | | **(U/L)** | | | | | | |
|---|---|---|---|---|---|---|---|---|---|---|
| Day 1 | 24 | 19.1 | 47.9 | 25.7 | 29.7 | 13.3 | 19.0 | 15.6 | 18.6 | 21.2 |
| Day 2 | 25.9 | 27.8 | 32.9 | 24.3 | 24.2 | 10.6 | 19.5 | 14.6 | 22.1 | 21.1 |
| Day 3 | 19.7 | 20.4 | 35.0 | 25.0 | 31.0 | 17.7 | 23.8 | 18.2 | 21.0 | 26.1 |
| Day 4 | 24.3 | 38.2 | 37.6 | 26.2 | 39.9 | 17.5 | 24.2 | 16.9 | 18.9 | 21.8 |

| **Alanine aminotransferase (ALT) (U/L)** | | | | | | | | | | |
|---|---|---|---|---|---|---|---|---|---|---|
| Day 1 | 10.6 | 29.7 | 39.5 | 31.1 | 32.9 | 7.9 | 24.3 | 8.4 | 9.4 | 13.4 |
| Day 2 | 12.9 | 22.6 | 35.1 | 29.4 | 29.9 | 6.3 | 23.3 | 8.4 | 13.3 | 12.6 |
| Day 3 | 15.3 | 20.4 | 31.8 | 28.0 | 37.5 | 10.8 | 23.9 | 13.4 | 11.9 | 16.7 |
| Day 4 | 11.0 | 34.4 | 38.5 | 29.8 | 41.2 | 13.6 | 24.6 | 13.2 | 11.7 | 14.5 |

| **Gamma-glutamyl transpeptidase (γ-GT) (U/L)** | | | | | | | | | | |
|---|---|---|---|---|---|---|---|---|---|---|
| Day 1 | 12 | 19 | 27 | 18 | 32 | 9 | 32 | < 7 | 9 | 16 |
| Day 2 | 11 | 18 | 24 | 16 | 30 | 10 | 31 | < 7 | 11 | 15 |
| Day 3 | 10 | 19 | 25 | 13 | 28 | 8 | 29 | 10 | 10 | 18 |
| Day 4 | 9 | 20 | 25 | 15 | 32 | < 7 | 32 | < 7 | 10 | 16 |
| S1 - S10: subject 1 - 10. | | | | | | | | | | |

As in the FIM study, NA serum levels were mostly below the detection limit of 12 µg/L due to its rapid metabolisation to NAM (see Example 2). Therefore, only NAM levels are shown in Tables 18 and 19 and in the respective Figures 13 and 14.

Despite the 10-fold dose escalation with microencapsulated NA, the PK curves of NAM serum levels in the NA group suggested only a minor increase in exposure on a high background level (Figure 13). In the NAM group, all subjects showed significantly reduced exposure after the 900 mg and 1500 mg doses, and most also after the 3000 mg dose (Figure 14). AUC data for the subjects of the NAM group (Table 20) show that the mean exposure after ingestion of 3000 mg of microencapsulated NAM was not significantly different from that after 900 mg of free NAM. Variance between individuals increased with dose. In addition to the reduced systemic exposure, also the latency time was significantly increased with microencapsulated NAM (see Figure 14 and peak values in Table 19).

In summary, the PK results confirm that the microcapsules of the present invention lead to significantly reduced systemic exposure and delayed release compared to unformulated vitamin B3.

**Table 18: NAM serum levels of the subjects in the NA group of the PK study**

| **NAM (µg/L)** | **S1** | **S2** | **S3** | **S4** | **S5** | **S6** | **S7** | **S8** | **S9** | **S10** |
|---|---|---|---|---|---|---|---|---|---|---|
| **30 mg free NA** | | | | | | | | | | |
| **0 h** | 22.8 | 13.4 | 10.6 | 7.3 | 10.5 | 10.2 | 24.6 | 17.5 | 19.8 | 18.5 |
| **1 h** | 23.4 | 23.7 | 14.9 | 12.1 | 12.5 | 26.8 | 41.8 | 35.1 | 18.8 | 20.4 |
| **2 h** | 42.3 | 31.7 | 36.3 | 21.9 | 25.9 | 30.5 | 59.1 | 49.9 | 23.1 | 25.9 |
| **3 h** | 40.1 | 54.8 | 47.8 | 22.2 | 14.5 | 51.1 | 50.8 | 48.2 | 20.2 | 20.4 |
| **4 h** | 59.8 | 27.6 | 46.1 | 26.2 | 10.4 | 23.5 | 45.3 | 25.5 | 22.9 | 35.1 |
| **5 h** | 44.7 | 12.5 | 45.5 | 14.3 | 9.8 | 14.9 | 29.7 | 23.6 | 9.7 | 63.7 |
| **6 h** | 45.2 | 12.7 | 52.3 | 13.6 | 11.0 | 25.7 | 35.3 | 36.7 | 26.7 | 93.1 |
| **7 h** | 38.2 | 24.3 | 59.2 | 17.5 | 62.1 | 24.1 | 39.0 | 42.4 | 16.5 | 16.2 |
| **8 h** | 36.1 | 38.4 | 59.9 | 24.4 | 96.8 | 16.4 | 38.3 | 37.7 | 20.1 | 39.8 |
| **10 h** | 33.2 | 16.8 | 33.8 | 15.5 | 20.9 | 6.1 | 26.7 | 18.6 | 14.9 | 17.2 |
| **12 h** | 23.0 | 11.5 | 32.4 | 11.2 | 13.7 | 6.8 | 29.8 | 17.8 | 18.4 | 32.3 |

| **30 mg microencapsulated NA** | | | | | | | | | | |
|---|---|---|---|---|---|---|---|---|---|---|
| **0 h** | 12 | 31.9 | 14.2 | 20.8 | 22.1 | 27.8 | 17.5 | 15.3 | 17.5 | 6.2 |
| **1 h** | 47.2 | 45.7 | 51.2 | 25.0 | 35.9 | 38.0 | 23.3 | 22.4 | 17.7 | 12.7 |
| 2 **h** | 51.8 | 40.5 | 94.7 | 39.5 | 47.5 | 61.1 | 42.0 | 54.2 | 18.2 | 15.6 |
| **3 h** | 66.7 | 43.5 | 92.9 | 35.5 | 33.6 | 92.3 | 75.2 | 76.4 | 42.2 | 19.4 |
| **4 h** | 38.7 | 31.1 | 15.9 | 12.0 | 35.1 | 39.9 | 61.0 | 71.8 | 27.1 | 39.5 |
| **5 h** | 16.5 | 19.5 | 13.6 | 8.0 | 14.6 | 28.8 | 39.0 | 64.2 | 19.4 | - |
| **6 h** | 17.8 | 33.9 | 28.4 | 7.5 | 45.0 | 31.3 | 43.5 | 57.0 | 9.3 | 12.0 |
| **7 h** | 20.9 | 28.8 | 22.9 | 8.9 | 35.7 | 35.2 | 57,9 | 55.8 | 11.5 | 10.3 |
| **8 h** | 21.2 | 16.7 | 14.0 | 11.7 | 28.4 | 37.5 | 44.2 | 54.8 | 11.4 | 15.0 |
| **10 h** | 10.0 | 12.8 | 16.3 | 16.6 | 22.6 | 25.0 | 16.5 | 32.7 | 13.7 | 8.6 |
| **12 h** | 39.1 | 17.7 | 15.8 | 16.2 | 7.6 | 33.1 | 30.6 | 28.6 | 18.6 | 7.0 |

| 150 **mg microencapsulated NA** | | | | | | | | | | |
|---|---|---|---|---|---|---|---|---|---|---|
| **0 h** | 35.7 | 34.2 | 29.9 | 33.6 | 21.6 | 10.9 | 36.4 | - | 24.6 | 19.0 |
| **1 h** | 42.0 | 33.4 | 29.9 | 34.6 | 40.5 | 46.5 | 55.6 | - | 29.0 | 21.3 |
| 2 **h** | 68.9 | 32.0 | 52.4 | 40.8 | 37.7 | 56.7 | 69.9 | - | 28.8 | 16.4 |
| **3 h** | 93.2 | 32.7 | 66.4 | 53.6 | 79.1 | 74.8 | 44.4 | - | 20.9 | 32.1 |
| **4 h** | 95.4 | 29.9 | 45.3 | 77.2 | 31.5 | 40.9 | 24.4 | - | 15.6 | 47.6 |
| **5 h** | - | 17.1 | 19.9 | 31.2 | 27.6 | 26.6 | 25.9 | - | 19.2 | 69.2 |
| **6 h** | 68.6 | 18.0 | 18.3 | 20.0 | 16.7 | 28.3 | 44.5 | - | 16.0 | 50.9 |
| **7 h** | 58.2 | 19.7 | 14.9 | 16.1 | 33.2 | 31.7 | 21.1 | - | 13.5 | 25.9 |
| **8 h** | 42.8 | 14.2 | 18.6 | 18.2 | 33.5 | 30.9 | 14.3 | - | 13.3 | 19.7 |
| **10 h** | 44.5 | 21.3 | 20.5 | 21.8 | 25.8 | 12.9 | 12.8 | - | 16.4 | 19.5 |
| **12 h** | 49.5 | 21.4 | 29.9 | 20.9 | 12.7 | 23.0 | 20.7 | - | 15.7 | 12.7 |

| **300 mg microencapsulated NA** | | | | | | | | | | |
|---|---|---|---|---|---|---|---|---|---|---|
| **0 h** | 27.2 | 28.1 | 21.0 | 17.3 | 24.8 | 11.2 | 23.4 | - | 20.1 | 13.8 |
| **1 h** | 35.3 | 22.0 | 43.5 | 55.8 | 13.1 | 36.4 | 55.5 | - | 17.1 | 26.9 |
| **2 h** | 46.6 | 65.5 | 88.6 | 30.6 | 31.9 | 98.0 | 59.5 | - | 21.6 | 34.4 |
| **3 h** | 86.8 | 204.0 | 81.6 | 41.4 | 37.6 | 73.3 | 47.4 | - | 20.2 | 56.6 |
| **4 h** | 89.8 | 200.0 | 96.0 | 34.0 | 27.1 | 96.9 | 30.7 | - | 13.6 | 71.7 |
| **5 h** | 106.0 | 155.0 | 71.8 | 26.6 | 16.6 | 65.3 | 32.1 | - | 13.5 | 43.1 |
| **6 h** | 89.1 | 37.2 | 64.9 | 27.7 | 23.1 | 42.0 | 35.0 | - | 20.4 | 42.9 |
| **7 h** | 92.2 | 85.1 | 70.8 | 30.3 | 37.1 | 44.6 | 25.2 | - | 16.5 | 51.6 |
| **8 h** | 66.6 | 32.9 | 52.6 | 25.7 | 25.2 | 30.7 | 28.7 | - | 18.6 | 63.3 |
| **10 h** | 67.8 | - | 29.4 | 51.8 | 22.1 | 12.6 | 38.8 | - | 16.6 | 12.0 |
| **12 h** | 38.5 | 15.7 | 41.3 | 41.6 | 7.0 | 10.4 | 28.0 | - | 15.9 | 13.0 |
| S1 - S10: subject 1 - 10. | | | | | | | | | | |

**Table 19: NAM serum levels of the subjects in the NAM group of the PK study**

| **NAM (µg/L)** | **S1** | **S2** | **S3** | **S4** | **S5** | **S6** | **S7** | **S8** | **S9** | **S10** |
|---|---|---|---|---|---|---|---|---|---|---|
| **900 mg free NAM** | | | | | | | | | | |
| **0 h** | 14.1 | 15.3 | 4.9 | 23.0 | 16.3 | 26.8 | 14.1 | 13.0 | 30.6 | 38.4 |
| **1 h** | **19,200** | **14,500** | **19,600** | **16,700** | **16,500** | **32,400** | **15,500** | **19,700** | **21,800** | **21,000** |
| **2 h** | 16,400 | 11,900 | 15,700 | 13,800 | 13,900 | 24,200 | 11,300 | 16,000 | 20,400 | 17,500 |
| **3 h** | 11,500 | 9,190 | 12,000 | 11,100 | 10,900 | 17,500 | 9,310 | 12,000 | 16,500 | 13,800 |
| **4 h** | 8,330 | 7,350 | 7,660 | 9,300 | 8,470 | 14,600 | 7,610 | 8,410 | 13,400 | 11,400 |
| **5 h** | 5,740 | 5,300 | 5,470 | 9,270 | 6,730 | 11,000 | 5,380 | 6,930 | 9,190 | 7,730 |
| **6 h** | 3,160 | 3,640 | 3,280 | 6,370 | 5,200 | 8,150 | 3,660 | 5,010 | 7,330 | 6,670 |
| **7 h** | 1,400 | 2,670 | 1,910 | 4,600 | 4,260 | 4,820 | 2,380 | 3,360 | 5,670 | 4,450 |
| **8 h** | 437 | 994 | 532 | 3,760 | 2,980 | 2,830 | 1,280 | 2,110 | 3,790 | 2,400 |
| **10 h** | 57.2 | 189 | 79.9 | 1,860 | 945 | 459 | 245 | 355 | 920 | 565 |
| **12 h** | 25.6 | 75.9 | 48 | 366 | 250 | 40.7 | 63.0 | 47.7 | 130 | 117 |

| **900 mg microencapsulated NAM** | | | | | | | | | | |
|---|---|---|---|---|---|---|---|---|---|---|
| **0 h** | 15.3 | 11.3 | 13.3 | 9.8 | 14.0 | 7.4 | 13.4 | 10.9 | 15.9 | 15.4 |
| **1 h** | 34.8 | 21.5 | 20.8 | 36.5 | 45.2 | 5.2 | 17.9 | 17.7 | 27.9 | 22.8 |
| 2 **h** | 615 | 50.1 | 87.6 | 93.6 | 84.0 | 67.6 | 26.9 | 63.0 | 52.6 | 36.4 |
| **3 h** | 626 | 348 | 510 | 919 | 540 | 2,090 | 210 | 348 | 881 | 199 |
| **4 h** | **927** | **830** | **671** | **2,030** | **1,370** | **1,670** | **1,150** | **1,780** | **2,180** | 72.3 |
| **5 h** | 617 | 587 | 468 | 1,610 | 1,170 | 1,080 | 984 | 321 | 1,930 | 17.1 |
| **6 h** | 343 | 304 | 230 | 1,370 | 1,220 | 366 | 202 | 165 | 1,400 | 42.3 |
| **7 h** | 79.6 | 135 | 152 | 1,110 | 817 | 144 | 69.7 | 84.9 | 1,060 | 35.0 |
| **8 h** | 45.0 | 219 | 161 | 401 | 360 | 25.5 | 51.5 | 63.4 | 380 | 24.5 |
| **10 h** | 31.4 | 18.3 | 46.2 | 80.0 | 84.4 | 18.0 | 29.5 | 30.0 | 116 | 20.8 |
| **12 h** | 28.0 | 18.8 | 35.9 | 46.9 | 53.5 | 18.7 | 18.8 | 32.0 | 73.3 | 45.5 |

| **1500 mg microencapsulated NAM** | | | | | | | | | | |
|---|---|---|---|---|---|---|---|---|---|---|
| **0 h** | 21.5 | 24.6 | 30.2 | 23.6 | 27.0 | 25.3 | 21.1 | 18.7 | 49 | 45.1 |
| **1 h** | 30.7 | 33.3 | 31.9 | 35.9 | 35.5 | 34.8 | 14.1 | 30.2 | 38.2 | 37.4 |
| **2 h** | 149 | 180 | 146 | 116 | 209 | 165 | 73.9 | 311 | 296 | 326 |
| **3 h** | 1,220 | 1,280 | 1,240 | 585 | 1,190 | 1,610 | 882 | 1,780 | 3,010 | 2,640 |
| **4 h** | **3,600** | 2,350 | **2,270** | 936 | 5,640 | 3,540 | **1,880** | **3,610** | **5,310** | **4,500** |
| **5 h** | 3,000 | **2,820** | 1,710 | 1,310 | **10,700** | **4,220** | 1,650 | 3,420 | 5,300 | 3,920 |
| **6 h** | 2,390 | 2,230 | 1,170 | **1,540** | 10,500 | 3,290 | 1,190 | 3,230 | 5,140 | 3,010 |
| **7 h** | - | 1,880 | 951 | 1,140 | 9,980 | 2,360 | 513 | 2,420 | 4,560 | 2,390 |
| **8 h** | - | 1,320 | 543 | 378 | 8,810 | 1,490 | 278 | 1,630 | 4,000 | 1,710 |
| **10 h** | - | 290 | 96.0 | 214 | 5,360 | 228 | 48.5 | 929 | 2,330 | 443 |
| **12 h** | 38.5 | 40.3 | 39.0 | 87.5 | 2,820 | 79.9 | 26.1 | 269 | 975 | 131 |

| **3000 mg microencapsulated NAM** | | | | | | | | | | |
|---|---|---|---|---|---|---|---|---|---|---|
| **0 h** | 18.8 | 19.7 | 21.1 | 30.6 | 26.2 | 27.3 | 16.8 | 23.4 | 21.5 | 40.4 |
| **1 h** | 20.8 | 51.9 | 26.8 | 48.0 | 38.0 | 36.8 | 13.9 | 22.1 | 42.3 | 37.1 |
| **2 h** | 331 | 240 | 147 | 429 | 211 | 738 | 125 | 239 | 974 | 385 |
| **3 h** | 4,400 | 2,260 | 57.4 | 2,220 | 2,300 | 6,370 | 2,590 | 3284 | 9,760 | 6,550 |
| **4 h** | 10,000 | 7,700 | 6,160 | 3,560 | 6,910 | 12,600 | 6,330 | 9,000 | 22,200 | 14,400 |
| **5 h** | 12,600 | 17,400 | 7,660 | 3,630 | 8,450 | 15,300 | 11,100 | 10,680 | 25,000 | **16,600** |
| **6 h** | **12,800** | 26,400 | 7,990 | 2,880 | 9,840 | 16,600 | **11,900** | 12,600 | 25,400 | 11,440 |
| **7 h** | 12,500 | **36,700** | **8,070** | 1,870 | **10,500** | 17,800 | 10,800 | **12,800** | **25,700** | 13,100 |
| **8 h** | 11,300 | 33,200 | 7,130 | 1,960 | 9,040 | **18,000** | 9,360 | 12,700 | 23,900 | 12,800 |
| **10 h** | 6,780 | 29,300 | 4,180 | 673 | 7,920 | 14,000 | 6,830 | 8,840 | 20,500 | 8,240 |
| **12 h** | 101 | 24,100 | 1,740 | 251 | 5,980 | 10,300 | 3,870 | 6,770 | 16,400 | 6,060 |
| **Bold** figures represent peak values; | | | | | | | | | | |
| S 1 - S 10: subject 1 - subject 10 | | | | | | | | | | |

**Table 20: AUC data of the subjects in the NAM group of the PK study**

| | **900 mg free** | **900 mg enc.¹** | **1500 mg enc.** | **3000 mg enc. (non-extrap.²)** | **3000 mg enc. (extrap.³)** |
|---|---|---|---|---|---|
| **Subject 1** | 66533 | 3408 | 16941 | 83272 | 83273 |
| **Subject 2** | 56503 | 2665 | 13386 | 223262 | 369970 |
| **Subject 3** | 66628 | 2516 | 8580 | 50917 | 52023 |
| **Subject 4** | 80878 | 7982 | 6757 | 19189 | 19460 |
| **Subject 5** | 72578 | 6016 | 65023 | 73642 | 99359 |
| **Subject 6** | 117887 | 5519 | 18003 | 134758 | 167844 |
| **Subject 7** | 57620 | 2822 | 6754 | 74437 | 79216 |
| **Subject 8** | 75339 | 2972 | 19383 | 92137 | 107463 |
| **Subject 9** | 101960 | 8415 | 35314 | 202337 | 260041 |
| **Subject 10** | 87416 | 556,5 | 20428 | 104272 | 117363 |
| **Mean** | 78334,20 | 4287,15 | 21011,90 | 105822,30 | 135601,20 |
| **SD** | 19538,11 | 2571,91 | 17616,94 | 67276,47 | 105558,36 |
| **p vs. 900 mg free NAM** | not applicable | p = 0.000* | p = 0.000* | p = 0.201 | p = 0.059 |
| ¹enc., microencapsulated; | | | | | |
| ²non-extrap., non-extrapolated (raw) data; | | | | | |
| ³extrap., extrapolated: if serum levels after 12 hours were above baseline, curves were extrapolated using trend lines as described in the Methods section; | | | | | |
| *significant difference (p < 0.05), p values refer to the comparison with the area under the curve (AUC) after ingestion of 900 mg of free NAM (paired T-test for the normally distributed data of 900 mg, 1500 mg and 3000 mg of microencapsulated NAM without extrapolation; Wilcoxon test for the non-normally distributed extrapolated data for 3000 mg of free NAM). | | | | | |

### Example 5: NAM serum levels in relation to the metabolic state

### Aim of the study

To investigate whether patients with obesity and type 2 diabetes (T2D) have altered systemic NA or NAM levels.

### Methods

### Study population and design

The study was performed at the Department of Internal Medicine 1 of the University Hospital Schleswig-Holstein, Campus Kiel (Kiel, Germany). The study was approved by the ethics committee of the University of Kiel, and written informed consent was obtained from each volunteer.

To gain insights into the physiology of Trp, NA and NAM in human obesity and T2D, n = 511 subjects were recruited from the FoCus cohort, a cohort established in 2011-2015 in Kiel (Germany) to examine genetic and environmental factors in the pathogenesis of nutrition-associated diseases. The 511 subjects were subdivided into four groups: (1) underweight [body mass index (BMI) <20 kg/m², n = 66], (2) lean (BMI 20-25 kg/m², n = 149), (3) obese without T2D (BMI>30 kg/m², n = 148) and (4) obese with T2D (BMI>30 kg/m², n = 148). Obese and lean groups were matched by age and sex. The baseline characteristics of the study population are summarised in Table 21.

**Table 21: Baseline characteristics of the study population**

| | **BMI < 20 kg/m²** | **BMI 20-25 kg/m²** | **BMI > 30 kg/m²** | **BMI > 30 kg/m²** |
|---|---|---|---|---|
| | n = 66 | n = 149 | without T2D n = 148 | with T2D n = 148 |
| **Age (years)** | 45.53 ± 15.58^{a} | 52.90 ± 10.82 | 52.82 ± 10.86 | 52.93 ± 10.83 |
| **Gender (% female)** | 86.4 % | 67.1 % | 66.9 % | 66.2 % |
| **Height (m)** | 1.70° (1.64; 1.75) | 1.72 (1.68; 1.79) | 1.70 (1.64; 1.78) | 1.70 (1.64; 1.80) |
| **Weight (kg)** | 55.45 (50.38; 58.43) | 66.80 (61.35; 74.40) | 111.30 (95.73; 132.20) | 123.25 (102.50; 148.08) |
| **BMI (kg/m²)** | 19.09 (18.18; 19.73) | 22.81 (21.45; 23.98) | 37.08 (32.40; 45.13) | 42.80 (36.75; 47.94) |
| **Blood pressure [mmHg (sys)]** | 120.00 (110.00; 130.00) | 120.00 (115.00; 130.00) | 130.00 (130.00; 140.00) | 140.00 (130.00; 140.00) |
| **Blood pressure [mmHg (dia)]** | 80.00 (70.00; 80.00) | 80.00 (70.00; 80.00) | 80.00 (80.00; 90.00) | 80.00 (80.00; 90.00) |
| **Glucose (mg/dl)** | 88.00 (85.00; 95.25) | 93.00 (87.00; 99.00) | 100.00 (91.00; 108.00) | 123.00 (104.25; 162.00) |
| **Insulin (mIU/I)** | 5.25 (3.90; 8.30) | 6.50 (5.10; 9.28) | 17.20 (11.00; 24.23) | 25.50 (15.53; 43.88) |
| **HOMA-IR index** | 1.14 (0.83; 1.89) | 1.49 (1.08; 2.26) | 4.15 (2.57; 5.71) | 7.87 (4.18; 16.94) |
| **Triglycerides (mg/dl)** | 68.00 (54.75; 92.50) | 87.00 (64.00; 112.50) | 121.50 (91.25; 179.25) | 166.00 (122.00; 237.75) |
| **Lp(a) (mg/l)** | 117.00 (95.00; 361.00) | 121.00 (95.00; 320.00) | 148.00 (95.00; 378.50) | 108.00 (95.00; 324.25) |
| **IL-6 (pg/ml)** | 2.15 (1.50; 3.73) | 2.50 (1.50; 4.20) | 4.00 (2.90; 5.35) | 5.20 (3.33; 7.08) |
| **CRP (mg/l)** | 0.90 (0.90; 1.13) | 0.90 (0.90; 1.90) | 4.35 (1.75; 8.65) | 5.85 (2.90; 10.88) |
| ^{a}Mean ± standard deviation (all such values); ^{b}median and (25^{th}; 75^{th}) percentiles (all such values); BMI, body mass index; CRP, C-reactive protein; dia, diastolic blood pressure; HOMA-IR index, Homeostasis Model Assessment Insulin Resistance Index; IL-6, interleukin-6; Lp(a), lipoprotein (a); mmHg, mm of mercury column; n.s., not significant.; sys, systolic blood pressure. | | | | |

### Estimation of Trp and vitamin B3 intake

The average nutritional intake of Trp, vitamin B3 (niacin; NA and NAM) and niacin equivalents (NE) in the study subjects was estimated by using the 12-month retrospective EPIC food frequency questionnaires and the EPICsoft database (Kroke et al. 1999, Am J. Clin. Nutr. 70:439; Schulz et al. 2008, Br. J. Nutr. 100:942). NE intake is commonly calculated as niacin intake (mg) plus 1/60 Trp intake (mg) (Horwitt et al. 1981, Am. J. Clin. Nutr. 34:423; SCF (European Scientific Committee on Food) 2002, SCF/CS/NUT/UPPLEV/39 Final 6 May 2002).

### Measurements of NAM serum concentrations

NAM levels in serum were determined by LC-MS/MS at an external reference laboratory (Medizinisches Labor Bremen, www.mlhb.de; see Example 1).

### Results and discussion

No significant difference in the nutritional intake of niacin, Trp or NE was observed between the lean, the obese and the obese and T2D group. Underweight individuals, however, were found to have a (partly significantly) lower intake of niacin, Trp and NE compared to the other three groups, which is most likely due to a generally reduced food intake in underweight subjects. Still, NE intake of all underweight subjects was adequate according to intake recommendations (data not shown).

Serum concentrations of Trp, NA and NAM were measured by liquid chromatography and mass spectrometry in all 511 study subjects. With respect to Trp, the results are in line with the food frequency questionnaires, showing deviations only in the underweight group (Figure 15A). In contrast to Trp, major differences between the groups were observed for NAM serum levels (Figure 15B): underweight subjects showed the highest levels, followed by lean subjects and obese subjects without T2D. The lowest NAM serum levels were observed in obese subjects with T2D (Figure 15B). As in the FIM study (Example 2) and in the PK study (Example 4), NA serum levels were almost all below the detection limit of 12 µg/L due to its rapid metabolisation to NAM and are therefore not shown.

Taken together with the beneficial increase of Bacteroidetes observed in overweight subjects in response to vitamin B3 formulations of the present invention (Example 2), the finding that obese subjects, especially those with T2D, obviously have a vitamin B3 deficit despite sufficient nutritional intake and sufficient Trp levels strongly supports the notion of the present invention that vitamin B3 nutritional supplementation targeting the intestinal microbiota holds great promise for the treatment of gut microbiota-associated disorders.

## Claims

1. A microcapsule comprising a core containing vitamin B3 (1, 2), **characterised by** a coating layer system comprising an inner layer of shellac (3), an outer layer of shellac (4) and a pH-modulating substance (5, 6) provided between the two layers of shellac (3, 4).

2. The microcapsule according to claim 1, **characterised in that** vitamin B3 comprises or consists of nicotinic acid, and the pH-modulating substance comprises or consists of a basic substance, preferably selected from the group consisting of hydrogen carbonate, carbonate salts, acetate salts, and their mixtures.

3. The microcapsule according to claim 1, **characterised in that** vitamin B3 comprises or consists of nicotinamide, and the pH-modulating substance comprises or consists of an acidic substance, preferably selected from the group consisting of organic acids, inorganic acids, and their mixtures.

4. The microcapsule according to claim 1 or 2, **characterised in that** the pH-modulating substance comprises or consists of sodium hydrogen carbonate (sodium bicarbonate).

5. The microcapsule according to claim 1 or 3, **characterised in that** the pH-modulating substance comprises or consists of citric acid.

6. The microcapsule according to any one of claims 1 to 5, for use as a medicament, nutraceutical, dietary supplement, food ingredient or food.

7. The microcapsule according to any one of claims 1 to 6, for use for beneficially influencing the intestinal microbiota and/or for beneficially influencing or preventing unfavourable and/or abnormal and/or imbalanced blood and/or plasma and/or serum lipid levels and/or for beneficially influencing or preventing intestinal inflammation and/or other unfavourable and/or abnormal changes in the intestine, wherein the microcapsule releases vitamin B3 for at least partial topical efficacy in the lower small intestine, preferably in the terminal ileum, and/or in the colon.

8. The microcapsule according to any one of claims 1 to 7, for use in the therapy and/or prophylaxis of
a disease and/or syndrome associated with and/or accompanied by unfavourable or abnormal or imbalanced intestinal microbiota and/or
a disease and/or syndrome associated with and/or accompanied by unfavourable or abnormal or imbalanced blood and/or plasma and/or serum lipid levels, preferably in non-alcoholic fatty liver disease (NAFLD) and/or non-alcoholic steatohepatitis (NASH), for decreasing the liver fat content and/or beneficially influencing blood and/or plasma and/or serum lipid levels, and/or
a disease and/or syndrome associated with and/or accompanied by intestinal inflammation, preferably in inflammatory bowel diseases (IBD), and/or
other unfavourable or abnormal changes in the intestine, and/or
one or more selected from the group consisting of lipid metabolism disorders, dyslipidemia, NAFLD, NASH, cardiovascular diseases, arteriosclerosis, atherosclerosis, metabolic syndrome, obesity, diabetes, inflammatory diseases of the small intestine and/or colon, IBD, Crohn's disease, ulcerative colitis, indeterminate colitis, irritable bowel syndrome, colon carcinoma, psoriasis, allergy, atopic eczema, asthma, chronic obstructive pulmonary disease, cystic fibrosis, and other diseases and/or syndromes associated with and/or accompanied by unfavourable or abnormal or imbalanced blood and/or plasma and/or serum lipid levels and/or intestinal inflammation and/or other unfavourable or abnormal changes in the intestine and/or unfavourable or abnormal changes in the intestinal microbiota and/or an impaired interaction between the intestinal microbiota and the intestine.

9. The microcapsule according to any one of claims 1 to 8, comprising variable and/or fixed dose combinations of nicotinic acid and/or nicotinamide and/or one or more other active substances, preferably within the core of the capsule, and preferably being selected from the group of probiotics; prebiotics; synbiotics; polyphenols; substances, proteins and/or enzymes supporting probiotics; antibiotic, antimycotic, antiprotozoal, antihelminthic, antiviral or anti-inflammatory agents; aminosalicylates; acetylsalicylic acid; prostaglandin D2 antagonists, preferably laropiprant; short-chain fatty acids; medium-chain fatty acids; systemic or topical corticosteroids; β2-adrenergic receptor agonists; theophylline and other substances of the xanthine family; statins, preferably selected from the group consisting of atorvastatin, cerivastatin, fluvastatin, lovastatin, mevastatin, pitavastatin, pravastatin, rosuvastatin and simvastatin; small molecules; peptides, biologicals, fusion proteins, monoclonal antibodies or derivatives thereof.

10. A composition comprising a microcapsule according to any one of claims 1 to 9.

11. The composition according to claim 10, formulated for oral administration with controlled and/or delayed release of vitamin B3.

12. The composition according to claim 11, **characterised in that** the oral application is performed with an active substance content for nicotinic acid and/or nicotinamide of 1-5000 mg each per finished dosage form, preferably with an active substance content of 30-3000 mg each per finished dosage form.

13. The composition according to any one of claims 10 to 12, comprising variable and/or fixed dose combinations with one or more other active substances and/or compositions, preferably being selected from the group of probiotics; prebiotics; synbiotics; polyphenols; substances, proteins and/or enzymes supporting probiotics; antibiotic, antimycotic, antiprotozoal, antihelminthic, antiviral or anti-inflammatory agents; aminosalicylates; acetylsalicylic acid; prostaglandin D2 antagonists, preferably laropiprant; short-chain fatty acids; medium-chain fatty acids; systemic or topical corticosteroids; β2-adrenergic receptor agonists; theophylline and other substances of the xanthine family; statins, preferably selected from the group consisting of atorvastatin, cerivastatin, fluvastatin, lovastatin, mevastatin, pitavastatin, pravastatin, rosuvastatin and simvastatin; small molecules; peptides, biologicals, fusion proteins, monoclonal antibodies or derivatives thereof.

14. The composition according to any one of claims 10 to 13, for use for beneficially influencing the intestinal microbiota and/or for beneficially influencing or preventing unfavourable and/or abnormal and/or imbalanced blood and/or plasma and/or serum lipid levels and/or for beneficially influencing or preventing intestinal inflammation and/or other unfavourable and/or abnormal changes in the intestine, and/or for use in the therapy and/or prophylaxis of
a disease and/or syndrome associated with and/or accompanied by unfavourable or abnormal or imbalanced intestinal microbiota and/or
a disease and/or syndrome associated with and/or accompanied by unfavourable or abnormal or imbalanced blood and/or plasma and/or serum lipid levels, preferably in non-alcoholic fatty liver disease (NAFLD) and/or non-alcoholic steatohepatitis (NASH), for decreasing the liver fat content and/or beneficially influencing blood and/or plasma and/or serum lipid levels, and/or
a disease and/or syndrome associated with and/or accompanied by intestinal inflammation, preferably in inflammatory bowel diseases (IBD), and/or
other unfavourable or abnormal changes in the intestine, and/or
one or more selected from the group consisting of lipid metabolism disorders, dyslipidemia, NAFLD, NASH, cardiovascular diseases, arteriosclerosis, atherosclerosis, metabolic syndrome, obesity, diabetes, inflammatory diseases of the small intestine and/or colon, IBD, Crohn's disease, ulcerative colitis, indeterminate colitis, irritable bowel syndrome, colon carcinoma, psoriasis, allergy, atopic eczema, asthma, chronic obstructive pulmonary disease, cystic fibrosis, and other diseases and/or syndromes associated with and/or accompanied by unfavourable or abnormal or imbalanced blood and/or plasma and/or serum lipid levels and/or intestinal inflammation and/or other unfavourable or abnormal changes in the intestine and/or unfavourable or abnormal changes in the intestinal microbiota and/or an impaired interaction between the intestinal microbiota and the intestine.

15. A method for producing a microcapsule according to any one of claims 1 to 9, or a composition according to any one of claims 10 to 14, comprising the steps of
a. providing a core material comprising or consisting of vitamin B3,
b. coating the core material with a first shellac layer,
c. providing a pH-modulating substance,
d. applying the pH-modulating substance onto the first shellac layer, and
e. applying a second shellac layer.

## Patentansprüche

1. Mikrokapsel, umfassend einen Vitamin B3 enthaltenden Kern (1, 2), **gekennzeichnet durch** ein Überzugssystem, umfassend eine innere Schellackschicht (3), eine äußere Schellackschicht (4) und eine zwischen den beiden Schellackschichten (3, 4) bereitgestellte pH-modulierende Substanz (5, 6).

2. Mikrokapsel nach Anspruch 1, **dadurch gekennzeichnet, dass** das Vitamin B3 Nicotinsäure umfasst oder daraus besteht und die pH-modulierende Substanz eine basische Substanz, vorzugsweise aus der aus Hydrogencarbonat, Carbonatsalzen, Acetatsalzen und deren Mischungen bestehenden Gruppe, umfasst oder daraus besteht.

3. Mikrokapsel nach Anspruch 1, **dadurch gekennzeichnet, dass** das Vitamin B3 Nicotinamid umfasst oder daraus besteht und die pH-modulierende Substanz eine saure Substanz, vorzugsweise ausgewählt aus der aus organischen Säuren, anorganischen Säuren und deren Mischungen bestehenden Gruppe, umfasst oder daraus besteht.

4. Mikrokapsel nach Anspruch 1 oder 2, **dadurch gekennzeichnet, dass** die pH-modulierende Substanz Natriumhydrogencarbonat (Natriumbicarbonat) umfasst oder daraus besteht.

5. Mikrokapsel nach Anspruch 1 oder 3, **dadurch gekennzeichnet, dass** die pH-modulierende Substanz Citronensäure umfasst oder daraus besteht.

6. Mikrokapsel nach einem der Ansprüche 1 bis 5 zur Verwendung als Medikament, Nutrazeutikum, Nahrungsergänzungsmittel, Nahrungsbestandteil oder Nahrung.

7. Mikrokapsel nach einem der Ansprüche 1 bis 6 zur Verwendung zur günstigen Beeinflussung der Darmmikrobiota und/oder zur günstigen Beeinflussung oder zur Verhinderung ungünstiger und/oder anormaler und/oder unausgeglichener Blut- und/oder Plasma- und/oder Serumlipidspiegel und/oder zur günstigen Beeinflussung oder Prävention von Darmentzündung und/oder anderen ungünstigen und/oder anormalen Veränderungen im Darm, wobei die Mikrokapsel Vitamin B3 mit mindestens partieller topischer Wirksamkeit im unteren Dünndarm, vorzugsweise im terminalen Ileum und/oder im Kolon, freisetzt.

8. Mikrokapsel nach einem der Ansprüche 1 bis 7 zur Verwendung in der Therapie und/oder Prophylaxe von
einer mit ungünstigen oder anormalen oder unausgeglichenen Darmmikrobiota assoziierten und/oder davon begleiteten Krankheit und/oder einem solchen Syndrom und/oder
einer mit ungünstigen oder anormalen oder unausgeglichenen Blut- und/oder Plasma- und/oder Serumlipidspiegeln assoziierten und/oder davon begleiteten Krankheit und/oder einem solchen Syndrom, vorzugsweise bei nichtalkoholischer Fettleberkrankheit (Non-Alcoholic Fatty Liver Disease, NAFLD) und/oder nichtalkoholischer Steatohepatitis (NASH), zur Verminderung des Leberfettgehalts und/oder zur günstigen Beeinflussung von Blut- und/oder Plasma- und/oder Serumlipidspiegeln, und/oder
einer mit Darmentzündung assoziierten und/oder davon begleiteten Krankheit und/oder einem solchen Syndrom, vorzugsweise bei entzündlichen Darmkrankheiten (Inflammatory Bowel Diseases, IBD), und/oder
anderen ungünstigen oder anormalen Veränderungen im Darm und/oder
einem oder mehreren ausgewählt aus der Gruppe bestehend aus Lipidstoffwechselstörungen, Dyslipidämie, NAFLD, NASH, Herz-Kreislauf-Krankheiten, Arteriosklerose, Atherosklerose, metabolischem Syndrom, Obesitas, Diabetes, entzündlichen Krankheiten vom Dünndarm und/oder Kolon, IBD, Morbus Crohn, Colitis ulcerosa, unbestimmter Colitis, Reizdarmsyndrom, Kolonkarzinom, Psoriasis, Allergie, atopischem Ekzem, Asthma, chronischer obstruktiver Lungenkrankheit, zystischer Fibrose und anderen mit ungünstigen oder anormalen oder unausgeglichenen Blut- und/oder Plasma- und/oder Serumlipidspiegeln und/oder Darmentzündung und/oder anderen ungünstigen oder anormalen Veränderungen im Darm und/oder ungünstigen oder anormalen Veränderungen in der Darmmikrobiota und/oder einer gestörten Wechselwirkung zwischen der Darmmikrobiota und dem Darm assoziierten und/oder davon begleiteten Krankheiten und/oder Syndromen.

9. Mikrokapsel nach einem der Ansprüche 1 bis 8, umfassend variable Kombinationen und/oder Kombinationen mit festgelegter Dosis von Nicotinsäure und/oder Nicotinamid und/oder einer oder mehreren anderen Wirksubstanzen, vorzugsweise im Kern der Kapsel, und vorzugsweise ausgewählt aus der Gruppe der Probiotika; Präbiotika; Synbiotika; Polyphenole; Substanzen, Proteine und/oder Enzyme, die Probiotika unterstützen; antibiotischen, antimykotischen, antiprotozoischen, antihelminthischen, antiviralen oder entzündungshemmenden Mitteln; Aminosalicylaten; Acetylsalicylsäure; Prostaglandin-D2-Antagonisten, vorzugsweise Laropiprant; kurzkettigen Fettsäuren; mittelkettigen Fettsäuren; systemischen oder topischen Kortikosteroiden; Agonisten des β2-adrenergen Rezeptors; Theophyllin und anderen Substanzen der Xanthinfamilie; Statinen, vorzugsweise ausgewählt aus der Gruppe bestehend aus Atorvastatin, Cerivastatin, Fluvastatin, Lovastatin, Mevastatin, Pitavastatin, Pravastatin, Rosuvastatin und Simvastatin; kleinen Molekülen; Peptiden, Biologika, Fusionsproteinen, monoklonalen Antikörpern oder Derivaten davon.

10. Zusammensetzung, umfassend eine Mikrokapsel nach einem der Ansprüche 1 bis 9.

11. Zusammensetzung nach Anspruch 10, formuliert für die orale Verabreichung mit gesteuerter und/oder verzögerter Freisetzung von Vitamin B3.

12. Zusammensetzung nach Anspruch 11, **dadurch gekennzeichnet, dass** die orale Anwendung mit einem Wirksubstanzgehalt von Nicotinsäure und/oder Nicotinamid von jeweils 1-5000 mg pro fertiger Dosierungsform erfolgt, vorzugsweise mit einem Wirksubstanzgehalt von jeweils 30-3000 mg pro fertiger Dosierungsform.

13. Zusammensetzung nach einem der Ansprüche 10 bis 12, umfassend variable Kombinationen und/oder Kombinationen mit festgelegter Dosis mit einer oder mehreren anderen Wirksubstanzen und/oder Zusammensetzungen, vorzugsweise ausgewählt aus der Gruppe der Probiotika; Präbiotika; Synbiotika; Polyphenole; Substanzen, Proteine und/oder Enzyme, die Probiotika unterstützen; antibiotischen, antimykotischen, antiprotozoischen, antihelminthischen, antiviralen oder entzündungshemmenden Mitteln; Aminosalicylaten; Acetylsalicylsäure; Prostaglandin-D2-Antagonisten, vorzugsweise Laropiprant; kurzkettigen Fettsäuren; mittelkettigen Fettsäuren; systemischen oder topischen Kortikosteroiden; Agonisten des β2-adrenergen Rezeptors; Theophyllin und anderen Substanzen der Xanthinfamilie; Statinen, vorzugsweise ausgewählt aus der Gruppe bestehend aus Atorvastatin, Cerivastatin, Fluvastatin, Lovastatin, Mevastatin, Pitavastatin, Pravastatin, Rosuvastatin und Simvastatin; kleinen Molekülen; Peptiden, Biologika, Fusionsproteinen, monoklonalen Antikörpern oder Derivaten davon.

14. Zusammensetzung nach einem der Ansprüche 10 bis 13 zur Verwendung zur günstigen Beeinflussung der Darmmikrobiota und/oder zur günstigen Beeinflussung oder Prävention ungünstiger und/oder anormaler und/oder unausgeglichener Blut- und/oder Plasma- und/oder Serumlipidspiegel und/oder zur günstigen Beeinflussung oder Prävention von Darmentzündung und/oder anderen ungünstigen und/oder anormalen Veränderungen im Darm und/oder zur Verwendung bei der Therapie und/oder Prophylaxe von
einer mit ungünstigen oder anormalen oder unausgeglichenen Darmmikrobiota assoziierten und/oder davon begleiteten Krankheit und/oder einem solchen Syndrom und/oder
einer mit ungünstigen oder anormalen oder unausgeglichenen Blut- und/oder Plasma- und/oder Serumlipidspiegeln assoziierten und/oder davon begleiteten Krankheit und/oder einem solchen Syndrom, vorzugsweise bei nichtalkoholischer Fettleberkrankheit (Non-Alcoholic Fatty Liver Disease, NAFLD) und/oder nichtalkoholischer Steatohepatitis (NASH), zur Verminderung des Leberfettgehalts und/oder zur günstigen Beeinflussung von Blut- und/oder Plasma- und/oder Serumlipidspiegeln, und/oder
einer mit Darmentzündung assoziierten und/oder davon begleiteten Krankheit und/oder einem solchen Syndrom, vorzugsweise bei entzündlichen Darmkrankheiten (Inflammatory Bowel Diseases, IBD), und/oder
anderen ungünstigen oder anormalen Veränderungen im Darm und/oder
einem oder mehreren ausgewählt aus der Gruppe bestehend aus Lipidstoffwechselstörungen, Dyslipidämie, NAFLD, NASH, Herz-Kreislauf-Krankheiten, Arteriosklerose, Atherosklerose, metabolischem Syndrom, Obesitas, Diabetes, entzündlichen Krankheiten vom Dünndarm und/oder Kolon, IBD, Morbus Crohn, Colitis ulcerosa, unbestimmter Colitis, Reizdarmsyndrom, Kolonkarzinom, Psoriasis, Allergie, atopischem Ekzem, Asthma, chronischer obstruktiver Lungenkrankheit, zystischer Fibrose und anderen mit ungünstigen oder anormalen oder unausgeglichenen Blut- und/oder Plasma- und/oder Serumlipidspiegeln und/oder Darmentzündung und/oder anderen ungünstigen oder anormalen Veränderungen im Darm und/oder ungünstigen oder anormalen Veränderungen in der Darmmikrobiota und/oder einer gestörten Wechselwirkung zwischen der Darmmikrobiota und dem Darm assoziierten und/oder davon begleiteten Krankheiten und/oder Syndromen.

15. Verfahren zur Herstellung einer Mikrokapsel nach einem der Ansprüche 1 bis 9 oder einer Zusammensetzung nach einem der Ansprüche 10 bis 14, umfassend die folgenden Schritte:
a. die Bereitstellung eines Vitamin B3 umfassenden oder daraus bestehenden Kernmaterials,
b. die Beschichtung des Kernmaterials mit einer ersten Schellackschicht,
c. die Bereitstellung einer pH-modulierenden Substanz,
d. das Aufbringen der pH-modulierenden Substanz auf die erste Schellackschicht und
e. das Aufbringen einer zweiten Schellackschicht.

## Revendications

1. Microcapsule comprenant un noyau contenant de la vitamine B3 (1, 2), **caractérisée par** un système de couches d'enrobage comprenant une couche interne de gomme laque (3), une couche externe de gomme laque (4) et une substance modulant le pH (5, 6) fournie entre les deux couches de gomme laque (3, 4).

2. Microcapsule selon la revendication 1, **caractérisée en ce que** la vitamine B3 comprend ou consiste en l'acide nicotinique, et la substance modulant le pH comprend ou consiste en une substance basique, de préférence sélectionnée dans le groupe consistant en l'hydrogénocarbonate, les sels de carbonate, les sels d'acétate, et leurs mélanges.

3. Microcapsule selon la revendication 1, **caractérisée en ce que** la vitamine B3 comprend ou consiste en le nicotinamide, et la substance modulant le pH comprend ou consiste en une substance acide, de préférence sélectionnée dans le groupe consistant en les acides organiques, les acides inorganiques, et leurs mélanges.

4. Microcapsule selon la revendication 1 ou 2, **caractérisée en ce que** la substance modulant le pH comprend ou consiste en l'hydrogénocarbonate de sodium (bicarbonate de sodium).

5. Microcapsule selon la revendication 1 ou 3, **caractérisée en ce que** la substance modulant le pH comprend ou consiste en l'acide citrique.

6. Microcapsule selon l'une quelconque des revendications 1 à 5, pour une utilisation comme médicament, nutraceutique, complément alimentaire, ingrédient alimentaire ou aliment.

7. Microcapsule selon l'une quelconque des revendications 1 à 6, pour une utilisation pour influencer de manière bénéfique le microbiote intestinal et/ou pour influencer ou prévenir de manière bénéfique des taux de lipides dans le sang et/ou le plasma et/ou le sérum défavorables et/ou anormaux et/ou déséquilibrés et/ou pour influencer ou prévenir de manière bénéfique une inflammation intestinale et/ou d'autres changements défavorables et/ou anormaux dans l'intestin, dans laquelle la microcapsule libère de la vitamine B3 pour une efficacité topique au moins partielle dans l'intestin grêle inférieur, de préférence dans l'iléon terminal, et/ou dans le côlon.

8. Microcapsule selon l'une quelconque des revendications 1 à 7, pour une utilisation dans la thérapie et/ou la prophylaxie
d'une maladie et/ou d'un syndrome associé à et/ou accompagné d'un microbiote intestinal défavorable ou anormal ou déséquilibré et/ou
d'une maladie et/ou d'un syndrome associé à des et/ou accompagné de taux de lipides dans le sang et/ou le plasma et/ou le sérum défavorables ou anormaux ou déséquilibrés, de préférence dans la maladie du foie gras non alcoolique (NAFLD) et/ou la stéatohépatite non alcoolique (NASH), pour diminuer la teneur en graisse hépatique et/ou influencer de manière bénéfique les taux de lipides dans le sang et/ou le plasma et/ou le sérum, et/ou
d'une maladie et/ou d'un syndrome associé à et/ou accompagné d'une inflammation intestinale, de préférence dans les affections abdominales inflammatoires (IBD), et/ou
d'autres changements défavorables ou anormaux dans l'intestin, et/ou
d'un ou de plusieurs sélectionnées dans le groupe consistant en les troubles du métabolisme des lipides, la dyslipidémie, la NAFLD, la NASH, les maladies cardiovasculaires, l'artériosclérose, l'athérosclérose, le syndrome métabolique, l'obésité, le diabète, les maladies inflammatoires de l'intestin grêle et/ou du côlon, l'IBD, la maladie de Crohn, la rectocolite hémorragique, une colite indéterminée, le syndrome du côlon irritable, un carcinome du côlon, le psoriasis, une allergie, un eczéma atopique, l'asthme, une bronchopneumopathie chronique obstructive, une fibrose kystique, et d'autres maladies et/ou syndromes associés à des et/ou accompagnés de taux de lipides dans le sang et/ou le plasma et/ou le sérum défavorables ou anormaux ou déséquilibrés et/ou une inflammation intestinale et/ou d'autres changements défavorables ou anormaux dans l'intestin et/ou des changements défavorables ou anormaux du microbiote intestinal et/ou une interaction altérée entre le microbiote intestinal et l'intestin.

9. Microcapsule selon l'une quelconque des revendications 1 à 8, comprenant des combinaisons de doses variables et/ou fixes d'acide nicotinique et/ou de nicotinamide et/ou d'une ou de plusieurs autres substances actives, de préférence à l'intérieur du noyau de la capsule, et étant sélectionnées de préférence dans le groupe comprenant les probiotiques ; les prébiotiques ; les synbiotiques ; les polyphénols ; les substances, protéines et/ou enzymes supportant les probiotiques ; les agents antibiotiques, antimycosiques, antiprotozoaires, antihelminthiques, antiviraux ou anti-inflammatoires ; les aminosalicylates ; l'acide acétylsalicylique ; les antagonistes de la prostaglandine D2, de préférence le laropiprant ; les acides gras à chaîne courte ; les acides gras à chaîne moyenne ; les corticostéroïdes systémiques ou topiques ; les agonistes du récepteur adrénergique β2 ; la théophylline et d'autres substances de la famille des xanthines ; les statines, de préférence sélectionnées dans le groupe consistant en l'atorvastatine, la cérivastatine, la fluvastatine, la lovastatine, la mévastatine, la pitavastatine, la pravastatine, la rosuvastatine et la simvastatine ; les petites molécules ; les peptides, les produits biologiques, les protéines de fusion, les anticorps monoclonaux ou les dérivés de ceux-ci.

10. Composition comprenant une microcapsule selon l'une quelconque des revendications 1 à 9.

11. Composition selon la revendication 10, formulée pour une administration orale avec une libération régulée et/ou retardée de vitamine B3.

12. Composition selon la revendication 11, **caractérisée en ce que** l'application orale est effectuée avec une teneur en substance active pour l'acide nicotinique et/ou le nicotinamide de 1 à 5000 mg chacun par forme galénique finie, de préférence avec une teneur en substance active de 30 à 3000 mg chacun par forme galénique finie.

13. Composition selon l'une quelconque des revendications 10 à 12, comprenant des combinaisons de doses variables et/ou fixes avec une ou plusieurs autres substances actives et/ou compositions, étant sélectionnées de préférence dans le groupe comprenant les probiotiques ; les prébiotiques ; les synbiotiques ; les polyphénols ; les substances, protéines et/ou enzymes supportant les probiotiques ; les agents antibiotiques, antimycosiques, antiprotozoaires, antihelminthiques, antiviraux ou anti-inflammatoires ; les aminosalicylates ; l'acide acétylsalicylique ; les antagonistes de la prostaglandine D2, de préférence le laropiprant ; les acides gras à chaîne courte ; les acides gras à chaîne moyenne ; les corticostéroïdes systémiques ou topiques ; les agonistes du récepteur adrénergique β2 ; la théophylline et d'autres substances de la famille des xanthines ; les statines, de préférence sélectionnées dans le groupe consistant en l'atorvastatine, la cérivastatine, la fluvastatine, la lovastatine, la mévastatine, la pitavastatine, la pravastatine, la rosuvastatine et la simvastatine ; les petites molécules ; les peptides, les produits biologiques, les protéines de fusion, les anticorps monoclonaux ou les dérivés de ceux-ci.

14. Composition selon l'une quelconque des revendications 10 à 13, pour une utilisation pour influencer de manière bénéfique le microbiote intestinal et/ou pour influencer ou prévenir de manière bénéfique des taux de lipides dans le sang et/ou le plasma et/ou le sérum défavorables et/ou anormaux et/ou déséquilibrés et/ou pour influencer ou prévenir de manière bénéfique une inflammation intestinale et/ou d'autres changements défavorables et/ou anormaux dans l'intestin, et/ou pour une utilisation dans la thérapie et/ou la prophylaxie
d'une maladie et/ou d'un syndrome associé à et/ou accompagné d'un microbiote intestinal défavorable ou anormal ou déséquilibré et/ou
d'une maladie et/ou d'un syndrome associé à des et/ou accompagné de taux de lipides dans le sang et/ou le plasma et/ou le sérum défavorables ou anormaux ou déséquilibrés, de préférence dans la maladie du foie gras non alcoolique (NAFLD) et/ou la stéatohépatite non alcoolique (NASH), pour diminuer la teneur en graisse hépatique et/ou influencer de manière bénéfique les taux de lipides dans le sang et/ou le plasma et/ou le sérum, et/ou
d'une maladie et/ou d'un syndrome associé à et/ou accompagné d'une inflammation intestinale, de préférence dans les affections abdominales inflammatoires (IBD), et/ou
d'autres changements défavorables ou anormaux dans l'intestin, et/ou
d'un ou de plusieurs sélectionnées dans le groupe consistant en les troubles du métabolisme des lipides, la dyslipidémie, la NAFLD, la NASH, les maladies cardiovasculaires, l'artériosclérose, l'athérosclérose, le syndrome métabolique, l'obésité, le diabète, les maladies inflammatoires de l'intestin grêle et/ou du côlon, l'IBD, la maladie de Crohn, la rectocolite hémorragique, une colite indéterminée, le syndrome du côlon irritable, un carcinome du côlon, le psoriasis, une allergie, un eczéma atopique, l'asthme, une bronchopneumopathie chronique obstructive, une fibrose kystique, et d'autres maladies et/ou syndromes associés à des et/ou accompagnés de taux de lipides dans le sang et/ou le plasma et/ou le sérum défavorables ou anormaux ou déséquilibrés et/ou une inflammation intestinale et/ou d'autres changements défavorables ou anormaux dans l'intestin et/ou des changements défavorables ou anormaux du microbiote intestinal et/ou une interaction altérée entre le microbiote intestinal et l'intestin.

15. Procédé de production d'une microcapsule selon l'une quelconque des revendications 1 à 9, ou d'une composition selon l'une quelconque des revendications 10 à 14, comprenant les étapes suivantes
a. la fourniture d'un matériau de noyau comprenant ou consistant en de la vitamine B3,
b. l'enrobage du matériau de noyau avec une première couche de gomme laque,
c. la fourniture d'une substance modulant le pH,
d. l'application de la substance modulant le pH sur la première couche de gomme laque, et
e. l'application d'une seconde couche de gomme laque.
